# EUROPEAN PATENT SPECIFICATION

(11) **EP 2 334 819 B1**
(45) Date of publication and mention of the grant of the patent: **30.10.2013**
(21) Application number: 09785723.9
(22) Date of filing: 02.10.2009
(51) Int. Cl.: C12Q 1/68, C12N 1/21, C12N 15/10, C12N 15/63, C12N 15/66, C40B 40/06

(54) **TRANSCRIPTION FACTOR DECOYS**
TRANSKRIPTIONSFAKTORKÖDER
LEURRES DE FACTEURS DE TRANSCRIPTION

(30) Priority: 03.10.2008 WO PCT/GB2008/003353; 03.10.2008 US 102414 P; 08.04.2009 GB 0906130; 08.04.2009 US 167592 P
(43) Date of publication of application: 22.06.2011
(73) Proprietor: Procarta Biosystems Limited, Norwich, Norfolk NR4 7UH (GB)
(72) Inventor: MCARTHUR, Michael, Rocklands All Saints, Norfolk NR17 1XR (GB); MOORE, Jane Marion, Taverham, Norfolk NR8 6XP (GB)
(74) Representative: Boxall, Sarah Jane
(86) International application number: PCT/GB2009/051301
(87) International publication number: WO 2010/038083

(56) References cited:
- "PROKARYOTIC GENE THERAPY TO COMBAT MULTIDRUG RESISTANT BACTERIAL INFECTION" GENE THERAPY, MACMILLAN PRESS LTD., BASINGSTOKE, GB, vol. 7, no. 9, 1 May 2000 (2000-05-01), pages 723-725, XP009004366 ISSN: 0969-7128
- CRANENBURGH R M ET AL: "Escherichia coli strains that allow antibiotic-free plasmid selection and maintenance by repressor titration." NUCLEIC ACIDS RESEARCH 1 MAR 2001, vol. 29, no. 5, 1 March 2001 (2001-03-01), page E26, XP002514866 ISSN: 1362-4962
- TOLEDANO M B ET AL: "Redox-dependent shift of OxyR-DNA contacts along an extended DNA-binding site: A mechanism for differential promoter selection" CELL, CELL PRESS, CAMBRIDGE, NA, US, vol. 78, no. 5, 9 September 1994 (1994-09-09), pages 897-909, XP024246465 ISSN: 0092-8674 [retrieved on 1994-09-09]
- ONIZUKA T ET AL: "CO2 response for expression of ribulose-1,5-bisphosphate carboxylase/oxygenase genes is inhibited by AT-rich decoy in the cyanobacterium" FEBS LETTERS, ELSEVIER, AMSTERDAM, NL, vol. 542, no. 1-3, 8 May 2003 (2003-05-08), pages 42-46, XP004422720 ISSN: 0014-5793
- MCARTHUR MICHAEL ET AL: "Manipulating and understanding antibiotic production in Streptomyces coelicolor A3(2) with decoy oligonucleotides" PROCEEDINGS OF THE NATIONAL ACADEMY OF SCIENCES OF THE UNITED STATES OF AMERICA, vol. 105, no. 3, January 2008 (2008-01), pages 1020-1025, XP002561749 ISSN: 0027-8424

## Description

The present invention relates to methods and compositions for modulating phenotypes of prokaryotes, particularly pathogenic bacteria, using transcription factor decoy sequences.

Control of bacterial growth and virulence poses an increasing problem, particularly in medical and veterinary applications, and may become a major challenge to public health. Antibiotics for use against pathogenic bacteria are well known in the art. However, extensive use of such antibiotics has led to the emergence of bacteria which are resistant to at least one, and in some cases, multiple antibiotics (so-called multi-drug resistant strains). The situation is exacerbated by a decrease in the numbers of conventional antibiotics being discovered and under development. Indeed, antibiotic resistance is a major challenge for antibacterial research and threatens the potency of marketed antibiotics as well as those still under development. Consequently there is a need for new anti-bacterial agents which can be used to tackle bacterial spread and infection.

As an example, *Staphylococcus aureus* represents a major challenge to global health causing numerous human and animal diseases, with a broad spectrum of severity from skin infections to fatal sepsis by toxic shock (Lowy, New Engl. J. Med. (1998) 339:520-532). It is estimated that 20% of the human population are carriers of the bacteria in soft tissue infection, often with unnoticeable clinical features, from where the bacteria can penetrate the body to infect the blood and thereafter bone and cardiac tissues (Gordon and Lowy, Clin. Infect. Dis. (2008) 40 (S5):S350-9). Though primarily thought of as an extracellular pathogen, substantial evidence is emerging that suggests that S. *aureus* can avoid antibacterial action or macrophage engulfment by persisting intercellularly (Garzoni and Kelley, Trends Microbiol. (2009) 17:59-65), potentially complicating its treatment. Resistance mechanisms have also been acquired or have developed since first exposure to antibiotics to such an extent that multiply-drug resistant strains are now the norm in the clinic (Hawkey, J. Antimicrob. Chemother. (2008) 62 (S1):il-9), which has further limited the treatment options.

There are several reasons why *S. aureus* is such a versatile pathogen: it possesses mechanisms to evade the host's immune response (Foster, Nat. Rev. Microbiol. (2005) 3:948-958); it can produce a wide range of virulence determinants (Novick, Mol. Micro. (2003) 48:1429-1449); it has the ability to catabolise host tissues (Vojtov et al. (2002) Proc. Natl. Acad. Sci. USA 99:10102-10107) and it can readily to adapt to the nutrient-limited and anoxic environments found inside the host (Haselbeck et al, Curr. Pharm. Des. (2002) 8:1155-1172). Many of these processes are controlled at the level of transcription and as such are not currently the targets of traditional antibiotics (which act mostly on cell wall, protein or DNA synthesis).

*Streptococcus pyogenes* (Group A Streptococcus: GAS) infections can usually be treated with many different antibiotics. Early treatment may reduce the risk of death from invasive group A streptococcal disease. However, even the best medical care does not prevent death in every case. For those with very severe illness, supportive care in an intensive care unit may be needed. For persons with necrotizing fasciitis, surgery often is needed to remove damaged tissue. Strains of *S. pyogenes* resistant to macrolide antibiotics have emerged, however all strains remain uniformly sensitive to penicillin.

Resistance of *Streptococcus pneumoniae* to penicillin and other beta-lactams is increasing worldwide. The major mechanism of resistance involves the introduction of mutations in genes encoding penicillin-binding proteins. Selective pressure is thought to play an important role, and use of beta-lactam antibiotics has been implicated as a risk factor for infection and colonization. Streptococcus pneumoniae is responsible for pneumonia, bacteremia, otitis media, meningitis, sinusitis, peritonitis and arthritis.

Penicillin-resistant pneumonia caused by *Streptococcus pneumoniae* (commonly known as *pneumococcus*), was first detected in 1967, as was penicillin-resistant gonorrhoea. Resistance to penicillin substitutes is also known as beyond *S. aureus.* By 1993 *Escher-ichia coli* was resistant to five fluoroquinolone variants. *Mycobacterium tuberculosis* is commonly resistant to isoniazid and rifampin and sometimes universally resistant to the common treatments. Other pathogens showing some resistance include *Salmonella, Campylobacter,* and *Streptococci.*

*Enterococcus faecium* is another superbug found in hospitals. Penicillin-Resistant Enterococcus was seen in 1983, vancomycin-resistant enterococcus (VRE) in 1987, and Linezolid-Resistant Enterococcus (LRE) in the late 1990s.

*Pseudomonas aeruginosa* is a highly prevalent opportunistic pathogen. One of the most worrisome characteristics of *P. aeruginosa* consists in its low antibiotic susceptibility. This low susceptibility is attributable to a concerted action of multidrug efflux pumps with chromosomally-encoded antibiotic resistance genes (e.g. *mexAB-oprM, mexXY* etc) and the low permeability of the bacterial cellular envelopes. Besides intrinsic resistance, *P. aeruginosa* easily develop acquired resistance either by mutation in chromosomally-encoded genes, or by the horizontal gene transfer of antibiotic resistance determinants. Development of multi-drug resistance by *P. aeruginosa* isolates requires several different genetic events that include acquisition of different mutations and/or horizontal transfer of antibiotic resistance genes. Hypermutation favours the selection of mutation-driven antibiotic resistance in *P. aeruginosa* strains producing chronic infections, whereas the clustering of several different antibiotic resistance genes in integrons favours the concerted acquisition of antibiotic resistance determinants. Some recent studies have shown that phenotypic resistance associated to biofilm formation or to the emergence of small-colony-variants may be important in the response of *P. aeruginosa* populations to antibiotics treatment (Cornelis P. (editor) Pseudomonas: Genomics and Molecular-Biology (1st ed.) (2008) Caister Academic Press).

*Clostridium difficile* is a nosocomial pathogen that causes diarrhoeal disease in hospitals world wide. Clindamycin-resistant *C. difficile* was reported as the causative agent of large outbreaks of diarrhoeal disease in hospitals in New York, Arizona, Florida and Massachusetts between 1989 and 1992 (Johnson S. et al, New England Journal of Medicine (1999) 341:1645-1651). Geographically dispersed outbreaks of *C. difficile* strains resistant to fluoroquinolone antibiotics, such as ciprofloxacin and levofloxacin, were also reported in North America in 2005 (Loo V. et al N. Engl. J. Med. (2005) 353 (23):2442-9).

*E. coli* and *Salmonella* come directly from contaminated food. Of the meat that is contaminated with E. coli, eighty percent of the bacteria are resistant to one or more drugs made; it causes bladder infections that are resistant to antibiotics ("HSUS Fact Sheet"). Salmonella was first found in humans in the 1970s and in some cases is resistant to as many as nine different antibiotics ("HSUS Fact Sheet"). When both bacteria are spread, serious health conditions arise. Many people are hospitalised each year after becoming infected, and some die as a result.

On the 5th November 2004, the Centres for Disease Control and Prevention (CDC) reported an increasing number of *Acinetobacter baumannii* bloodstream infections in patients at military medical facilities in which service members injured in the Iraq/Kuwait region and in Afghanistan were treated. Most of these showed multi-drug resistance with a few isolates resistant to all drugs tested.

Norris et al (Gene Therapy (2000), 7:723-725) review gene therapy approaches using bacteriophage to combat bacteria which are resistant to multiple antibiotics, while Cranenburgh et al (Nucl. Acid Res. (2001) 29(5):e26) describe the creation of two *E. coli* strains which allow the use of repressor titration, rather than antibiotic selection, for plasmid selection and propagation.

DNA-based therapies constitute a new class of antibacterial agents that are designed to work on gene targets that are essential for viability or pathogenicity. Such therapies are potentially applicable to a vast array of pathogens and the development of resistance to these therapeutics, through target site modification, is anticipated to be a highly unlikely event. In addition, the agents have the advantages of potentially quicker development times and simultaneously acting upon a number of novel, resistance-free targets.

DNA-based therapies have the potential to overcome the limitations of existing therapies because they can be designed to treat potentially any pathogen either by preventing expression of an antibiotic resistance mechanism or by inhibiting growth by down-regulating essential or adaptive genes. In addition, bacteria are unlikely to develop resistance to these agents as this would require simultaneous mutations that affected both the transcription factor and its cognate binding site(s). This is particularly true for agents that control several essential genes.

Transcription Factor Decoys (TFDs) are one such DNA-based therapeutic. Decoy oligonucleotides are designed to mimic the binding sites of transcription factors and prevent the latter from binding to their cognate genomic targets, with a consequent modification of gene expression (Mann and Dzau, J. Clin. Investigation (2000) 106:1071-1075).

Their utility has been demonstrated primarily in eukaryotic systems, where a spur to their development was their potential to function as novel classes of therapeutic agents (Mann & Dzau (2000)). To this end, decoy oligonucleotides have been used to demonstrate that transcription factor EF2 represses smooth muscle proliferation in rats (Morishita et al, Proc. Natl. Acad. Sci. USA (1995) 95: 5855-5859); to block STAT3-mediated proliferation of carcinomas (Leong et al, Proc. Natl. Acad. Sci. USA (2003) 100:4138-4143); and to show that targeting of the cAMP response element can control cancer proliferation *in vivo* (Park et al, J. Biol. Chem. (1999) 274: 1573-1580).

TFDs have distinct advantages over other DNA-based therapeutics. Their mechanism of action is simple - they control gene expression by sequestering transcription factors, preventing the latter from binding to promoters by flooding the cell with numerous copies of the specific binding sequences (hence, the term "decoys"). This is in contrast to antisense strategies where targets are difficult to define due to the complex secondary structure of mRNA. In comparison to antisense approaches, TFDs have the further advantages that they act rapidly, preventing expression of genes, whereas antisense approaches deal with the consequences of expression. As a result, TFDs are effective at much lower concentrations, because a single TFD-transcription factor interaction can block transcription of a single gene that otherwise may have given rise to many thousands of copies of mRNA, which constitute the targets for the antisense approach.

To the knowledge of the inventors, two reports have been made of the use of decoys in prokaryotes. In the first, an AT-rich decoy oligonucleotide, designed to mimic an AT-rich element in the promoter of ribulose 1,5,-bisphosphate carboxylase/oxygenase (rbc) genes, was used to alter CO₂-regulation of the expression of the rbc genes in *Cyanobacterium (*Onizuka et al, FEBS Lett. (2003) 542:42-46).

In the second report, decoy oligonucleotides were used to identify new cis-regulatory sequences in the promoter of actII-orf4 in *S. coelicolor* A3(2), which encodes the pathway specific activator for production of the antibiotic actinorhodin (McArthur and Bibb, PNAS (2008) 105:1020-1025).

It is against this background that the present invention has been devised.

The inventors have used TFD technology to disrupt regulation of cellular pathways, responses and resistance mechanisms in prokaryotes in order to alter cell viability. For example, the methods may be used to alter one or more of antibiotic resistance phenotype, cellular adaptive responses to environmental conditions, cellular virulence and cellular essential metabolism. In doing so, the inventors have provided means for treating bacterial infections and containing bacterial growth.

Accordingly in one aspect the invention resides in the use of transcription decoy factors (TFDs) to reduce the viability of prokaryotic cells.

In particular, the invention encompasses a method of reducing the viability of prokaryotic cells, the method comprising:
(a) providing a decoy polynucleotide comprising a binding site for a target transcription factor (a decoy sequence);
(b) introducing the decoy polynucleotide into a prokaryotic cell which comprises a binding site for the transcription factor, operably linked to a gene or genes;
wherein introduction of the decoy polynucleotide reduces binding of the target transcription factor to the binding site in the cell and causes an alteration in expression of the operably linked gene or genes;
and wherein the target transcription factor comprises a regulator of expression of a gene or genes encoding one or more of:
(i) a cellular adaptive response;
(ii) a cellular intrinsic antibiotic resistance mechanism;
(iii) a cellular virulence factor;
(iv) a cellular stress response; or
(v) a cellular essential gene.

In another aspect, the invention provides:
- a method of increasing prokaryotic antibiotic susceptibility, the method comprising:
   (a) providing a decoy polynucleotide comprising a binding site for a target transcription factor (a decoy sequence);
   (b) introducing the decoy polynucleotide into a prokaryotic cell which comprises a binding site for the transcription factor, operably linked to a gene or genes;
   wherein introduction of the decoy polynucleotide reduces binding of the target transcription factor to the binding site in the cell and causes an alteration in expression of the operably linked gene or genes, thereby increasing antibiotic susceptibility of the cell;
   and wherein the target transcription factor comprises a regulator of expression of a gene or genes encoding one or more of:
   (i) a cellular adaptive response;
   (ii) a cellular intrinsic antibiotic resistance mechanism;
   (iii) a cellular virulence factor;
   (iv) a cellular essential gene.

Also disclosed is:
- a decoy polynucleotide comprising a binding site for a target transcription factor, wherein the binding site is not operably linked to a gene, and wherein the transcription factor comprises a regulator of expression of a gene or genes encoding one or more of:
   (i) a cellular adaptive response;
   (ii) a cellular intrinsic antibiotic resistance mechanism;
   (iii) a cellular virulence factor;
   (iv) a cellular essential gene.

The target transcription factor may be selected from: WhiB7 (**SEQ ID NOs: 5 & 9**); FabB (**SEQ ID NO: 6**); LytM (**SEQ ID NO: 7**); Ssa (**SEQ ID NO: 8**); FadR (**SEQ ID NO: 10**); YycG/YycF (**SEQ ID NOs: 11 & 12**); Sigma 54 (or SigA) (**SEQ ID NOs: 13 & 14**); Fur (**SEQ ID NOs: 15, 16 & 17**); TcdR (**SEQ ID NO: 18**); Vfr (**SEQ ID NOs: 19, 20 & 21**); NtrC (**SEQ ID NO: 22**); ArsR (**SEQ ID NOs: 23 & 24**); TcaA (**SEQ ID NO: 25**); AgrA (**SEQ ID NOs: 26 & 27**); WalR (**SEQ ID NOs: 44, 45, 48, 49** & **57**); sigB (**SEQ ID NO: 58**); or Ksig (**SEQ ID NOs: 59 & 60**); or a functional variant or homolog of any thereof.

Also disclosed is a decoy polynucleotide as described above comprising more than one binding site for more than one target transcription factor.
- a cell comprising an exogeneous decoy polynucleotide, the polynucleotide comprising a binding site for a target transcription factor which is not operably linked to a gene; wherein the cell comprises a binding site for the transcription factor operably linked to a gene or genes; and wherein the target transcription factor comprises a regulator of expression of a gene or genes encoding one or more of:
   (i) a cellular adaptive response;
   (ii) a cellular intrinsic antibiotic resistance mechanism;
   (iii) a cellular virulence factor;
   (iv) a cellular essential gene.
- a method of treating bacterial infection in a subject comprising administering a decoy polynucleotide of the invention, optionally in combination with one or more antibiotics and/or antibacterial agents.
- an *ex vivo* method of killing bacteria, inhibiting bacterial growth, or reducing bacterial virulence, the method comprising applying a decoy polynucleotide of the invention, optionally in combination with one or more antibiotics and/or antibacterial agents.
- a pharmaceutical composition comprising a decoy polynucleotide of the invention and a pharmaceutically acceptable excipient or carrier, optionally in combination with one or more antibiotics and/or antibacterial agents.
- a disinfectant composition comprising a decoy polynucleotide of the invention, optionally in combination with one or more antibiotics and/or antibacterial agents.
- a cleaning composition comprising a decoy polynucleotide of the invention, optionally in combination with one or more antibiotics and/or antibacterial agents.
- a kit comprising a decoy polynucleotide of the invention, and one or more antibiotics and/or antibacterial agents, wherein the decoy and the one or more antibiotics and/or antibacterial agents are for combined use in killing bacteria, inhibiting bacterial growth, or reducing bacterial virulence.
- a method of reducing virulence of a prokaryotic cell, the method comprising:
   (a) providing a decoy polynucleotide comprising a binding site for a target transcription factor;
   (b) introducing the decoy polynucleotide into a prokaryotic cell which comprises a binding site for the transcription factor, operably linked to a gene or genes;
   wherein introduction of the decoy polynucleotide reduces binding of the target transcription factor to the binding site in the cell and causes an alteration in expression of the operably linked gene or genes, thereby reducing cellular virulence;
   and wherein the target transcription factor comprises a regulator of expression of a gene or genes encoding one or more of:
   (i) a cellular adaptive response;
   (ii) a cellular intrinsic antibiotic resistance mechanism;
   (iii) a cellular virulence factor;
   (iv) a cellular essential gene.
- a decoy polynucleotide comprising a binding site for a target transcription factor, wherein the binding site has a sequence set out in SEQ ID NO: 57, SEQ ID NO: 58 or SEQ ID NO: 60.
- use of a decoy polynucleotide comprising the sequence of SEQ ID NO: 57, SEQ ID NO: 58 or SEQ ID NO: 60 in the treatment of bacterial infection.

In general, a transcription factor decoy (TFD) sequence (or decoy sequence) comprises a transcription factor binding site which comprises or competes with a native or endogenous *cis*-regulatory sequence in a cell for binding to a cognate transcription factor in the cell. When introduced into suitable host cells comprising the cis-regulatory sequence (by a method described herein or otherwise) the decoy sequence competes with the cis-regulatory sequence in a cell for binding to the cognate transcription factor. Such competition reduces binding of the transcription factor to the cis-regulatory sequence in the cell and alters the expression of a gene(s) whose expression is regulated by binding of the transcription factor to the cis-regulatory sequence. Decoy function as used herein refers to the capability of a sequence to compete with a cis-regulatory sequence for binding to a cognate transcription factor in this way.

A *cis*-regulatory sequence or element generally refers to a nucleotide sequence which occurs upstream (5') or downstream (3') of the transcriptional start site of a gene or genes and which functions to regulate expression of the gene or genes. Typically, a *cis*-regulatory sequence comprises a binding site for a protein (transcription factor) and binding of the protein regulates transcription of the given gene(s). Binding of the protein to the sequence results directly or indirectly in modulation of expression of the gene(s). For example, the bound protein may interact with another protein bound to a nearby region which is needed for transcription and anchor the protein in the correct position, or may inhibit binding of another protein which is necessary for transcription. Typically the cis-regulatory sequence or element occurs in the promoter region of a gene, but it is not unusual in prokaryotes for *cis*-regulatory sequences to be positioned hundreds of base pairs upstream or downstream of the genes they affect.

A cis-regulatory sequence may be repressive (inhibits or reduces transcription of the gene(s) when bound by a transcription factor) or activatory (activates or increases transcription of the gene(s) when bound by a transcription factor). Thus a transcription factor which binds a *cis*-regulatory sequence may be a negative effector (repressor protein) or a positive effector (activator).

A target sequence for a decoy polynucleotide or decoy sequence refers to the cellular transcription factor binding site with which it competes for binding of transcription factor. Similarly the target regulator is the transcription factor which the decoy sequence binds. The target gene(s) of the decoy sequence is the gene(s) which is/are operably linked to the cellular transcription factor binding site. Thus a decoy sequence disrupts the regulation of expression of the target gene(s).

The inventors have used decoys to alter prokaryotic phenotypes, especially phenotypes which are relevant to bacterial pathogenicity and to tackling bacterial infection. In particular the inventors have used decoys to disrupt expression of cellular pathways, responses and resistance mechanisms in order to render cells more susceptible to killing or growth inhibition, and/or less capable of survival under hostile conditions, and/or less virulent.

In one aspect, the present methods disrupt gene expression in order to render the cell less viable, e.g. under the prevailing conditions. For example, cells may become less viable because of increased susceptibility to antibiotics, impaired stress response, impaired virulence response and/or disrupted expression of essential genes. The present methods may use decoys to weaken cell defence or survival systems and or virulence systems. The methods may make the cells more vulnerable to cell death and/or inhibit growth or pathogenicity. Thus in one aspect, the present decoys may produce a bactericidal or bacteristatic effect.

For example, in one aspect the inventors have used decoys to alter the antibiotic susceptibility phenotype of prokaryotes, in particular bacteria and especially pathogenic bacteria. In one aspect the increase in susceptibility is not specific to one antibiotic or class of antibiotic. In one aspect the increase in susceptibility is not specific to the structure and/or mechanism of any one antibiotic or class of antibiotic. In one aspect sensitivity to a broad spectrum of antibiotics or classes of antibiotics is increased.

In one aspect an increase in susceptibility is not specific to any one antibiotic or class of antibiotic selected from: aminoglycosides (such as kanamycin); from the carbapenems (such as meropenem); the cephalosporins (such as cefepime); the glycopeptides (such as vancomycin and daptomycin); the penicillins (such as ampicillin, carbenicillin and penicillin); the polypeptide antibiotics (such as polymixcin B); the quinolines (such as levaquin); the sulfonamides (such as Bactrim); the tetracyclines (such as tetracycline); and variously, chloramphenicol, rifampicin, Zyvox.

The decoy methods described herein can also be used to alter phenotypes such as cellular adaptive responses, or cellular virulence. The methods may also be used to alter cellular expression of essential genes and cell survival, e.g. under given culture or physiological conditions, e.g. limiting nutrients.

In general the methods provide new means for targeting bacterial infections and associated diseases. This may be by potentiating the effects of an antibiotic, or by a direct inhibitory effect on the bacteria, e.g. growth or survival inhibition, inhibition of expression of virulence determinants, inhibition of expression of essential genes. Thus, a TFD may act as an antibiotic or an antibacterial agent in its own right. In one aspect the methods use decoys which disrupt expression of genes which determine cell viability.

As above, the present methods use decoys to disrupt regulation of cellular pathways, responses and resistance mechanisms. In some cases this involves targeting binding of a global regulatory molecule. Targeted gene(s) may those which are regulated in response to antibiotic.

Targets may include regulators of intrinsic antibiotic resistance mechanisms, including intrinsic multi-drug resistance mechanisms. Intrinsic mechanisms may provide a physical barrier to drugs such as antibiotics, for example by increasing the rate at which antibiotics are removed from the cell or by reducing penetrability or permeability of the cell wall. Intrinsic mechanisms may provide resistance to antibiotics which have entered the cytoplasm. Intrinsic resistance mechanisms may be induced in response to environmental conditions, such as stress or other conditions listed herein. In some cases, expression of genes encoding intrinsic resistance mechanisms is regulated in response to antibiotics. For example, these may be genes encoding proteins with a physiological role whose expression is regulated differently in response to antibiotics. Genes encoding proteins with a role in intrinsic resistance may encode for example, efflux pumps, or proteins determining cell wall composition or density or cell wall metabolism. In one aspect, the targeted genes encode a resistance mechanism that is non-antibiotic specific or non-antibiotic class specific, e.g. in terms of antibiotic structure or mechanism.

Targets may include regulators of cellular adaptive responses. Typically these are adaptive responses to environmental conditions, e.g. hostile environmental conditions. For example, these include stress responses such as oxidative stress responses or peroxide stress responses, responses to lack of nutrients, e.g. nitrogen limitation, responses to toxins, e.g. metallotoxins such as high iron conditions, responses to high acidity (low pH) conditions. Thus targets may include regulators of stress response genes, metalloregulatory proteins, and regulators of nitrogen fixation genes.

Cellular adaptive genes are those whose expression are determined by environmental factors and influence the ability of the bacterium to survive and cause disease in that environment. For example, a bacterium may need to adapt to conditions of low iron concentration found inside a host and do so by inducing a set of genes that express proteins capable of sequestering iron and bringing it into the cell. It will also include the formation ofbiofilms, cell wall physiology and changes to primary metabolism.

Stress response genes form a class of cellular adaptive genes. These are a limited number of sets of genes, all or some of which are induced in response to a wide range of stresses. The stresses can include physical factors (temperature changes, acidity, low oxygen), biotic factors (in response to host or other bacterium) and chemical factors (such as treatment with antibiotics).

Also included are responses to physiological conditions. Thus targets may include regulators of expression of pathogenicity genes or virulence factors, such as toxins. Targets also include regulators of proteins which determine bio-film formation and nutrient (e.g. iron) scavenging.

Virulence factor genes are those that control the production of specific molecules from the bacterium that cause disease, such as toxins, or elicit a response from the host that affect the ability of the bacterium to survive and cause disease.

Targets also include regulators of essential genes. These are genes whose expression is essential for cell survival in the prevailing environmental conditions. For example, a target may be a regulator of genes encoding proteins needed for DNA replication, primary metabolic pathways, fatty acid synthesis or cell division.

Essential genes are those that are required for the bacterium to survive in any environment.

Often there is overlap in the cell between the above described systems. Thus one regulatory protein may regulate expression of genes encoding virulence factors and cell wall metabolism in response to environmental stress. In another example, a regulatory protein may control expression of a gene which is essential to the cell under particular environmental conditions, e.g. nutrient shortage.

In one aspect the present method may target genes, the regulation of expression of which determines the ability of the cell to survive in the presence of antibiotics generally, e.g. more than one antibiotic or more than one class of antibiotic.

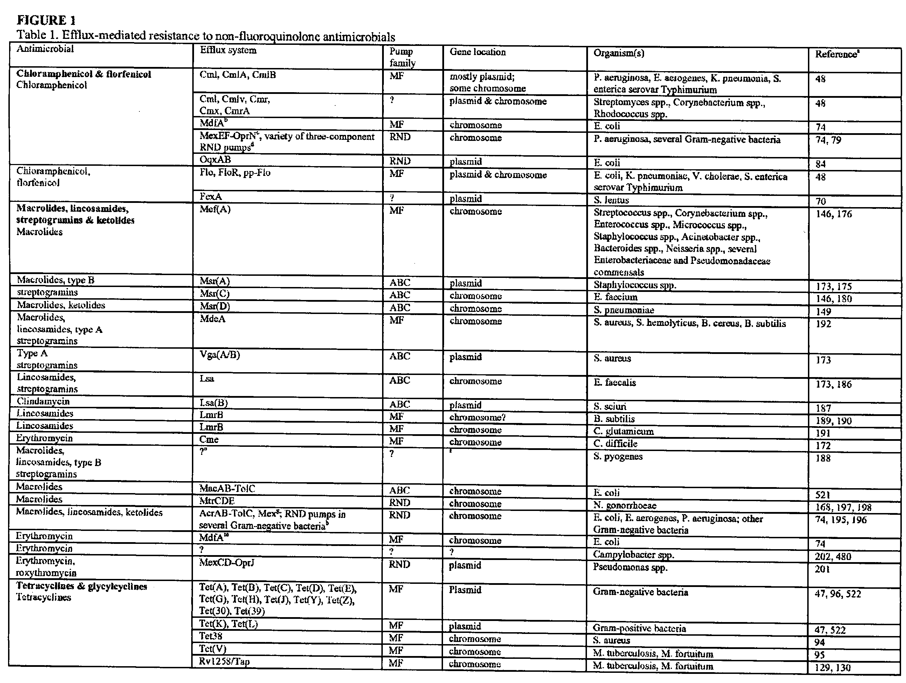

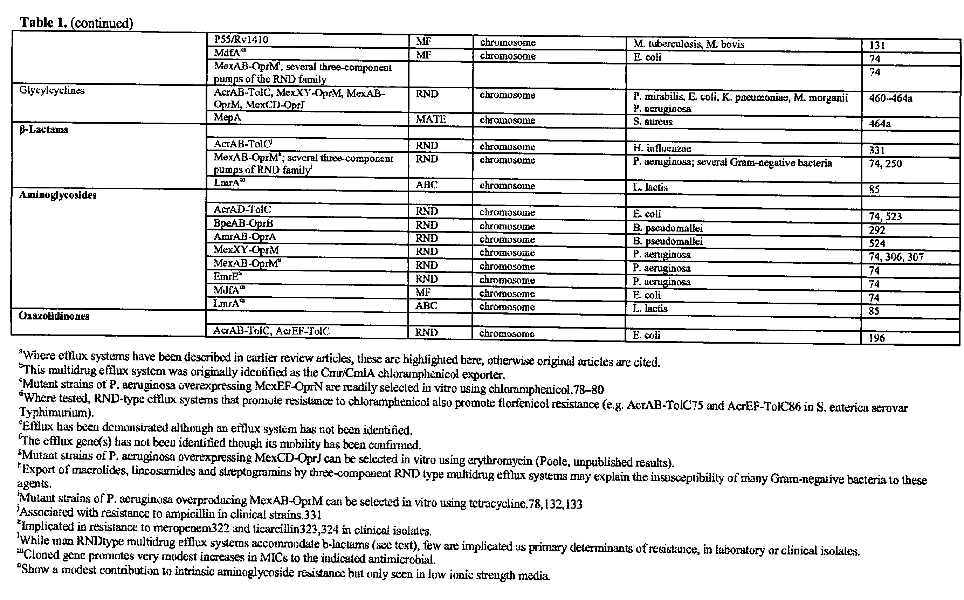

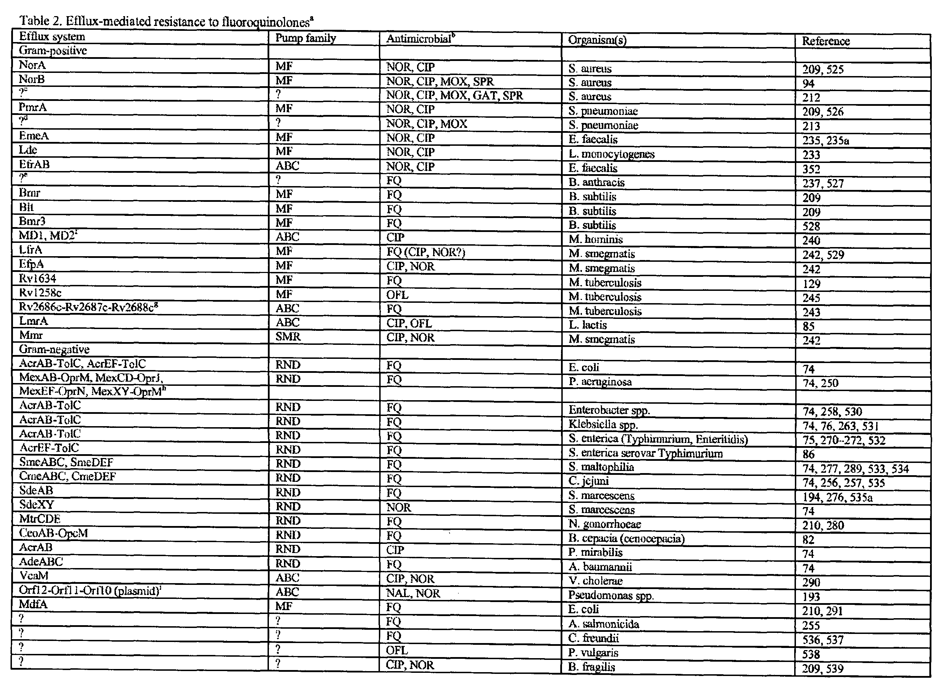

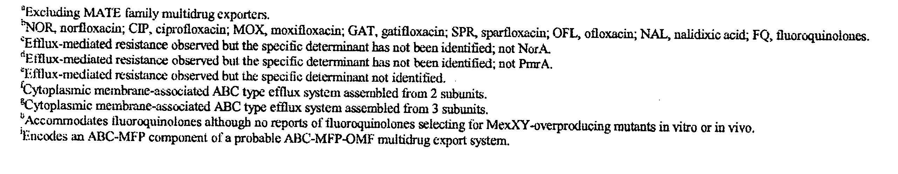

Examples of antibiotic resistance genes are disclosed in co-pending application PCT/GB2008/003353. Decoys such as those described herein may be used to target any one or more of the genes in PCT/GB2008/003353 encoding beta lactamases, efflux pumps e.g. those in Figure 1, aminoglycoside-modifying enzymes or ermB gene, in order to alter, e.g. increase, antibiotic resistance. Indeed, decoys may contain two or more sequences targeted to act on more than one gene or transcription factor and/or more than one bacterial strain. For example, a decoy may comprise the Sig binding set from Gram-negative and Gram-positive bacteria.

TFDs may be used to treat a variety of bacterial infections wherever they occur within the human body. Five general areas of bacterial infection can be described. Respiratory tract infections are amongst the commonest, the upper respiratory tract infections including the ears, throat, and nasal sinuses that can be treated with tropical applications or aerosol preparations. Lower tract infections include pneumonia (which is caused by a range of bacterial pathogens, bronchitis and infective complications of cystic fibrosis. A common problem in both community and hospital practice is urinary tract infections, where the urine becomes infected and antibacterials need to enter the bladder, prostrate, ureter and kidneys. The gut is vulnerable to infections (an example of gastrointestinal infections), where bacteria cause disease by either by mucosal invasion or toxin production, an example of which include cholera epidemics, and when used antibiotics are either ingested or administered intravenously. Skin and soft tissue infections, which can be treated by topical applications, are common following traumatic injury or burns, which allow colonisation and ingression of micro-organisms resulting in infections that are both localised or have spread rapidly through tissues. Microbes responsible for skin infections often arise from normal skin flora, such as Streptococcus pyogenes causing superficial skin infections (impetigo), cellulitis (more deep-seated infection that can spread to the blood) and necrotizing fasciitis, a rapidly progressive infection that is often life-threatening. Finally infections of the central nervous system, such as bacterial meningitis, are perhaps the most challenging to treat as therapies must penetrate the blood-brain barrier, as too have the pathogenic bacteria.

Decoys may be prepared and tested as described in co-pending application PCT/GB2008/003353.

Any one or more of the above aspects may be combined when considering the present methods.

The present methods use decoy polynucleotides to disrupt gene expression in prokaryotes. A decoy polynucleotide comprises a transcription factor binding site (decoy sequence). Generally in the present methods, a decoy polynucleotide is introduced into a target cell, comprising a gene or genes operably linked to a *cis*-regulatory sequence comprising a binding site for the transcription factor. The polynucleotide titrates transcription factor from the cellular binding site and disrupts regulation of expression of the operably linked gene or genes in the cell. The alteration in gene expression causes changes in cellular phenotypes.

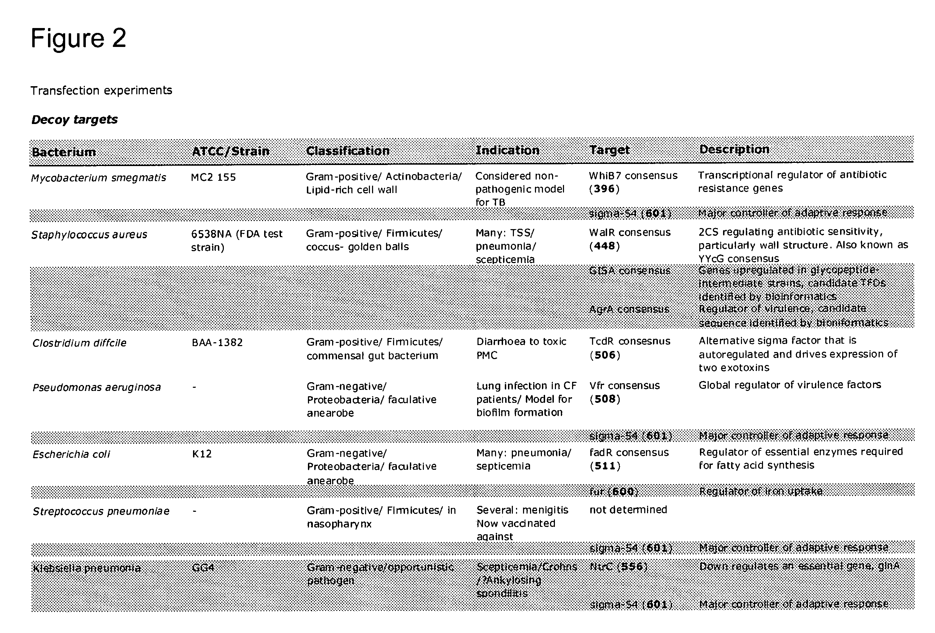

Transcription factors and genes which may be targeted using the present methods include those described herein and in co-pending application PCT/GB2008/003353. Specific examples include the following regulators and regulatory sequences which are also listed in Figure 2.

Reference to each of the specific regulators includes reference to that regulator in the species listed and to any species homologues thereof. For each target a native binding site and/or a consensus binding sequence is presented. A decoy sequence targeting the regulator may comprise the native sequence or consensus sequence presented or a variant or fragment thereof which has decoy function as described herein, e.g. a native binding site in another species. Methods for testing a variant sequence for decoy function are described in co-pending application PCT/GB2008/003353. Decoys targeting a particular regulator are particularly suitable for use in prokaryotes in which the regulator occurs, as listed below. Also listed for each regulator are examples of uses of decoys targeting the regulator, e.g. antibiotics to which a cell typically become more susceptible following treatment with a decoy.

The sequences provided herein illustrate single strands of the binding sites. However, it will be appreciated that in nature and in the TFDs of the present invention, the sequences will be double stranded. The complementary strand to the sequences listed herein are clearly and easily derivable, for example from Molecular Cloning: A Laboratory Manual (3rd Edition), 2001 by Joseph Sambrook and David Russell.

### WhiB7

WhiB7 is a transcriptional regulator of antibiotic resistance genes in Actinomycetes including Mycobacteria (e.g. *M. smegmatis* and *M. tuberculosis*) and Streptomyces (Nguyen et al, Trends in Microbiology (2006) 14:304-312).

The native WhiB7 binding site in *M*. *smegmatis* str MC2 155 comprises:

5'- CACCAGCCGA AAAGGCCACG GACCCGCAGT CACCCGGATC CGTGGCCATT
TTTGTCGGAC CCCCCGAGAA ATCTGGTCGC AGGATCCATC AGCTCAGACA
GATCAC- 3' (SEQ ID NO: 9)

LOCUS CP000480 6988209 bp DNA circular BCT 12-DEC-2006 DEFINITION Mycobacterium smegmatis str. MC2 155, complete genome.
ACCESSION CP000480
VERSION CP000480.1 GI:118168627
COORDINATES 20313637-2031742

*Uses*: increase efficacy of a broad range of hydrophilic/hydrophobic antibiotics, including macrolides, lincosamides, chloramphenicol, imipenem, pristinamycin, rifampicin, streptomycin, spectinomycin, tetracycline, isoniazid, ethambutol.

One example of a WhiB7 TFD sequence comprises:

WhiB7 TFD 5’ TGG CCA CGG ATC CGG GTG ACT GCG GGT CCG TGG CCT 3’
(SEQ ID NO: 5; Example 2)

### FadR

FadR is a regulator of expression of genes in an essential pathway for fatty acid synthesis (including fabA and fabB) in E. coli (Campbell & Cronan, J. Bacteriology (2001) 183:5982-5990)

The native FadR binding site in E. coli K12 comprises the sequence:

5' AGTAAGTTTC GAATGCACAA TAGCGTACAC TTGTACGCCG AACAAGTCCG
ATCAGCCATT TAA-3' (SEQ ID NO: 10)

LOCUS CP000948 4686137 bp DNA circular BCT 05-JUN-2008
DEFINITION Escherichia coli str. K12 substr. DH10B, complete genome.
ACCESSION CP000948
VERSION CP000948.1 GI:169887498
COORDINATES 2531419-2531481

*Uses:* Increase efficacy of any antibiotic that targets fatty acid synthesis, such as: platensimycin, platnecin derivatives, phomallenic acid, corytuberine, cyclic solfones, anthranilic acid derivatives, cerulenic acid.

One example of a FadR TFD sequence comprises

FabB TFD 5' TTT ATT CCG AAC TGA TCG GAC TTG TTC AGC GTA CAC GTG TTA
GCT ATC CTG CGT GCT TCA 3'.
(SEQ ID NO: 6; Example 3)

### YycG/YycF

YycG/YycF is a 2-component regulator in low G+C gram positive bacteria, including S. *aureus, B. subtilis, S. pneumoniae, S. pyogenes, Listeria monocytogenes.* YycG/YycF is known to be an essential regulator of at least 12 genes, including LytM and SsaA, and also genes for virulence determination and cell wall synthesis (Dubrac & Msadek, Journal of Bacteriology (2004) 186:1175-1181).

The native binding sites for YycF and YycG in *S. aureus* (in the LytM and Ssa promoters) comprise:

### YycF_LytM

5'- GCTATTTTGTAATGACAATGTAATGAGTTTAGTAAAAA-3' (**SEQ ID NO: 11**)

LOCUS CP000730 2872915 bp DNA circular BCT 16-NOV-2007 DEFINITION Staphylococcus aureus subsp. aureus USA300_TCH1516, complete genome.
ACCESSION CP000730
VERSION CP000730.1 GI:160367075
COORDINATES 322073-322109

### YycF_SsaA

5'- ATTACAAATTTGTAACAGACTTATTTTA-3' (**SEQ ID NO: 12**)

LOCUS CP000730 2872915 bp DNA circular BCT 16-NOV-2007 DEFINITION Staphylococcus aureus subsp. aureus USA300_TCH1516, complete genome.
ACCESSION CP000730
VERSION CP000730.1 GI:160367075
COORDINATES 2415067-2415093

*Uses:* Increase efficacy of macrolide-lincosamide-streptogramin B (MLSB) antibiotics

Examples of a YycG/YycF TFD sequence include:

LytM TFD 5' GCT ATT TTG TAA TGA CAA TGT AAT GAG TTT AGT AAA AA 3'
SsaA TFD 5' ATT ACA AAT TTG TAA CAG ACT TAT TTT A 3'.
(SEQ ID NOs 7 & 8; Example 4)

### Sigma 54 or Sig^{B}

Sigma 54 or Sig^{B}_is a major controller of the adaptive response and occurs in about 60% of bacteria, including *M. smegmatis, P.aeruginosa, Streptococcus pneu*moniae, *Klebsiella pneumoniae* (Wigneshweraraj, Molecular Microbioloy (2008) 68: 538-546).

The native binding sites in *S. aureus* and *K. pneumoniae* comprise:

### SA_sig

5'- TTATTATATA CCCATCGAAA TAATTTCTAA TCTTC-3' (**SEQ ID NO: 13)**

LOCUS CP000730 2872915 bp DNA circular BCT 16-NOV-2007 DEFINITION Staphylococcus aureus subsp. aureus USA300_TCH1516, complete genome.
ACCESSION CP000730
VERSION CP000730.1 GI:160367075
COORDINATES 2187051-2187017

### KP_sig

5'- CCGATAAGGG CGCACGGTTT GCATGGTTAT-3' (**SEQ ID NO: 14**)

LOCUS X13303 24206 bp DNA linear BCT 18-APR-2005 DEFINITION Klebsiella pneumoniae DNA for nif gene cluster.
ACCESSION X13303
VERSION X13303.1 GI:43820
COORDINATES 18301-18330

*Uses:* Increase efficacy of bactericidal and bacteriostatic antibiotics, for example, those described herein.

### Fur

Fur regulation was initially described as being important for iron regulation but is increasingly recognised to be involved in other adaptive pathways, most notably metalloregulatory (Zn- Zur) and also determining resistance to peroxidise (Per). Fur regulation occurs in at least *S. aureus, E. coli, Helicobacter pylori, B. subtilis* (Fur, PerR, Zur, MntR), (Horsburgh 2001; Lavarr 2003; and Delany 2001).

A consensus sequence for Fur binding in *S. aureus* and *E. coli* comprises:

### SA_fur

5'- ACT ACA AGT ACT ATT AGT AAT AGT TAA CCC TT-3' (**SEQ ID NO: 15**)

Consensus sequence ('Fur BOX') as described in Horsburgh, J. Bacteriology (2001) 183:468.

### EC_fur

5'- GATAATGATAATCATTATC-3' (**SEQ ID NO: 16**)

Consensus sequence as described in de Lorenzo, J. Mol. Biol. (1998) 283:537.

A native binding sequence in *H. pylori* comprises:

### HP_fur

5'- GTT GTC CCA TAA TTA TAG CAT AAA TGA TAA TGA AAA AGT AAA-3' **(SEQ ID NO: 17)**

LOCUS CP001072 1608548 bp DNA circular BCT 12-JUN-2008
DEFINITION Helicobacter pylori Shi470, complete genome.
ACCESSION CP001072
VERSION CP001072.2 GI:189491895
COORDINATES 691415-691374

*Uses:* Increase efficacy of antibiotics in general which may induce stress (bactericidal). Fur decoys may be used without antibiotic to perturb stress-response mechanisms and environmental adaptation.

### TcdR

TcdR is an alternative sigma factor that is autoregulated and that regulates expression of two major exotoxins that determine virulence. TcdR regulation known to occur in *Clostridium difjicile, C. botulinium* (where the homologue is BotR), *C. tetani* (TetR) and *C. perfringens* (UviA) (Matamourous, Molecular Microbiology (2007) 64:1274-1288)

A consensus binding site in *C. difficile* comprises:

### TcdR

5'- AAG TTT ACA AAA TTA TAT TAG AAT AAC TTT TTT A TT-3' (**SEQ ID NO: 18**)

Consensus sequence (TcdR, where -35 and -10 boxes are underlined) as described in Dupuy, Mol. Micro. (2006) 55:1196.

*Uses:* Increase efficacy of many antibiotics most notably those with bacteristatic properties.

### Vfr

Vfr is a global regulator of virulence factors in P. aeruginosa and E. coli (Kanack, Microbiology (2006) 152:3485-3496). The *E. coli* homolog is CRP, with binding sites occurring in the promoter of the *toxA* gene and promoter P1 of the *regA* gene.

A consensus and two native binding sites in *P. aeruginosa* are:

### PA_Vfr

5'- AAA TGT GAT CTA GAT CAC ATT T-3' (**SEQ ID NO: 19**)

Consensus sequence as described in Kanack, Microbiol. (2006) 55:1196.

### PA_ToxA

5'- CACTCTGCAA TCCAGTTCAT AAATCC-3' (**SEQ ID NO: 20)**

LOCUS EU595736 26 bp DNA linear BCT 15-JUN-2008 DEFINITION Pseudomonas aeruginosa strain PACS458 clone fa1366, complete sequence.
ACCESSION EU595736 REGION: 10145..10170
VERSION EU595736.1 GI:187939551
COORDINATES 10145-10170

### PA_RegA

5'- GTAACAGCGGAACCACTGCACAG -3' (**SEQ ID NO: 21**)

LOCUS EU595736 26 bp DNA linear BCT15-JUN-2008 DEFINITION Pseudomonas aeruginosa strain PACS458 clone fa1366, complete sequence.
ACCESSION EU595736 REGION: 10145..10170
VERSION EU595736.1 GI:187939551
COORDINATES 312-334

*Antibiotics:* Decoys may be used without antibiotic to control the expression of a set of genes that determine virulence in *P.aeruginosa* and *E. coli.* Decoys may be used to increase the efficacy of antibiotics most notably those with bacteristatic properties.

### NtrC

NtrC is a regulator of an essential gene, glnA, which is required for environmental adaptation in at least *Klebsiella pneumoniae* (Minchin et al, The EMBO Journal (1989) 8:3491-3499).

A native binding site in *K. pneumoniae* comprises:

### KP_NtrC

5'- GCTTTGCACTACCGCGGCCCATCCCTGCCCCAAAACGATCGCT -3' (**SEQ ID NO: 22**)

LOCUS X 13303 24206 bp DNA linear BCT 18-APR-2005 DEFINITION Klebsiella pneumoniae DNA for nif gene cluster.
ACCESSION X 13303
VERSION X13303.1 GI:43820
COORDINATES 18159-18201

*Uses:* Decoys may be used without antibiotic to perturb expression of the essential gene, *glnA,* required for environmental adaptation, or in combination with antibiotics in general which may induce stress (bactericidal).

### ArsR

ArsR is a regulator of genes encoding acid adaptation in at least *Helicobacter pylori, H. acireonychis* and *H. felis* (Pflock et al, J. Bacteriology (2006) 188:3449-3462). Binding sites occur in the amiE promoter and the rocF promoter as described in Pflock *et al.*

Examples of a native binding sequence comprise:

### HP_AmiE

5'- ATAATCATAA TGATTAAAGT TTTCATATTC ATTATAAATC CGTTTACACA
ATTATT -3' (SEQ ID NO: 23)

LOCUS AE000511 56 bp DNA linear BCT 27-DEC-2005 DEFINITION Helicobacter pylori 26695, complete genome.
ACCESSION AE000511 REGION: 310910..310965
VERSION AE000511.1 GI:6626253
COORDINATES 310910-310965

### HP_RocF

5'- GAAATTGTTC TATTTATTAT CCATTTGCTT ATTAATAATT GGTTGTTAAT
TTTGGTTTAG A -3' (SEQ ID NO: 24)

LOCUS AE000511 61 bp DNA linear BCT 27-DEC-2005 DEFINITION Helicobacter pylori 26695, complete genome.
ACCESSION AE000511 REGION: 1459830..1459890
VERSION AE000511.1 GI:6626253
COORDINATES 1459830-1459890

*Uses:* Decoys may be used on their own to perturb expression of the essential genes mediating iron-uptake that are required for environmental adaptation, or in combination with antibiotics in general which may induce stress (bactericidal).

### Glycopeptide-resistant consensus sequence (GISA)

A consensus sequence was determined by bioinformatical analysis of non-proprietary microarray data. Motifs were found in genes shown to be up-regulated when comparing a glycopeptide resistant strain with its parent, and the same resistant strain with a revertant (Scherl, BMC Genomics (2006) 7:296). 17/22 promoters were found to have a shared consensus sequence.

An example of the consensus sequence, found in the promoter of *tcaA,* a known positive regulator of virulence (Maki, Antimicrobial Agents Chemother. (2004) 48:1953) may be used in a decoy (see below).

### SA_TcaA

5'- TGAACACCTTCTTTTTA -3' (**SEQ ID NO: 25**)

LOCUS CP000730 2872915 bp DNA circular BCT 16-NOV-2007 DEFINITION Staphylococcus aureus subsp. aureus USA300_TCH1516, complete genome.
ACCESSION CP000730
VERSION CP000730.1 GI:160367075
COORDINATES 2476452-2476436

The consensus sequence occurs at least in *S. aureus.* Decoys targeting the sequence may be used to target any of the regulatory proteins binding the sequences in the native promoters. Typically the genes regulated by binding at the native sequences contribute to glycopeptides resistance and/or virulence.

*Uses:* decoys may be used to increase efficacy of glycopeptides antibiotics.

### AgrA

A consensus sequence was determined by bioinformatical analysis of non-proprietary microarray data. Motifs were found in genes shown to be positively regulated by Agr, a regulator associated with virulence (Dunman, J. Bacteriol. (2001) 183:7341).

The putative motif was found to occur in two promoters thought likely to be involved in determining pathogenicity (SA2093 upstream of an AraC-like transcriptional regulator and SA1269, a Bit-like gene).

### SA_Agr_2093

5'- AGA AAG ACA AAC AGG AGT AA -3' (**SEQ ID NO: 26**)

LOCUS CP000730 2872915 bp DNA circular BCT 16-NOV-2007 DEFINITION Staphylococcus aureus subsp. aureus USA300_TCH1516, complete genome.
ACCESSION CP000730
VERSION CP000730.1 GI:160367075
COORDINATES 2414790-2414771

### SA_Agr_1269

5'- GAA GAA ACA AAA AGC AGC AT -3' (**SEQ ID NO: 27**)

LOCUS AP009324 2880168 bp DNA circular BCT 09-JAN-2008 DEFINITION Staphylococcus aureus subsp. aureus Mu3 DNA, complete genome.
ACCESSION AP009324
VERSION AP009324.1 GI:156720466
COORDINATES 1549580-1549561

The binding motif occurs at least in *S. aureus* and contains homologues in many Gram-negative species, such as *P. aeruginosa* and *E. coli.* Decoys targeting the motif may be used to target any regulatory protein binding the motif in the native promoters. Typically the genes regulated by binding at the native sequences determine virulence in the cell.

*Uses:* Decoys may be used to control the expression of a set of genes that determine virulence in *P. aeruginosa* and *E. coli.*

Generally, the decoy sequence (transcription factor binding site) in the polynucleotide is not operably linked to a gene. The transcription factor binding site may be isolated from any other elements of a cognate promoter.

The decoy polynucleotide may comprise a plasmid vector. For example the decoy polynucleotide may comprise an n[snare] plasmid as described in co-pending application PCT/GB2008/003353 and/or prepared according to a method described in co-pending application PCT/GB2008/003353.

The decoy polynucleotide may comprise more than one copy of the decoy sequence. The polynucleotide may comprise a multimeric molecule comprising multiple copies of the decoy sequence. For example, from 1 to 1000 copies. Typically there are two or more copies, for example, 2-1000 copies, e.g. at least 10, 20, 30, 40, 50, 60, 70, 80, 90, 100, 200, 300, 400, 500, 600, 700, 800 or 900 copies. For example, there may be 10-200, 10-150, 20-120, 20-100, 30-100, 30-80, 30-50, 30-40 copies. For example, there may be 30 copies of the decoy sequence. Typically there are multiple copies of the decoy, for example, multiple direct repeats of the decoy sequence.

Alternatively, the decoy polynucleotide may comprise more than one decoy sequence as well as multiple copies of the decoy sequences.

The decoy polynucleotide may comprise additional sequences to the decoy sequence. Typically the additional sequence results in increased resistance to degradation of the decoy sequence due to the action of exo- and/or endonucleases. The decoy polynucleotide may comprise at least one element of secondary structure. Typically this secondary structure results in increased resistance to degradation of the decoy sequence due to the action of exo- and/or endonucleases. The decoy polynucleotide may comprise modified bases or sugars to confer greater nuclease resistance. The decoy polynucleotide may comprise 2' OH nucleotides or amines at the termini of the polynucleotide to reduce or inhibit exonuclease activity. The decoy polynucleotide may comprise a linear oligonucleotide. The decoy polynucleotide may comprise circular double stranded DNA, e.g. a so-called dumbbell configuration (Ahn et al, Biochemical Biophysical Res. Comm. (2003) 310:1048-1053). A dumbbell typically has a double stranded region incorporating the targeted sequence bracketed by small stem loop structures, or other sequences. The decoy polynucleotide may comprise a cholesterol modification at one or at each 5' end of the molecule.

The decoy polynucleotide may comprise any one or more of the above features in any suitable combination.

A decoy polynucleotide may comprise any of the decoy sequences described herein and/or identified according to the methods described in co-pending application PCT/GB2008/003353, and may also comprise additional sequence as described.

As above, a decoy polynucleotide may comprise an n[snare] plasmid. The plasmids have the advantage, e.g. compared to linear oligonucleotides, that they are easily introduced into bacteria, can be maintained by positive selection, and circumvent the problem of exonuclease degradation.

The advantages of creating plasmid-borne versions of decoy oligonucleotides include reduced cost of manufacture, increased resistance to in vivo degradation by nucleases, maintenance of plasmid concentration (by self-replication and positive selection if necessary), broad host range, and the ability to test combinations or cis-regulatory sequences using distinct but compatible n[snare] plasmids (to test for synergistic effects).

An n[snare] plasmid is suitable for use as a decoy polynucleotide in the present methods. n[snare] plasmids and libraries are also suitable for testing possible decoy function of a known or putative cis-regulatory sequence, or for screening for new cis-regulatory sequences (which may act as decoy sequences).

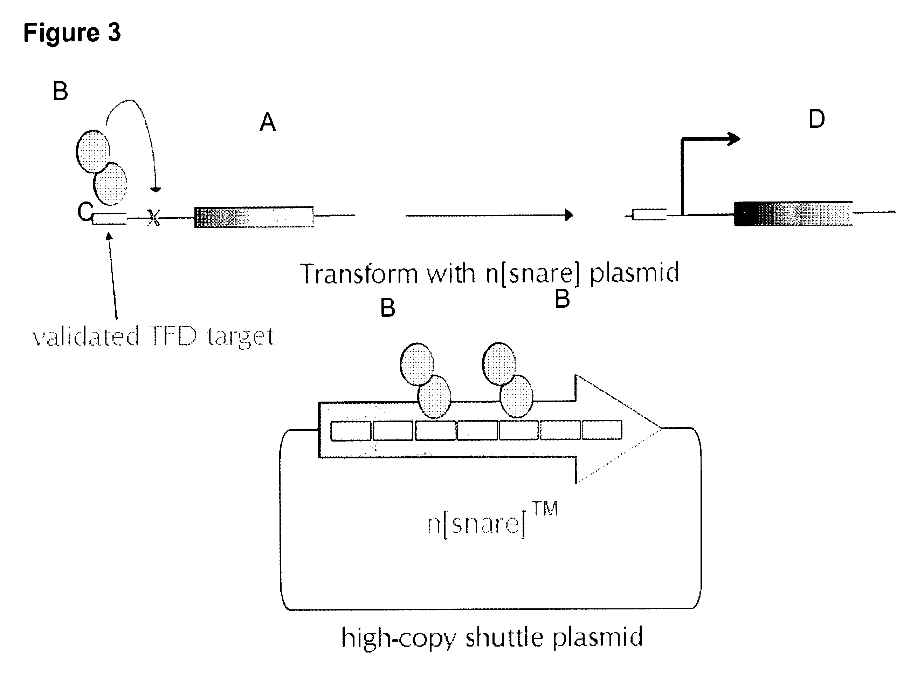

The principle of use of an n[snare] plasmid is illustrated in Figure 3. The plasmid comprises a "snare" sequence (shown in multiple copies in the Figure). If the "snare" comprises a transcription factor binding site which competes with a cellular cis-regulatory sequence for binding to a transcription factor (i.e. has decoy function), introduction of the n[snare] plasmid to the cell will result in a titration of transcription factor off the cellular *cis-*regulatory sequence and onto the snare. This can be detected as a change in expression of a gene or gene(s) whose expression is regulated in the cell by that cis-regulatory sequence, or by alteration in the phenotype of the cell.

In general an n[snare] plasmid comprises a plasmid vector and an insert sequence (the insert comprising the snare). Incorporating the snare sequence in a plasmid addresses the problems of decoy degradation in the art, and allows stable maintenance of the decoy (and any affect on gene expression) in the cell.

The insert sequence comprises one or more copies of a monomer sequence (which comprises the snare). Thus the insert may comprise (for example) from 1 to 200 monomer sequences. Typically there are two or more copies, for example, 2-200 copies, e.g. at least 10, 20, 30, 40, 50, 60, 70, 80, 90, 100, 110, 120, 130, 140, 150, 160, 170, 180, or 190 copies. For example, there may be 5-200, 5-150, 5-100, 10-150, 10-50, 5-50, 5-40, 10-40, 5-30 copies. For example, there may be 30 copies of the monomer sequence. The plasmid typically comprises a homopolymer of the monomer. Typically they are multiple copies of the monomer, for example, multiple direct repeats of the monomer sequence. Providing multiple copies of the monomer (and thus of the snare) increases the titrating power of the decoy.

The monomer sequence comprises the snare sequence.

Typically a transcription factor binding site in a snare is not operably linked to a gene, e.g. in the snare or snare plasmid. In that sense the binding site is isolated from its cognate gene or genes. A binding site in a snare may also be isolated from other elements in its cognate promoter. In one instance, the monomer sequence does not comprise a gene.

A monomer may comprise additional sequence in addition to the snare. Often such additional sequence derives from the method used to produce the snare and/or the plasmid insert. For example, a monomer may comprise an adaptor sequence, such as the adaptor sequence which typically results when "custom" snares are produced for a custom n[snare] library according to the methods herein. An adaptor sequence may comprise, for example, recognition and/or cutting sites for one or more restriction enzymes.

A monomer may comprise nucleotide sequence which provides a binding site for a primer, e.g. a primer used in production of the monomer or of an insert comprising multiple monomers.

For example, when a plasmid insert is prepared using a rolling circle amplification method as described in co-pending application PCT/GB2008/003353, a monomer typically comprises a segment which corresponds to the binding site for the primer used in rolling circle replication, e.g. a T7 primer.

A monomer comprising a randomised snare sequence typically also comprises a region or regions of constant sequence. For example, a randomised snare sequence of n nucleotides may be flanked by regions of constant sequence. Alternatively, a central core of constant sequence may be flanked by regions of randomised nucleotide sequence.

The length of a monomer can be, for example, up to 1000 nucleotides, for example, up to 900, 800, 700, 600, 500, 400, 300, 200, 100, 90, 80, 70, 60, 50, 40, 30, 20, 15, or 10 nucleotides. Typically the length of the monomer is in the range of, for example 10-100, 10-50, 20-75, 30-60, 30-50, 35-55 such as 35-54 nucleotides. For example, the length of a monomer may be 30, 40 or 50 nucleotides.

A snare portion of a monomer may typically range in size from 10-30 nucleotides, for example, 10-25, 10-20, 15-20, such as 15, 16, 17, 18, 19 or 20, for example 19 nucleotides.

An adaptor sequence may comprise, for example, 5-30 nucleotides, for example, 5-25, 5-20, 5-15 or 5-10 nucleotides such as 10, 11, 12, 13, 14 or 15 nucleotides.

Typically, an insert in the n[snare] plasmid comprises one or more copies of a monomer as described herein. Where the insert comprise multiple repeats of a monomer, these may be tandem repeats.

An insert in the plasmid vector may comprise, for example about 1.5kb, for example 1-2 kb, for example 1.1, 1.2, 1.3, 1.4, 1.5, 1.6, 1.7 or 1.8kb. However, any suitable insert size, which allows stable maintenance of the plasmid in a suitable host cell and efficient use in the present methods may be used.

Typically the sequences of all monomers in a single plasmid are the same.

The plasmid vector for use in the n[snare] plasmid may be suitable for use in a prokaryotic or eukaryotic host. For example, the vector may be for use in a prokaryotic such as a bacterial, e.g. actinomycete host. For example, the plasmid vector may be suitable for use in a *Streptomycete* or *E. coli* strain, e.g. one or more of *Streptomyces coelicolor* e.g. A3(2) (or strain M145 or M600), *E. coli, Streptomyces lividans* or *Streptomyces cinnamoneous.* Suitable hosts are described further herein.

Typically, the vector is a broad host range and/or shuttle vector and can therefore be maintained and propagated in more than one host. The plasmid may a conjugative plasmid. This allows easy transfer from one cell to another by conjugation.

Preferably the plasmid is self-replicating. Typically the plasmid is a high copy number plasmid. For example, the plasmid may be maintained at, for example, 20-100 copies per cell, for example 20, 30, 40, 50, 60, 70, 80, 90 or 95 or 100 copies per cell. High copy number increases titrating power of a decoy sequence in the snare.

Typically an n[snare] plasmid comprises an origin of replication. Suitable origins are known in the art. Typically the plasmid additionally comprises one or more detectable marker genes, for example, one or more genes encoding antibiotic resistance, e.g. the *aac* gene encoding apramycin resistance. Expression of the marker gene(s) allows screening for maintenance of the plasmid in a host cell.

Examples of suitable plasmid vectors include, for example, pIJ86. Suitable vectors are known in the art.

A snare may comprise a sequence derived or isolated from a genome or genomic fragment. A genomic fragment may comprise a gene or genes encoding a particular function or phenotype of interest. A snare may comprise, for example, a sequence derived or isolated from the promoter region of such gene(s) or surrounding sequences, e.g. sequence at a greater distance from a gene(s) than the promoter. A snare may comprise a sequence that competes with such a sequence for transcription factor binding, as described herein. Methods for preparing such snares are described herein and in co-pending application PCT/GB2008/003353.

Decoy polynucleotides may be prepared by any suitable method.

For example, dumbbells may be prepared as linear oligonucleotides and then ligated with T4 ligase. Alternatively dumbbell decoys may be prepared by PCR using appropriate primers, as described in Example 1. Each primer generally contains a portion which will form the stem loop of the dumbbell structure. Examples of such primers are given in Example 1. PCR amplification using the primers is typically followed by restriction digest of the amplification product and ligation to form the closed circle dumbbell.

Alternatively, dumbbells can be prepared by restriction digest of a plasmid as described in Example 1. Digestion is followed by ligation to form the closed circle dumbbell structure.

Methods for preparing n[snare] plasmids and n[snare] plasmid libraries are described herein and in co-pending application PCT/GB2008/003353.

The present methods may comprise the introduction of more than one decoy sequence into a cell. For example, a cocktail of decoy sequence may be used, comprising variant sequences for binding of a regulator in difference strains, e.g. different clinical strains. Decoy sequences may be present in the cocktail in the same proportions that the different strains occur in the clinic. In another example, more than one regulator may be targeted.

In one aspect the methods described herein comprise *in vitro* methods.

A host cell as referred to herein may be one in which it is desired to alter gene expression (and phenotype) using a decoy molecule, e.g. a pathogenic bacteria.

Typically, where the method is used to alter a cell phenotype, the host cells display the phenotype in the absence of the decoy. Decoys for alteration of a medically or therapeutically relevant phenotype, e.g. for increasing antibiotic sensitivity, may be screened in a series of cells. For example, decoys may be tested first in a bacterial model of the phenotype, e.g. a model of antibiotic resistance, then further validated in e.g. a pathogen or clinical isolate. Decoys may be further validated in an animal model, e.g. a mouse model.

Where a decoy is a plasmid, typically the host cell is compatible with the plasmid vector and/or is one in which the plasmid can be stably maintained and replicated. The host cell is also typically compatible with any detectible marker gene in the plasmid.

In general, a host cell comprises the cis-regulatory sequence of interest, i.e. the *cis-*regulatory sequence that is being screened for, or with which the decoy sequence introduced into the cell is intended to compete, operably linked to a gene or genes. Modulation of expression of the gene or genes can be detected, directly or indirectly (e.g. by an alteration in phenotype).

Typically the cell comprises a promoter containing the cis-regulatory sequence, operably linked to the gene(s). By operably linked is meant that the cis-regulatory sequence and/or promoter is linked to the gene or genes in such a way that the sequence and/or promoter can function (under appropriate conditions, e.g. presence of the requisite transcription factor(s)) to regulate expression of the gene(s). Thus, when bound by the cognate transcription factor, the cis-regulatory sequence functions to regulate (repress or activate) expression of the gene or genes.

Functioning of the cis-regulatory sequence in the cell can be determined by monitoring for expression of the linked gene(s). This may be done by monitoring for expression of the gene directly, or by monitoring for expression of a particular phenotype which is associated with expression of the gene(s). For example, screening for function may comprise screening for resistance to one or more antibiotics, for stress response, for expression of virulence factors or for expression of essential genes. Methods for screening are described in co-pending application PCT/GB2008/003353.

A host cell may comprise a cis-regulatory sequence (e.g. a promoter containing the sequence) operably linked to its native (cognate) gene(s), i.e. linked to the gene or genes the expression of which the sequence (or promoter) regulates in its native occurrence. The *cis-*regulatory sequence and the regulated gene(s) may be endogeneous to the cell, e.g. genes encoding an intrinsic resistance mechanism in an antibiotic resistance pathogenic bacterium.

Suitable host cells are known in the art and are described herein in the present Examples.

The present methods are suitable for use in prokaryotes generally. In particular the methods may be used in pathogens, especially pathogenic bacteria, for example, pathogenic bacteria affecting humans. These include bacteria of the following genuses (listed according to result in the Gram stain test):
Gram negative: Acinetobacter; Bordetella; Borrelia; Brucella; Campylobacter; Escherichia; Francisella; Haemophilus; Helicobacter; Klebsiella; Legionella; Leptospira; Neisseria; Proteobacteria; Pseudomonas; Rickettsia; Salmonella; Shigella; Treponema; Vibrio; Yersinia.

Gram positive: Bacillus; Clostridium; Corynebacterium; Enterococcus; Listeria; Mycobacterium; Staphylococcus; Streptococcus.

Unstained: Chlamydia; Mycoplasma.

Examples of gram negative pathogenic species and of disease caused by them include: A. barumannii (Pneumonia, Bacteremia), Bordetella pertussis (whooping cough), Borrelia burgdorferi (Lyme's disease), Brucella abortus (Brucellosis), Campylobacter jejuni (Acute enteritis), Escherichia coli (septicemia and pneumonia), Francisella tularensis (Tularemia), Haemophilus influenzae (Influenza), Helicobacter pylori (Peptic ulcers), Klebsiella pneumoniae (pneumonia), Legionella pneumophilla (Legionnaire's disease), Neisseria gonorrhoeae (Gonorrhoeae), Acinetobacteria spp (Noscomia; infections), Pseudomonas aeruginosa (sepsis), Rickettsia rickettsii (Ricketts), Salmonella typhimurium (Typhoid), S. dysenteriae (Dysentery), Vibrio cholerae (Cholera) Yersinia pestis (plague).

Examples of gram positive pathogenic species and of disease caused by them include: Bacillus anthracis (Anthrax), Clostridium difficile (Pseudomembranous colitis), Corynebacterium diptheriae (Diptheria), Enterococcus faecalis (Noscomial infections), Listeris monocytogenes (Listerosis), Mycobacterium tuberculosis (Tuberculosis), Staphylococcus aureus (Septicemia), S. pneumoniae (Pneumonia).

Bacteria of the following genuses (including the species referred to above) are particularly suitable for with the present methods: Escherichia; Helicobacter; Klebsiella; Neisseria; Proteobacter; Pseudomonas; Salmonella; Bacillus; Clostridium; Enterococcus; Staphylococcus; Shigella.

In one aspect the invention relates to a host cell or cells comprising a decoy polynucleotide (decoy molecule) as described herein (e.g. introduced to the cell by any method herein). In particular, the invention relates to such a host cell(s) which display altered gene expression and/or phenotype, due to the presence of the decoy polynucleotide, e.g. increased susceptibility to antibiotic(s), decreased virulence, impaired adaptive response, compared to the cell in the absence of the plasmid/decoy molecule. For example, the invention relates to pathogens or clinical isolates which have been rendered less viable using decoy polynucleotides as described herein.

Decoy polynucleotides may be introduced to host cells by any suitable means. For example, transformation, transfection, conjugation. For example, where a polynucleotide comprises a conjugative plasmid, this may be introduced by conjugation. Decoy polynucleotides, e.g. circular dumbbell structures, may be introduced to cells by transfection. Cells may be in liquid culture and the decoy added to the liquid. Alternatively, cells may be cultured on solid media and decoys transfected from absorbent paper discs saturated with decoy and overlaid on the media. Typically, decoy polynucleotide is added to the culture medium and taken up by cells. Where cells are cultured on solid media, decoy polynucleotides may be added to a filter disc and taken up by cells from the disc. A permeability buffer may be used to aid transfection.

In one aspect, transfection of decoy polynucleotides may comprise the use of cholesterol. In particular, the methods may use linear decoy polynucleotides, bearing a cholesterol modification at one or both 5' ends. The modification is believed to facilitate uptake by the cells.

Decoys may additionally be labelled, e.g. at a 5' end with a detectable label such as a fluorescence dye, e.g. Cy5. This will facilitate monitoring of uptake and maintenance in the cell.

Cholesterol and/or detectably labelled decoys may be prepared using cholesterol and/or detectable labelled primers, as described in Example 1.

Transfection of decoy polynucleotide into a cell may comprise use of R9-cholesterol, which consists of a cholesterol molecule attached to a linear chain of nine D-arginines (Kim W.J. et al, Mol. Ther. (2006) 14:343-350).

In general, the uptake and/or maintenance of the decoy polynucleotides, or plasmid library in the cells is monitored. For example, a plasmid decoy may comprise a detectable marker e.g. encoding antibiotic resistance, which allows positive selection for the presence of the plasmid and monitoring of plasmid propagation. Presence of a decoy polynucleotide can also be monitored by qrt-PCR.

In general a decoy sequence comprises a binding site for a transcription factor that competes with a cellular transcription factor binding site for binding of a transcription factor. By titrating transcription factor from the cellular site, the decoy disrupts expression of the gene or genes operably linked to the binding site in the cell.

A decoy sequence may comprise the sequence of a native cellular binding site for a transcription factor. Native sequences are available in the art and examples of endogenous binding sequences are presented herein for specific regulators.

A decoy sequence may comprise a consensus binding site for a given regulator. Again, such consensus sites may be available in the art, and examples are given for some specific regulators herein.

Alternatively a decoy sequence may comprise a variant of a native or consensus binding sequence, which retains decoy function.

A variant may be prepared by altering, for example, 1, 2, 3, 4, 5, 6, 7, 8, 9, 10 or more nucleotides in the parent sequence. For example, footprinting experiments may indicate that particular nucleotides are less crucial for transcription factor binding and might be altered.

A putative decoy sequence can be tested for ability to compete with a given transcription factor binding site by introducing a decoy polynucleotide comprising the decoy sequence into a suitable host cell. The host cell includes the target transcription factor binding site operably linked to a gene or genes whose expression can be determined directly or indirectly, e.g. by screening for a change in phenotype. Decoy function of a test sequence can be determined by screening for a change in expression of the gene(s) or a change in the phenotype.

Any of the decoy polynucleotides described herein may be used. Any of the host cells described herein may be used.

For example, a test sequence may first be introduced into a suitable reporter cell and expression of a reporter gene monitored to assess decoy function. The generic reporter-based assay systems using "dead or alive" reporter cells described in PCT/GB2008/003353 may be used. This approach solves the problem of needing a scoreable phenotype as under normal circumstances each reporter causes cell death, it is only when an decoy relieves this by titrating off the transcription factor that the cells can grow. Hence selection for both reporters relies on cell survival, which greatly expedites the screening process. Another feature of the system is that such dead-or-alive screens can be readily automated as a basis of rapid, comprehensive or high-throughput screens. It is also possible to identify regulatory sequences controlling promoters which in their natural context have no easily scoreable phenotype.

Alternatively or additionally, a decoy sequence may be tested in host cells comprising the target binding site operably linked to its cognate gene(s), so that ability to disrupt expression of the gene(s) can be determined. Where a decoy is intended to produce a change in phenotype, the host cell used in screening displays the particular phenotype.

For example, decoy sequences for targeting regulators described herein may be tested by screening for increased sensitivity of the host cell to antibiotic. The antibiotic may be chosen as described herein.

In some cases, decoy sequences may be tested by screening for another phenotype appropriate to the target regulator. For example, decoys targeting regulators of stress response can be assessed by detecting impaired stress response (as measured by tolerance to physiological shock). Decoys targeting regulators of virulence factors can be assessed by determining an impaired virulence programme. An example of the latter could be low viability following re-suspension from a frozen vial or even the down-regulation of a specific gene (as measured by qRT-PCR).

For example, a FabB decoy which targets the regulation of genes essential for fatty acid synthesis may be tested against suitable strain, e.g. *E. coli* grown in e.g. Luria-Bertani LB media (1% [w/v] Bacto-tryptone, 0.5% [w/v] Yeast extract, 1% [w/v] NaCl) in the presence of an antibiotic which acts on the process of fatty acid synthesis, e.g. Cerulenin. For example, at concentrations of Cerulenin of 10 µg/ml, untreated bacteria grow normally and act as a comparator for bacteria treated with the decoy, which grow markedly more slowly and to lower final density.

For example, stress response can be induced in a suitable strain e.g. *S. aureus* by growing in, e.g. LB media under conditions of stress, e.g. alkali conditions. For example LB media containing KOH, e.g. at 30 mM may be used. Untreated bacteria grow well in this media, due to the induction of the stress response by the alkali conditions, whereas those treated with the SA_sigB decoy, targeting the regulation of *sigB* which is a major part of the regulatory response to stress, grow poorly.

In some instances, screening for expression of the relevant gene or genes comprises determining host cell viability under suitable culture conditions. For example, when the phenotype being assessed is antibiotic resistance, screening may comprise culturing cells in the presence of the antibiotic and determining whether the cells are viable. In a screen comprising the "dead or alive" reporter host cells, screening the cells comprises culturing the cells under conditions in which expression of the reporter gene(s) (the gene(s) operably linked to the cis-regulatory sequence of interest) is determinative for host cell viability, and isolating viable cells.

The present methods may comprise culture of the host cells in liquid media, as described herein, e.g. if the cell phenotype which is being determined is cell viability. This may have the advantage that only a small number of cells remain to be analysed.

An n[snare] plasmid as described herein may be prepared by a method comprising:
- providing a polynucleotide comprising one or more copies of a monomer sequence; and
- cloning the polynucleotide into a suitable plasmid vector.

The composition of the monomer is as described herein for the n[snare] plasmid. Suitable plasmid vectors have also been described.

The polynucleotide comprising one or more copies of a monomer sequence may be provided by a method comprising:
(1) providing a circular oligonucleotide comprising:
   (i) a sequence of interest; and
   (ii) a binding site for a primer suitable for use in rolling circle amplification;
   wherein the monomer sequence comprises (i) and (ii); and
(2) performing rolling circle amplification using the circular oligonucleotide as a template, thereby providing a polynucleotide comprising repeats of the monomer sequence.

Step (1) of the method may further comprise amplifying the rolling circle amplification products by PCR and isolating polynucleotide fragments of the required size, for example, fragments comprising 30-50 repeats of the monomer sequence. This can be done by, e.g PAGE analysis.

A circular oligonucleotide may be prepared by a method comprising:
- providing a linear single stranded oligonucleotide comprising: the test sequence of interest (i) and the binding site for a primer suitable for use in rolling circle amplification (ii);
- circularising the oligonucleotide, e.g. using Taq ligase, typically in the presence of a universal joining oligonucleotide;
- optionally digesting remaining linear DNA with an exonuclease; and
- recovering monomeric circular oligonucleotides, e.g. using PAGE.

Primers suitable for use in rolling circle amplification are known in the art. For example, a T7 primer may be used.

Methods for carrying out rolling circle amplification are known in the art. For example, *Bst*I polymerase may be used.

In one example, PCR amplification of the rolling circle amplification products is carried out using the same primer that was used for rolling circle amplification, e.g. T7 primer.

In general the sequence (i) in the monomer comprises a snare sequence as described herein.

As described herein, snare sequences may be isolated from genomic DNA, e.g. from an entire genome, or from a genomic fragment. A snare sequence, once isolated, may be used to form a n[snare] plasmid by the method described in co-pending application PCT/GB2008/003353.

A protocol for preparation of snare sequences from a genome or genomic fragment is described in PCT/GB2008/003353

As described herein, a snare sequence may comprise randomised nucleotide sequence. Typically the snare comprises a randomised (or variable) nucleotide sequence of "n" nucleotides in length. In general n may range from 5-50, for example 10-50, for example 20-40 e.g. 25-35 e.g. 6, 7, 8, 9, 10, 11, 12, 13, 14, 15, 16, 17, 18, 19 or 20 nucleotides.

An oligonucleotide comprising the randomised sequence (and optionally constant sequence as described) may be synthesised using methods known in the art.

A nucleotide bias may be introduced into randomised (or variable) region of the oligonucleotide. For example, a GC bias may be introduced if appropriate.

The monomer in the method above may comprise an oligonucleotide prepared in this way. An n[snare] plasmid may then be prepared.

As described, one aspect of the invention is to increase susceptibility of prokaryotes to antibiotics. For example, this may involve targeting an intrinsic resistance mechanism in the cell, or cellular adaptive responses which mean that the cell is less able to survive under conditions of stress induced by the antibiotic.

In one aspect, the present methods are suitable for increasing the efficacy of antibiotics generally (Alekshun and Levy, Cell (2007) 128:1037-1050). This includes both bacteristatic and bactericidal antibiotics. Examples include the class of antibiotics known as aminoglycosides (such a kanamycin); the carbapenems (such as meropenem); the cephalosporins (such as cefepime); the glycopeptides (such as vancomycin and daptomycin); the penicillins such an ampicillin, carbenicillin and penicillin); the polypeptide antibiotics (such as polymixcin B); the quinolines (such a levaquin); the sulfonamides (such a Bactrim); the tetracyclines (such as tetracycline); and variously, chloramphenicol, rifampicin and Zyvox.

In one aspect, methods which cause suppression of the stress response in a cell, or which cause the cell to become stressed are particularly suitable for potentiating the effects of bactericidal antibiotics (Kohanski et al, Cell (2007) 130:797-810).

Bactericidal antibiotics may include:
β-lactams
   (a) Penicillins
   (b) Carbapenems
   (c)Monbactams
   (d) Cephalosporins

First generation: Cefacetrile (cephacetrile), Cefadroxil (cefadroxyl; Duricef). Cefalexin (cephalexin; Keflex), Cefaloglycin (cephaloglycin), Cefalonium (cephalonium), Cefaloridine (cephaloradine), Cefalotin (cephalothin; Keflin), Cefapirin (cephapirin; Cefadryl), Cefatrizine, Cefazaflur, Cefazedone, Cefazolin (cephazolin; Ancef, Kefzol), Cefradine (cephradine; Velosef), Cefroxadine, Ceftezole;

Second generation: Cefaclor (Ceclor, Distaclor, Keflor, Raniclor), Cefonicid (Monocid), Cefprozil (cefproxil; Cefzil), Cefuroxime (Zinnat, Zinacef, Ceftin, Biofuroksym), Cefuzonam, Cefmctazole, Cefotetan, Cefoxitin, Carbacephems: loracarbef (Lorabid), Cephamycins: cefbuperazone, cefmetazole (Zefazone), cefminox, cefotetan (Cefotan), cefoxitin (Mefoxin)

Third generation: Cefcapene, Cefdaloxime, Cefdinir (Omnicef), Cefditoren, Cefetamet, Cefixime (Suprax), Cefmenoxime, Cefodizime, Cefotaxime (Claforan), Cefpimizole, Cefpodoxime (Vantin, PECEF), Cefteram, Ceftibuten (Cedax), Ceftiofur, Ceftiolene, Ceftizoxime (Cefizox), Ceftriaxone (Rocephin), Cefoperazone (Cefobid), Ceftazidime (Fortum, Fortaz), Oxacephems: latamoxef (moxalactam)

Fourth generation: Cefclidine, Cefepime (Maxipime), Cefluprenam, Cefoselis, Cefozopran, Cefpirome, Cefquinome, Oxacephems: flomoxef
Yet to be classified: Ceftobiprole, Cefaclomezine, Cefaloram, Cefaparole, Cefcanel, Cefedrolor, Cefempidone, Cefetrizole, Cefivitrit, Cefmatiten, Cefmepidium, Cefovecin, Cefoxazole, Cefrotil, Cefsumide, Ceftaroline, Ceftioxide, Cefuracetime

### Aminoglycosides:

(a) amikacin, gentamicin, kanamycin, neomycin, netilmicin, paromomycin, rhodostreptomycin, streptomycin, tobramycin, apramycin;
(b) Anthracyclines, e.g. doxorubicin

### Quinolones:

### (a) Fluoroquinolones;

First generation;
Second generation;
Third generation;
Fourth generation.

### Glycopeptide antibiotics:

(a) vancomycin, teicoplanin, telavancin, bleomycin, ramoplanin, decaplanin and dalbavancin.

### Peptide antibiotics:

(a) Lantibiotics;
   Duramycin, nisin, epidermin, actagardine, microbisporicin and mersacidin;
(b) Lipopeptides;
Cubicin.

### Streptogramins:

### Quinupristin +dalfopristin (Synercid®).

However, the present decoys and methods may also be used to increase efficacy of bacteriostatic antibiotics, such as Macrolides, Ketolides, Tetracyclines, Lincosamides (e.g. clindamycin), Oxazolidinones (Linezolid).

Where the present methods are used in therapy, a decoy polynucleotide may be applied in combination with one or more antibiotics, in general antibiotic(s) to which the cell is rendered more susceptible by the decoy. Antibiotics may be chosen from those described above.

In some instances, it is appropriate to select antibiotic(s) for use according to the bacterial species, and the bacterial infection which is being targeted.

For example, *M*. *tuberculosis* is the causative agent of tuberculosis which is currently treated using: isoniazid, rifampicin, ethambutol, pyrazinimide. Therefore, in a method targeting *M*. *tuberculosis,* one or more of these drugs could be selected for use with a decoy polynucleotide. Similarly, *S*. *aureus* infections are typically treated with penicillins, Gram-negative infections with bacteria producing extended spectrum β-lactamases e.g. *E. coli* or *K. pneumoniae*, are often treated with β-lactams.

In some instances, the type of decoy polynucleotide and the genes whose expression it disrupts in the cell will determine the type of antibiotic(s) that the cell becomes more sensitive to (and so the antibiotic(s)) which may be used in combination with the decoy. For example, the FabB TFD described herein, targets a binding site for a regulator (FadR) of essential enzymes required for fatty acid synthesis. Thus, a cell treated with the decoy becomes more susceptible in particular to antibiotics which inhibit fatty acid synthesis. Further information for specific targets is provided herein.

The present decoys may be used to alter cellular adaptive or physiological responses, e.g. any of the responses described herein. Decoys may also be used to alter expression of essential genes and therefore alter the viability of the cell under conditions where the gene(s) is essential. Decoys may also be used to alter the virulence of the cell.

Decoys may also be used to increase the uptake of antibiotic, for example by causing changes to the composition of the cell wall or membrane.

The decoys may also target regulation of unknown genetic systems, in particular those decoys discovered by the n[snare] process described in PCT/GB2008/003353. In these examples the consequence of the treatment of the bacteria with decoys may be measured, for example antibiotic susceptibility, but the genetic mechanism underpinning that change in susceptibility remain unknown.

In some cases treatment with decoys targeting regulation of known genes, such as WhiB7 and its homologues in mycobacteria, increase the susceptibility of the cell to antibiotics but the phenotypic mechanism underpinning that change remains unknown.

In some cases decoys targeting regulation of genes implicated in determining antibiotic sensitivity by bioinformatic methods, such as those identified by transcriptional profiling to be involved in glycopeptide resistance in *S. aureus* and a proportion of which found to have common cis-regulatory motifs within their promoters, increase the susceptibility of the cell to antibiotics but the phenotypic mechanism underpinning that change remain unknown.

The present decoy polynucleotides have a number of applications, including therapeutic use, e.g. medical or veterinary, and other *ex vivo* e.g. non-therapeutic applications, e.g. in disinfectants and cleaning products. The present decoys find use in methods where there is a need to reduce prokaryotic cell viability, kill cells, inhibit growth or reduce virulence.

As described, the present decoy polynucleotides and methods may be used to increase susceptibility of prokaryotes to antibiotics. Thus the decoys may be used to potentiate the effects of one or more antibiotics, such as those listed herein. This may be for medical or veterinary use, e.g. to treat or prevent a bacterial infection in humans or animals, or for in vitro use, e.g. in a cleaning composition. Thus the decoys may find use in bactericidal or bacteriostatic compositions.

Alternatively, the decoy polynucleotides and methods may be used to provide or increase the susceptibility of prokaryotes to fatal environmental factors or antibacterial agents.

The decoy polynucleotides and methods described herein may be used for treating bacterial infection in a subject comprising administering a decoy polynucleotide described herein. The subject may be a human or animal. A decoy polynucleotide described herein may also be used in medicine, e.g. for use in treating or preventing bacterial infection in a subject, and for the manufacture of a medicament for treating bacterial infection.

A decoy may be used in combination with an antibiotic(s) to which the decoy makes the cell more sensitive, and/or with another antibacterial agent. Suitable antibiotics are described herein. The antibiotic may be administered simultaneously with, or before or after the decoy. The antibiotic and decoy may be administered in the same or in separate compositions. Thus the invention includes combination therapies in which a decoy polynucleotide as identified, and/or as described herein, is administered to a subject in combination with one or more antibiotics or other antibacterial therapies.

The present disclosure includes a pharmaceutical composition or medicament comprising a decoy polynucleotide and a physiologically acceptable carrier or excipient. The composition may additionally comprise one or more antibiotics or other anti-bacterials as described.

Acceptable carriers or diluents for therapeutic use are well known in the pharmaceutical art, and are described, for example, in Remington' Pharmaceutical Sciences, Mack Publishing Co. (A. R. Gennaro edit. 1985). The choice of pharmaceutical carrier, excipient or diluent can be selected with regard to the intended route of administration and standard pharmaceutical practice. The pharmaceutical compositions may comprise as, or in addition to, the carrier, excipient or diluent any suitable binder, lubricant, suspending agent, coating agent, solubilising agent.

A variety of methods may be used to deliver the decoys of the present invention to the site of bacterial infection. Methods for *in vivo* and/or *in vitro* delivery include, but are not limited to, bucchal or oral delivery, intravenous delivery, direct injection into the infection or indirect injection (e.g. subcutaneous, intraperitoneal, intramuscular, or other injection methods), topical application, direct exposure in aqueous or media solution, transfection (e.g. calcium phosphate, electroporation, DEAE-dextran based, and lipid mediated), transgenic expression (e.g. a decoy expression system delivered by microinjection, embryonic stem cell generation, or retroviral transfer), or any of the other commonly used nucleic acid delivery systems known in the art. Administration may be in combination with a suitable dose of antibiotic, with the antibiotic(s) being administered at the same time as the decoy, or separately.

As described in the examples below the number ofTFDs needed to show a predictable effect on expression of the targeted gene and have a bacteriostatic effect can be as little as circa 5000 molecules per cell. It has been shown that in the standardised in vitro assays discussed in the examples as many as 1, 000, 000 bacterial cells are efficiently killed with as little as 1 nM of TFD, suggesting that it is sufficient to have a transfection efficiency of less than 0.001% to achieve killing. Quantifying transfection using fluorescence microscopy to measure the uptake of a fluorescently labelled TFD demonstrates that all *S. aureus* cells within view are transfected. In comparison with other nucleic acid-based strategies to tackle bacterial infections, such as antisense, this number of molecules needed to kill the cell is 100 to 1000-fold less. This partly reflects that though both antisense approaches and TFD act to inhibit genes, TFDs act at an early step to prevent transcription whilst antisense, in the most common iteration, sterically blocks the products of transcription: many thousands of mRNAs molecules. Secondly the TFDs have been designed to target essential genes that are positively induced, so need to be switched on for survival, and positively regulated (the transcription factor drives its own production). In vitro this later characteristic means that relatively few copies of the transcription factor are likely present when the gene is uninduced and so a small number of TFDs can block induction. It may be that in a therapeutic situation there are more transcription factors per cell, due to natural variety amongst the bacterial population or the gene being already induced. In this situation it is expected that more TFDs will be needed to see a therapeutic effect and estimate that increasing the dose by a factor of 100 (to 100 nM) or improving the transfection efficiency (by two orders of magnitude) will be sufficient to see a beneficial effect.

Some decoys may be used without antibiotics to treat or prevent bacterial infection. For example, some of the decoys described herein will reduce the ability of the cell to survive under conditions of stress, or to express essential genes or obtain essential nutrients. Some decoys will neutralise cell virulence, e.g. by preventing expression of virulence factors. Thus decoys used to target essential genes could be used on their own to prevent growth of particular pathogenic bacteria, either as a therapy in its own right or as a form of preventative medicine to tackle bacteria before they become infections. Similarly those decoys which target virulence determining genes could be used on their own to prevent the spread of infection. In another example the decoys listed herein which act against *H. pylori* could provide free standing therapies against stomach ulcers/development of peptic cancer.

Similarly decoys which act to prevent the growth of *Streptococcus mutans* could be used to treat or prevent teeth discolouration.

The particular infection or associated condition which the decoy is used to treat is in general dependent upon the pathogenic cell that the decoy targets. Bacterial infections and associated diseases which may be targeted using the present methods are listed herein in connection with particular bacteria, and in Figure 2.

Decoy polynucleotides described herein can also be used *in vitro,* for example, in antibacterial products such as those for cleaning, e.g. disinfectants, to kill or prevent growth or infectivity of bacteria. Where a decoy increases susceptibility to antibiotic, typically the antibacterial composition also includes one or more antibiotics and/or one or more anti-bacterial agents. In one aspect the invention relates to such a cleaning composition.

Decoy polynucleotides may further be used in products that are applied to a surface, such as a work bench or hands, for a time and under conditions that are sufficient to reduce or prevent growth of a microorganism, and/or kill a microorganism, thereby reducing or preventing growth or killing a microorganism. For example, one or more decoys may be sprayed onto the surface. Such spray application is useful, for example, for preparing a surface for preparation of a foodstuff or sterilising an object to be inserted into a patient. This is because spraying the decoy formulation reduces the handling of the surface or object, thereby further reducing the risk of contamination. Hand and mouth wash applications are also contemplated within the scope of the invention.

In the above circumstances, the decoys may be formulated in a suitable aqueous format. In which instance, the formulation may comprise water to forma and aqueous composition. The aqueous composition may further comprise aqueous and organic solvents and their combinations.

Also disclosed are kits for antibacterial use comprising a decoy polynucleotide as described herein and one or more antibiotics or other antibacterial agent(s) for combined use in killing or inhibiting growth or virulence of bacteria. Typically the kit includes instructions for use. Again the kit may be for therapeutic use, e.g. against bacterial infection, or for non-therapeutic use, e.g. for cleaning or disinfecting.

The invention will now be described in more detail by way of non-limiting examples with reference to the following figures in which:
**Figure 1****.** A copy of Table 1 from Poole J. (Antimicrobial Chemother. (2005) 56, 22-24) listing the efflux-mediated resistance to non-fluorquinoline antibiotics and fluoroquinoline antibiotics respectively. Reference numbers in the right hand column refer to those given in the paper.
**Figure 2****.** A copy of Table 2 from Poole J. (*supra*) listing examples of regulators and regulatory sequences which can be targeted according to the present methods. The Table also provides examples of bacterial strains in which the regulators or sequences occur and examples of disease indications associated with these bacteria.
**Figure 3****.** Provides a graphic representation of how n[snare] plasmids can be used to affect gene expression. A gene (A) is transcriptionally inactive due to the binding of a repressive transcription factor (B) to a cis-regulatory sequence (C) within the promoter of the gene. When introduced into a cell the n[snare] plasmid is able to affect expression of the targeted gene by titrating off the transcription factor B from the genomic promoter to relieve transcriptional repression of the downstream gene (D).

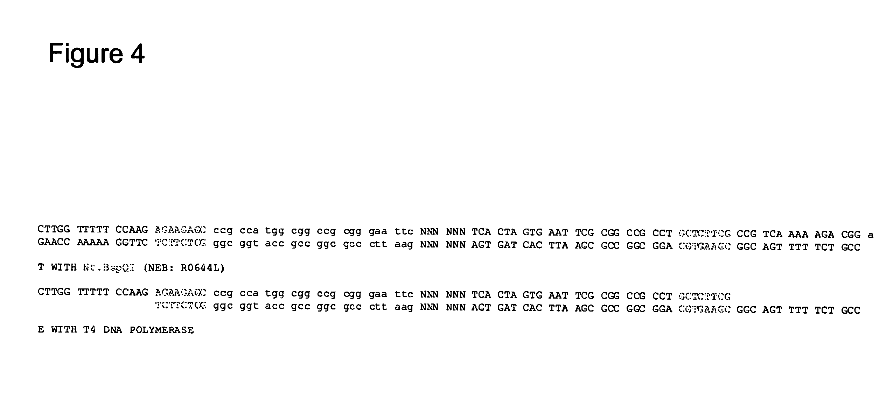

**Figure 4****.** Shows the amplification product obtained using the primers in **SEQ ID NOS: 3 & 4** and an appropriate vector substrate (Example 1.2).

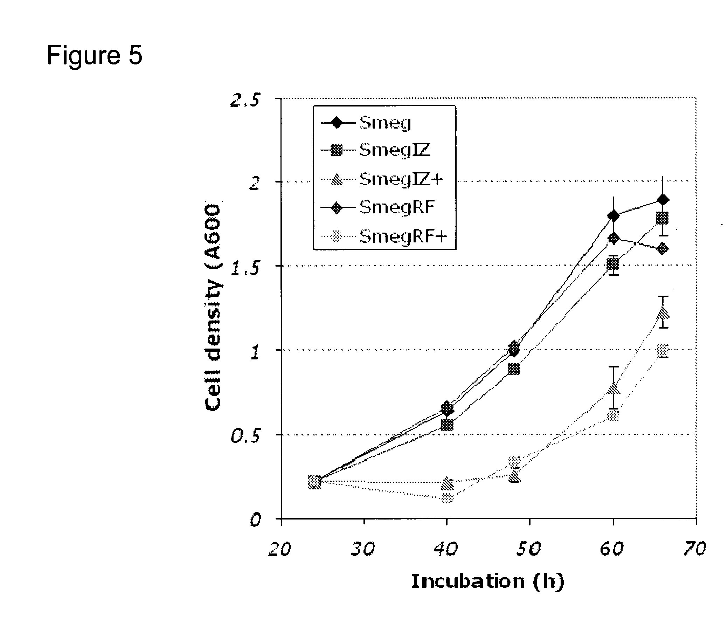

**Figure 5****.** Shows growth curves for *M. smegmatis* cultured in the presence of sub-inhibitory concentrations of isoniazid (IZ) or rifampicin (RF), where the bacteria have been treated with a WhiB7 decoy sequence (+) or treated with a negative control sequence (-) as in Example 2.

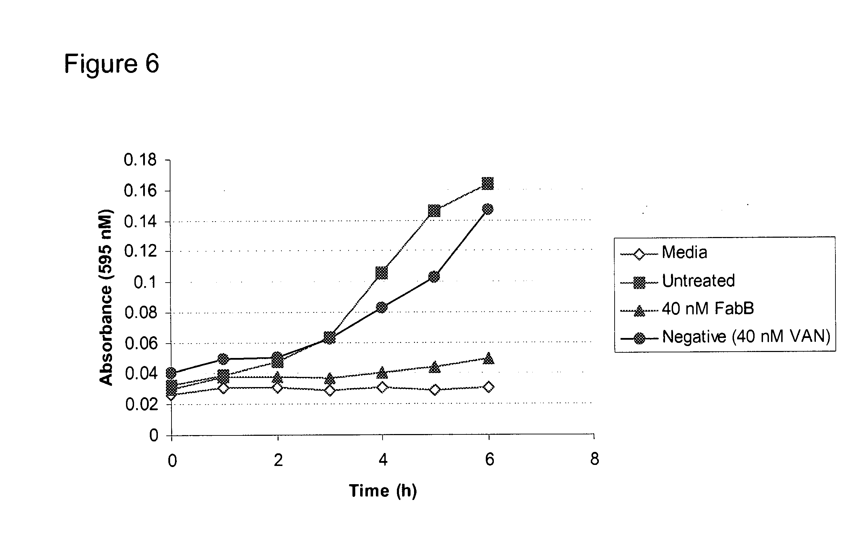

**Figure 6****.** Shows growth curves for *E. coli* grown in the presence of cerulenin, where the bacteria have been treated with a FabB decoy (targeting the FadR binding site) or treated with a negative control sequence, as in Example 3.

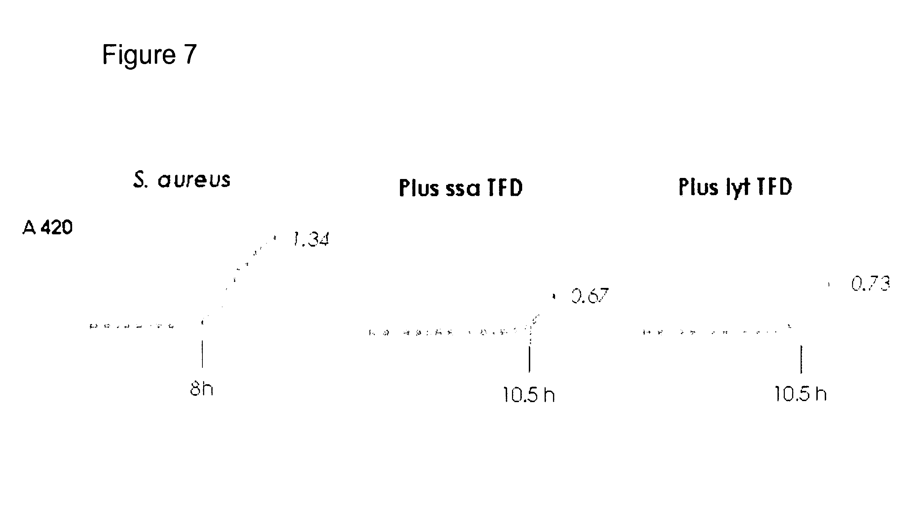

**Figure 7****.** Shows growth curves for *S. aureus* under conditions of stress arising from electroporation, where the bacteria are untreated (first curve) or have been treated with a SsaA decoy (second curve) or a LytM decoy (third curve) as in Example 4.

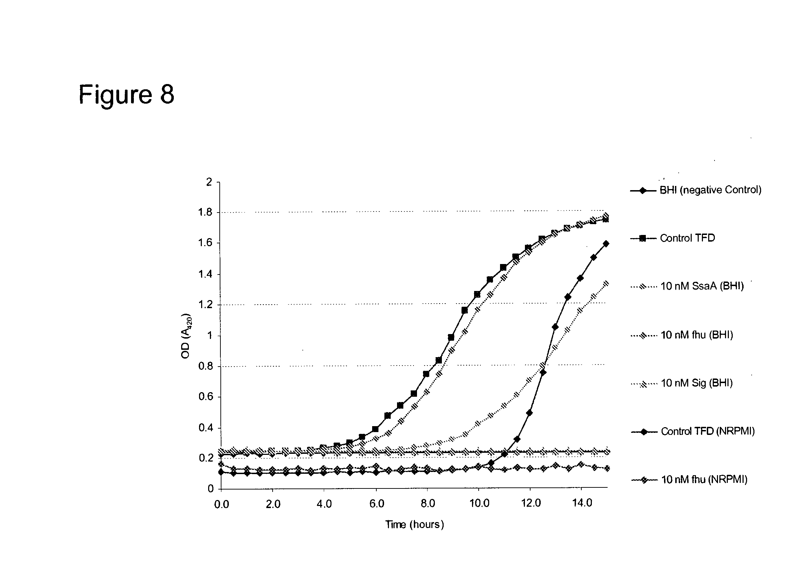

**Figure 8****.** Shows growth curves for *S.aureus* grown in the presence of uninocculated media (BHI negative control), transfected with a control TFD having a scrambled TFD sequence and grown in BHI media, transfected with SsaA TFD (containing a WalR site) and grown in BHI media, transfected with fhu TFD (designed to block iron uptake) and grown in BHI media, transfected with sig TFD and grown in BHI media, transfected with an unrelated TFD and grown in NRPMI media (iron-limited compared to BHI), and transfected with flm TFD and grown in NRPMI media.

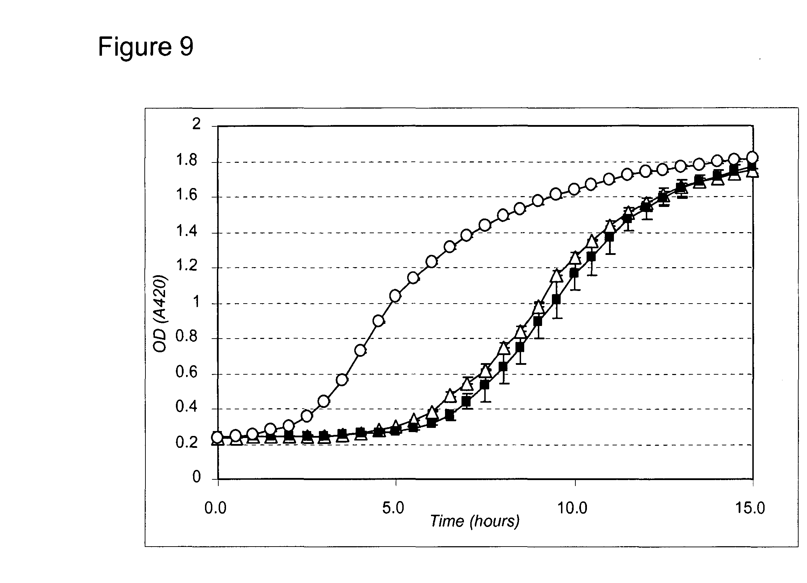

**Figure 9****.** Shows that Fur TFDs do not perturb growth of *S. aureus* in medium where iron is non-limiting. *S. aureus* was grown in BHI media with the Fur_TFD but without the Gramicidin as a transfection reagent (TFD alone; hollow circles), or combining 60 nM Gramicidin with 1 nM of a control TFD (Control TFD; hollow triangles) and the Fur_TFD (Fur_TFD; filled squares) that is designed to manipulate expression of the *fhu* operon. Results are the average of three repeats and error bars show the standard error.

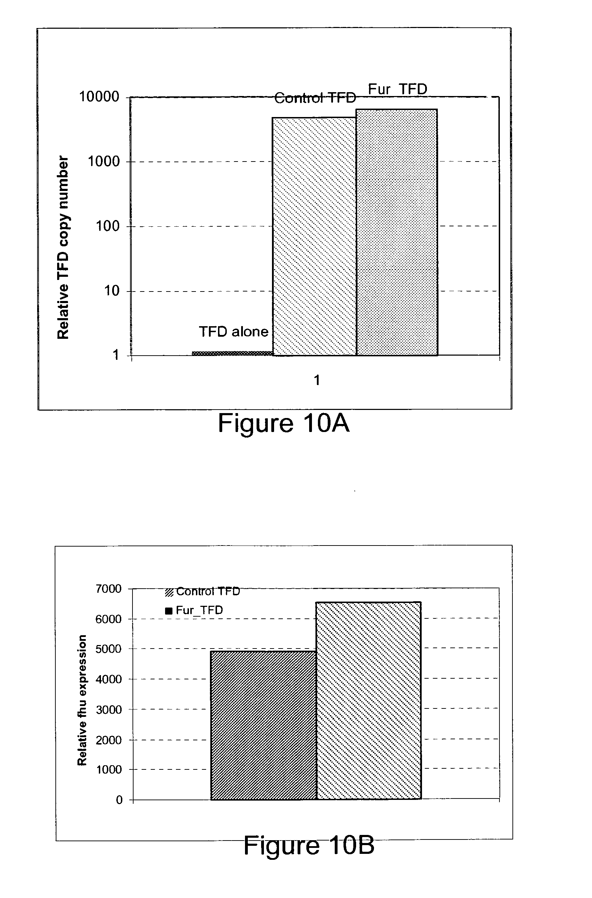

**Figure 10****.** Shows that Fur_TFD lifts repression of *fhu* genes. (**A**). Gramicidin-mediated transfection of *S. aureus.* Cells were treated with TFD alone, or Gramicidin and either the Control or Fur_TFD were harvested, washed and lysed. qPCR was used to establish the average numbers of TFD relative to the number of genomes. (**B**). Transfection of Fur_TFD represses fur genes in *S. aureus.* Parallel samples were used to determine relative levels of fhu expression by qPCR.

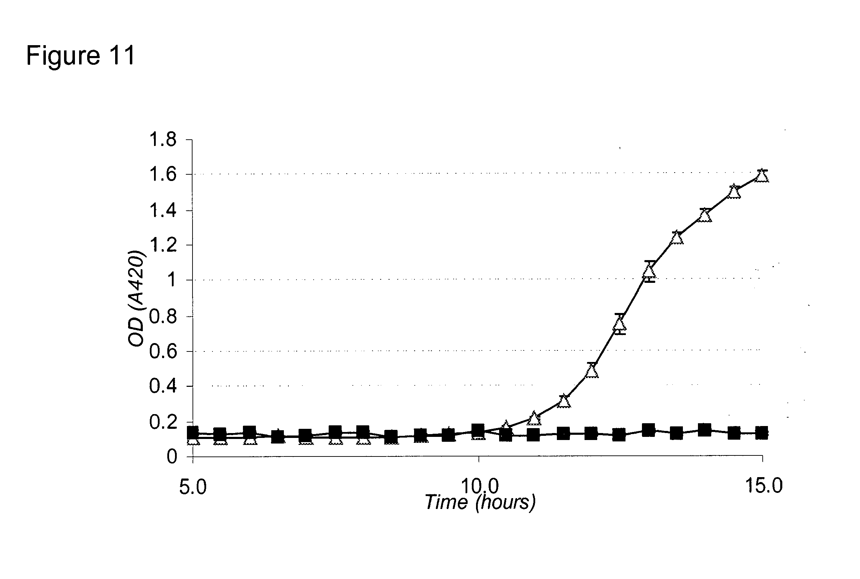

**Figure 11**. Shows that Fur_TFDs prevent growth of *S. aureus* in iron-limiting media. *S. aureus* was grown in an iron-limited media, NRPMI, with the Fur_TFD but without the Gramicidin as a transfection reagent (TFD alone; hollow circles), or combining 60 nM Gramicidin with 1 nM of a control TFD (Control TFD; hollow triangles) and the Fur_TFD (Fur_TFD; filled squares). Results are the average of three repeats and error bars show the standard error.

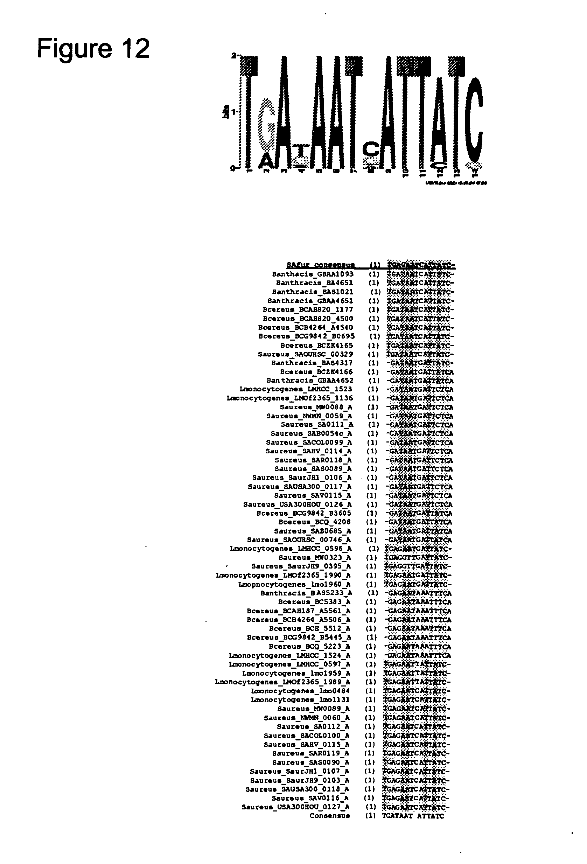

**Figure 12****.** Shows the results of genetic analysis to determine the potential effective host range of the S. *aureus* Fur targeted TFD (*SAfhu*). The MEME-derived consensus sequence shown in the top panel of the figure is a *S. aureaus* Fur binding site motif created from the sequences listed in the bottom panel.

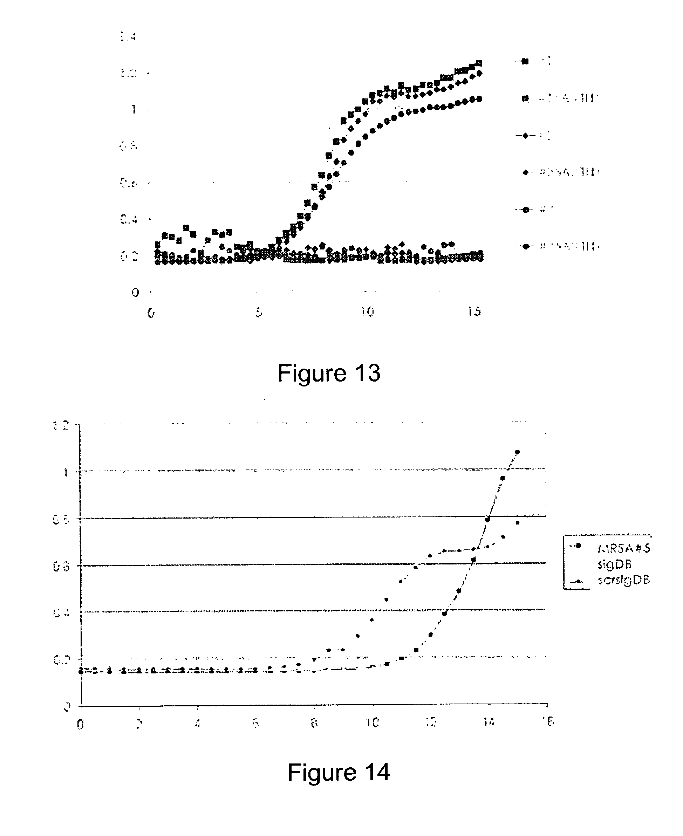

**Figure 13****.** Shows growth curves of three MRSA strains either in the presence of a control TFD or a TFD (SA3 TFD) containing the -10/-35 recognition sequences for the *S. aureus* alternative Sigma factor SigB.
**Figure 14****.** Shows that Sig TFDs in a "dumbbell" configuration repress the growth of the clinically-isolated MRSA strain MRSA-5.

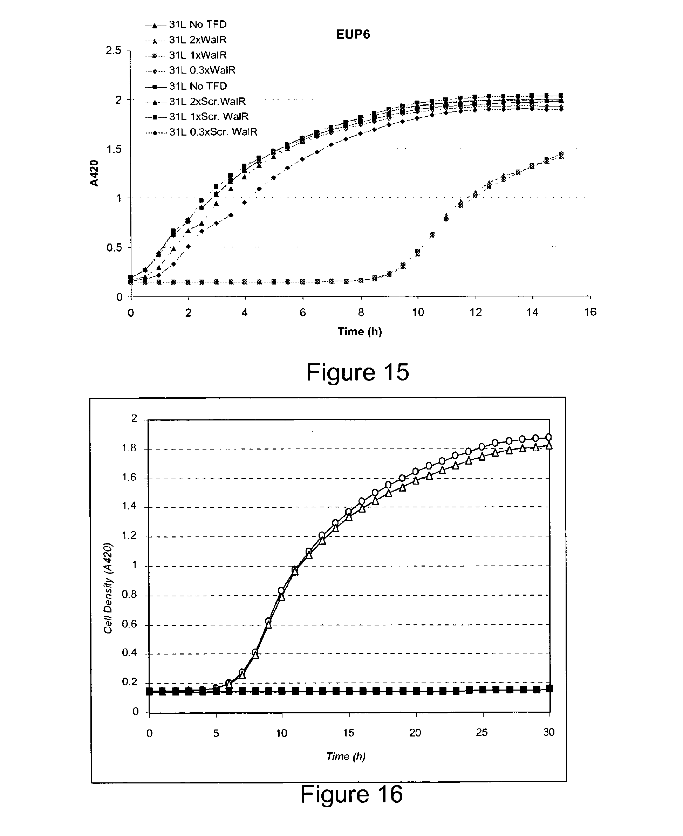

**Figure 15****.** Shows that WalR TFD in the dumbbell configuration prevents growth of EMRSA15. The fhu TFD is used as a control.
**Figure 16****.** Shows *S. aureus* grown in BHI media with 1 nM WalR_TFD/DOTAP mixture but without Lysostaphin as a transfection reagent (TFD alone; hollow circles), or combining 62 µg/ml Gramicidin with 1 nM of a control TFD (Scr_TFD; hollow triangles) and the WalR_TFD (filled squares) that is designed to manipulate expression of the WalKR regulon.

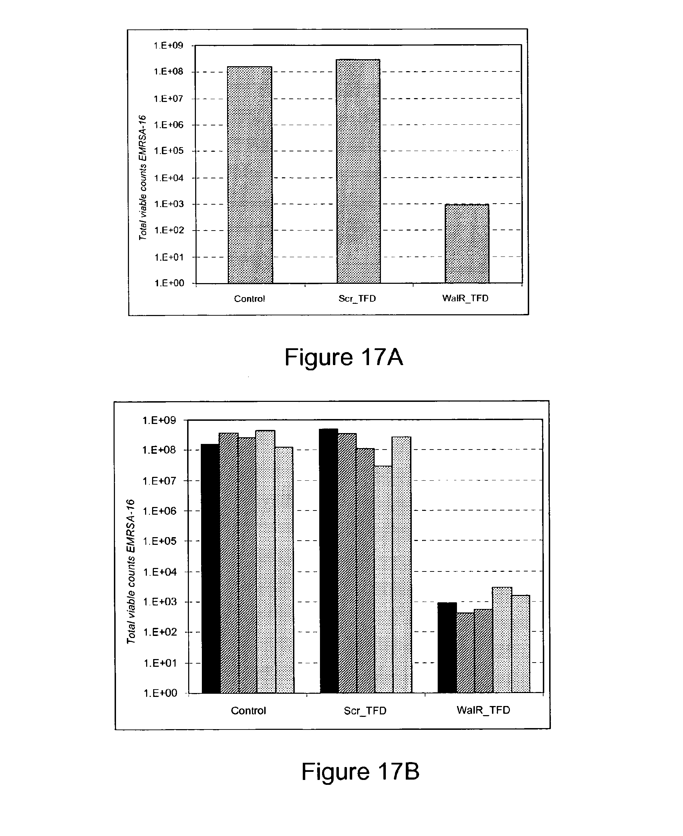

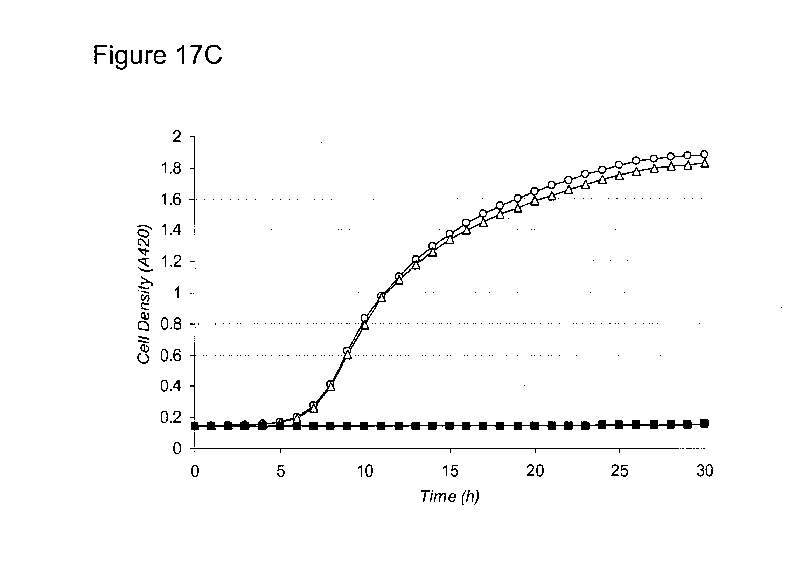

**Figure 17****.** No resistance mechanism has been detected. (**A**). Total viable counts of EMRSA-16 following completion of in vitro growth experiments described in Figure 1. (**B**). Cells that were not killed by WalR_TFD treatment were sub-cultured and tested in *in vitro* growth experiments. WalR_TFD effectively killed the sub-cultured cells. Five subsequent rounds were performed and no re-growth was seen for those cells treated with WalR_TFD as determined by measuring total viable counts. (**C**) Similarly growth curves of bacteria that had previously been exposed to four rounds of TFD-selection show no signs of resistance as they are efficiently killed. Cell growth was measured by recording A420 of cultures over a period of 30 h, cells were either untreated (media alone; open circles), treated with the control TFD plus transfection mixture (open triangles) or the WalR_TFD plus transfection mixture (filled squares).

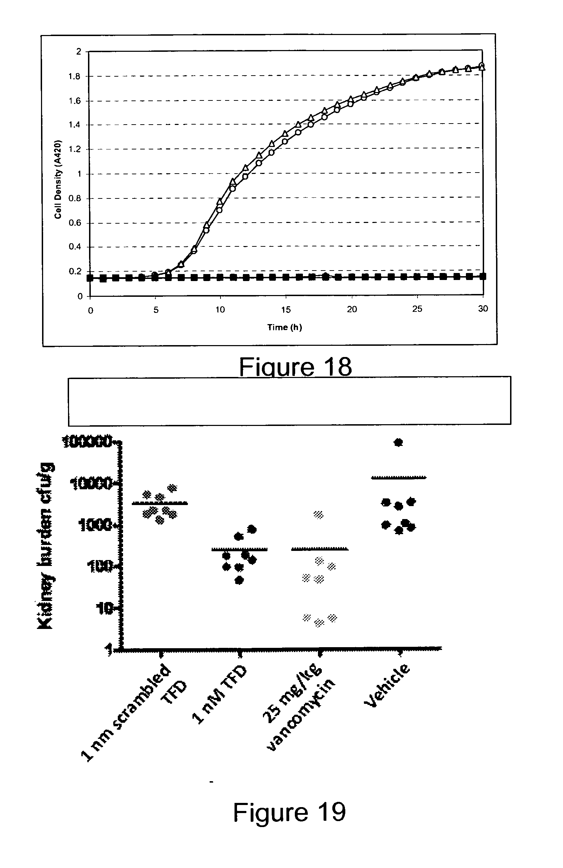

**Figure 18****.** Microdilution experiments demonstrate that only limited contact is needed for WalR_TFD to kill EMRSA-16. Cells were either untreated (media alone; open circles), treated with the control TFD plus transfection mixture (open triangles) or the WalR_TFD plus transfection mixture (filled squares) by incubation for 30 minutes before being diluted 100-fold into media without either transfection agents or TFD complexes. Only those cells originally treated with WalR_TFD were unable to grow.
**Figure 19****.** WalR_TFD is as effective as vancomycin in killing EMRSA-16 in a mouse sepsis model. Kidney burden of EMRSA-16 was measured following systemic infection of mice and treatment with the control TFD (Scr_TFD, 1 nM WalR_TFD, vancomycin and vehicle alone).

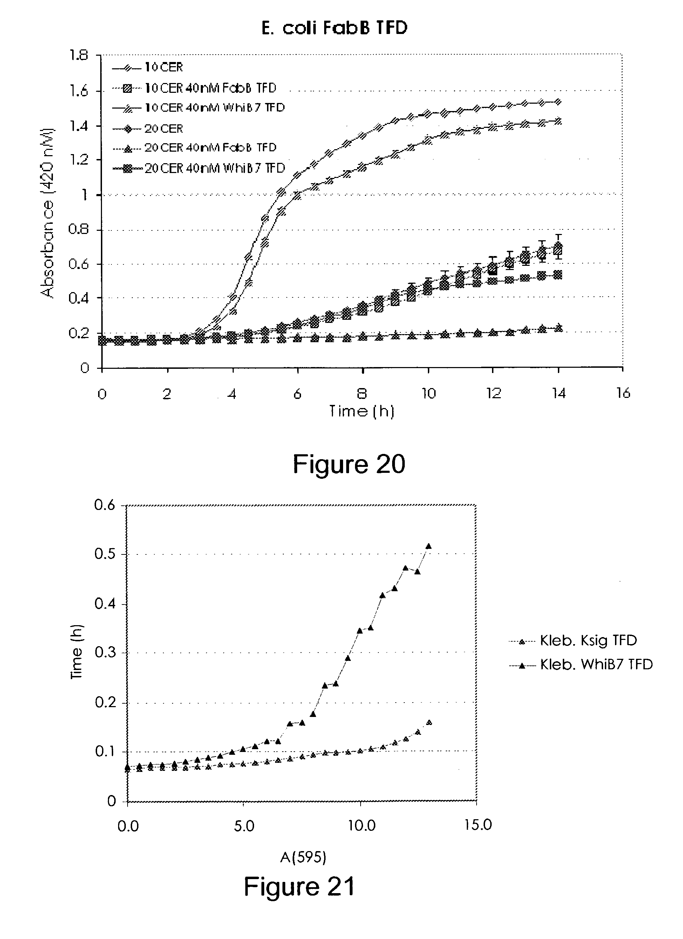

**Figure 20****.** Shows that transfection of *E. coli* with FabB TFD sensitises the bacteria to antibiotics (cerulenin) that inhibit fatty acid synthesis. WhiB7 TFD is used as a negative control as it is a FD carrying a sequence that does not occur in the *E. coli* genome.
**Figure 21****.** Shows that transfection of a TFD containing the recognition sequence for the σ54 factor of *Klebsiella pneumoniae* retards bacterial growth. WhiB7 TFD is used as a negative control.

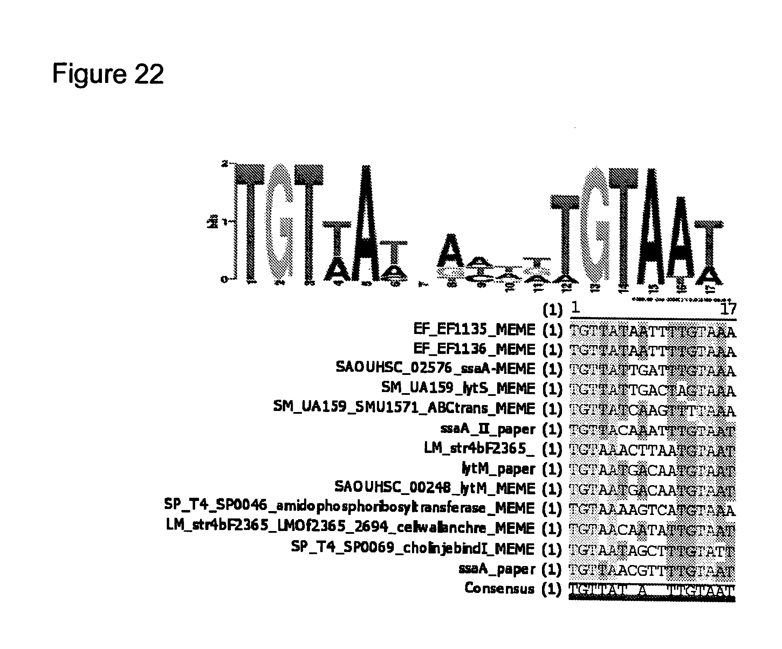

**Figure 22****.** Shows sequences identified as WalR binding sites to find close matches in *S aureus* and other organisms. A subset of hits is shown from which a consensus sequence has been derived.

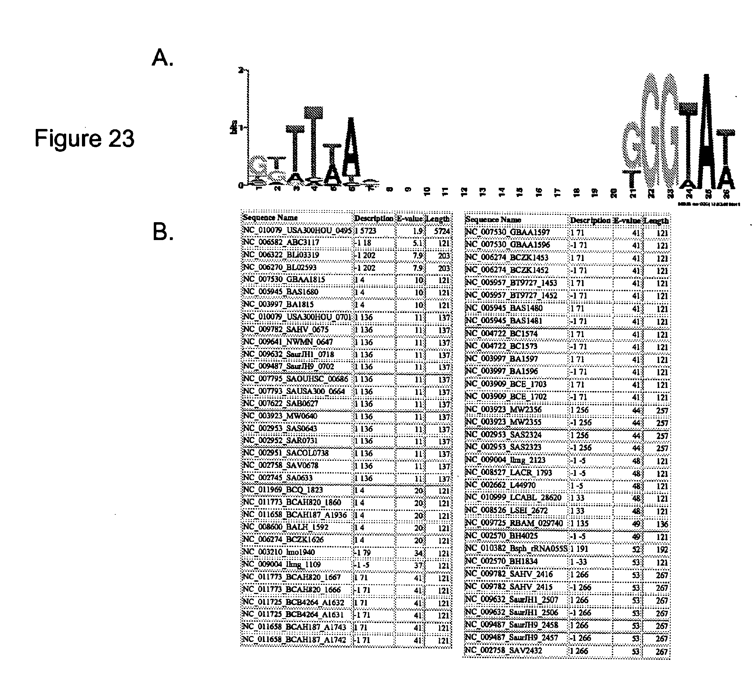

**Figure 23****.** Panel A shows a MEME-derived consensus sequence for the *S. aureus* SigB binding site. Panel B lists examples of occurrences (with E-values < 100) of matches to a consensus sequence in Bacillales (Gram-positive infections for which representative genera include *Bacillus, Listeria* and *Staphylococcus*).

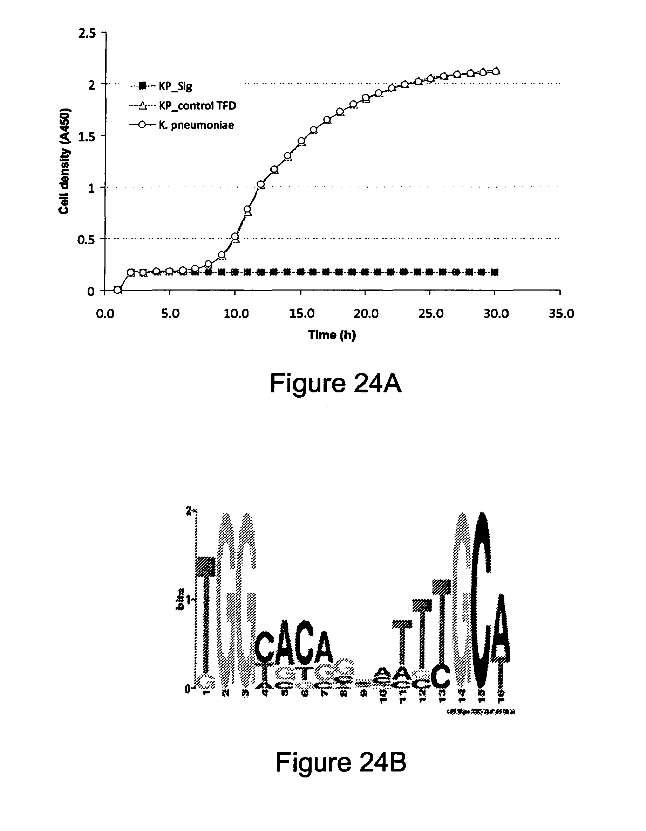

**Figure 24****.** Panel A shows that KP_Sig TFD kills *K. pneumoniae in vitro.* The TFD containing the KP_Sig sequence prevented cell growth *in vitro* (KP_Sig, filled square). Two controls were used: untreated cells (*K. pneumoniae,* hollow circle) and a control TFD consisting of a scrambled sequence (KP-control TFD, hollow triangle). Panel B shows a MEME-derived consensus sequence for the *K. pneumoniae* Sig binding site.

### Brief description of the sequences

SEQ ID NOS: 1 & 2 -oligonucleotide primers used for amplification of a target sequence from the pGEMT-Easy vector as in Example 1.1.
SEQ ID NOS: 3 & 4 - oligonucleotide primers used for amplification of a target sequence from the pGEMT-Easy vector in the production of dumbbell decoys as in Example 1.2.
SEQ ID NO: 5 - the WhiB7 decoy used in Example 2
SEQ ID NO: 6 - the FabB decoy used in Example 3
SEQ ID NO: 7 - the LytM decoy used in Example 4
SEQ ID NO: 8 - the SsaA decoy used in Example 4
SEQ ID NO: 9 - a native WhiB7 binding site in *M. smegmatis* str MC2 155
SEQ ID NO: 10 - a native FadR binding site in *E. coli* K12
SEQ ID NO: 11 - a native binding site for YycF/YycG in *S. aureus*
SEQ ID NO: 12 - a native binding site for YycF/YycG in *S. aureus*
SEQ ID NO: 13 - a native binding site for Sig^{B} in *S. aureus*
SEQ ID NO: 14 - a native binding site for Sig^{B} in *K. pneumoniae*
SEQ ID NO: 15 - a consensus sequence for Fur binding in *S. aureus*
SEQ ID NO: 16 - a consensus sequence for Fur binding in *E. coli*
SEQ ID NO: 17 - a native binding sequence for Fur in *H. pylori*
SEQ ID NO: 18 - a consensus binding site for TcdR in *C. diffcile*
SEQ ID NO: 19 - a consensus binding site for Vfr in *P. aeruginosa*
SEQ ID NO: 20 - a native binding site for Vfr in *P. aeruginosa*
SEQ ID NO: 21 - a native binding site for Vfr in *P. aeruginosa*
SEQ ID NO: 22 - a native binding site for NtrC in *K. pneumoniae*
SEQ ID NO: 23 - a native binding sequence for ArsR in *H. pylori*
SEQ ID NO: 24 - a native binding sequence for ArsR in *H. pylori*
SEQ ID NO: 25 - a glycopeptide-resistant consensus sequence in *S. aureus*
SEQ ID NO: 26 - an Agr binding motif in *S. aureus*
SEQ ID NO: 27 - an Agr binding motif in *S. aureus*
SEQ ID NO: 28 - forward primer sequence for PCR preparation of the SasigB TFD
SEQ ID NO: 29 - reverse primer sequence for PCR preparation of the SasigB TFD
SEQ ID NO: 30 - forward primer sequence for PCR preparation of the SAfhu TFD
SEQ ID NO: 31 - reverse primer sequence for PCR preparation of the SAfhu TFD
SEQ ID NO: 32 - forward primer sequence for PCR preparation of the SsaA TFD
SEQ ID NO: 33 - reverse primer sequence for PCR preparation of the SsaA TFD
SEQ ID NO: 34 - control sequence
SEQ ID NO: 35 - control sequence
SEQ ID NO: 36 - forward primer sequence for PCR amplification of 16s rRNA
SEQ ID NO: 37 - reverse primer sequence for PCR amplification of 16s rRNA
SEQ ID NO: 38 - forward primer sequence for PCR quantification of the fhu gene
SEQ ID NO: 39 - reverse primer sequence for PCR quantification of the fhu gene
SEQ ID NO: 40 - phosphorylated Sig dumbbell TFD oligonucleotide sequence
SEQ ID NO: 41 - phosphorylated Sig dumbbell TFD oligonucleotide sequence
SEQ ID NO: 42 - phosphorylated primer for dumbbell TFD containing scrambled version of Sig binding site
SEQ ID NO: 43 - phosphorylated primer for dumbbell TFD containing scrambled version of Sig binding site
SEQ ID NO: 44 - phosphorylated dumbbell oligonucleotide incorporating the binding sequence for WalR
SEQ ID NO: 45 - phosphorylated dumbbell oligonucleotide incorporating the binding sequence for WalR
SEQ ID NO: 46 - phosphorylated dumbbell oligonucleotide incorporating a scrambled version of the WalR binding site
SEQ ID NO: 47 - phosphorylated dumbbell oligonucleotide incorporating a scrambled version of the WalR binding site
SEQ ID NO: 48 - phosphorylated oligonucleotide incorporating the biding site for WalR SEQ ID NO: 49 - phosphorylated oligonucleotide incorporating the biding site for WalR SEQ ID NO: 50 - forward primer for FabB promoter
SEQ ID NO: 51 - reverse primer for FabB promoter
SEQ ID NO: 52 - forward primer for WhiB7 TFD
SEQ ID NO: 53 - reverse primer for WhiB7 TFD
SEQ ID NO: 54 - forward primer for TFD containing the recognition sequence for the σ54 factor of *K. pneumoniae*
SEQ ID NO: 55 - reverse primer for TFD containing the recognition sequence for the σ54 factor of *K. pneumoniae*
SEQ ID NO: 56 -sequence of a cell-penetrating peptide
SEQ ID NO: 57 - WalR TFD consensus sequence
SEQ ID NO: 58 - SigB TFD consensus sequence
SEQ ID NO: 59 - KP_Sig TFD sequence
SEQ ID NO: 60 - KP_Sig TFD consensus sequence.

### EXAMPLES

Although in general many of the techniques mentioned herein are well known in the art, reference may be made in particular to Sambrook and Russell, 3rd Edition 2001, Molecular Cloning: a laboratory manual.

### Example 1

### 1.1 Cholesterol labeled TFDs in the presence of Streptolysin-O

Using oligonucleotide primers, one of which has a 5' cholesterol modification and the other a similar modification at its 5' end or some other (such as a fluorescent dye, such as Cy5, so that the uptake of the transcription factor decoy (TFD) can be easily measured) a TFD is prepared by PCR. If the TFD has been previously cloned into a vector (pGEMT-Easy) the primers are designed to anneal to the vector sequences immediately flanking the insert, for example: 
SEQ ID NO: 1 Chol_TEf: 5' Cholesterol-TEG-GGC CGC CAT GGC GGC CGC GGG
AAT TC
 SEQ ID NO: 2 Cy5_TEr: 5' Cy5- AGG CGG CCG CGA ATT CAC TAG TG.

If the sequence to be used for a TFD has not been cloned it may either be directly synthesised (if short enough) and annealed to form the TFD, or amplified directly from genomic DNA using primers designed to anneal within the TFD.

The PCR product is ethanol precipitated and resuspended in TE buffer (10 mM Tris.HCl, 1 mM EDTA pH8.0) at a concentration of 500-1000 ng/µl. Typically antibiotic sensitivity assays are performed using 96 well plates, each well containing 200 ml of broth. For example, in the case of *Enterococcus faecium* this broth is BHI (Brain Heart Infusion) media (Becton Dickinson) supplemented with 0.2 U/ml Streptolysin-O (Sigma) and 5 µg/ml of vancomycin antibiotic and inoculated with a vancomycin-resistant strain of *E. faecium.*

### 1.2 Preparation of Dumbbells by PCR

Dumbbell decoys are covalently closed single stranded DNA characterised by a doublestranded centre, containing the binding site for the targeted factor, flanked by stem-loop structures. The stem-loops stabilise the decoy by preventing action of exonucleases which would otherwise degrade the decoy polynucleotide. Hence Dumbbell TFDs (DB) are so called because of their characteristic shape.

DBs are prepared by PCR using as a template a pGEMTEasy-derived plasmid containing the targeted binding site, as described in section 1.1. The primers used in amplification are:

SEQ ID NO: 3 DBTEf: 5' P- CTTGG TTTTT CCAAG AGAAGAGC CCG CCA TGG
CGG CCG CGG GAA TTC

SEQ ID NO: 4 DBTEr: P- CCG TCT TTT TGA CGG CGA AGA GCA GGC GGC CGC
GAA TTC ACT AGT GA

The portion of the primers which will form the stem-loops are underlined. Amplification with the appropriate vector gives the DNA product shown in Figure 4, where the portion of the DB which will bind to the transcription factor is given by 'NNN NNN'. The sequences shown in bold represent a binding site for the nicking restriction enzyme Nt.*BspQ*1. In the second part of Figure 4 the consequence of digesting the PCR product with Nt.*BspQ*1 is shown; this exposes the stem-loop structures as single stranded regions which will form a stem-loop and can subsequently be ligated by treatment with T4 DNA ligase to form a covalently closed circle and DB.

### 1.3 Preparation of Dumbbell oligonucleotides by restriction digest of plasmid

Alternatively dumbbells can be made by cloning the blunted PCR product shown in Figure 4 into a suitable PCR-cloning vector, confirming its identity and preparation of the plasmid. The plasmid can then be digested to release the insert which is additionally digested with Nt.*BspQ*1 to release the fragment shown in the second portion of Figure 4. This can be similarly treated with T4 DNA ligase in order to covalently close the DNA molecule and form a DB.

The advantage of this approach is that is more amenable, both practically and economically, to scaling up should the dumbbell be required in large quantities.

### 1.4 Transfection with R9-cholesterol agent.

R9-cholesterol has been described for its properties of aiding transfection of siRNA (or other nucleic-acid based therapy) molecules and the like in eukaryotic cells (US Patent Application No. 2007-0207966). Here we describe its utility in transfecting various bacteria with TFDs.

R9-cholesterol, which consists of a cholesterol molecule attached to a linear chain of nine D-arginines, was synthesised as previously described (Kim W.J. et al, Mol. Ther. (2006) 14:343-350). TFDs were mixed with increasing amounts ofR9-cholesterol in a TE based buffer supplemented with 5% glucose. The mixture was incubated at room temperature for 1 hour and then either used directly in transfections or analysed by agarose gel electrophoresis. Typically the minimum amount of R9-cholesterol was used that caused the complex with DNA not to run in the gel; i.e. the charge of the nucleic acid backbone had been neutralized by binding of poly-arginine. The cholesterol molecule helps the TFD associate with the bacterial membrane and so enter the cell.

TFD/R9-cholesterol conjugates were mixed at various concentrations into 200 µl of culture in a 96 well plate. For example, in the case of *Enterococcus faecium* this broth is BHI media (Becton Dickinson) supplemented with 0.2 U/ml Streptolysin-O (Sigma) and 5 µg/ml of vancomycin antibiotic and inoculated with a vancomycin-resistant strain of *E. faecium.*

### Example 2

### WhiB7 decoy to sensitise Mycobacterium smegmatis to antibiotics

WhiB7 is a gene in *Mycobacterium smegmatis* that has been shown to have a role in determining sensitivity of this bacterium to antibiotics. Functionally deleting this gene renders the bacterium hypersensitive to antibiotics (PNAS (2005) 102:12200). WhiB7 is believed to be a transcriptional regulator and several such genes have been described in the literature. A close homologue of WhiB7 exisits in the pathogen *Mycobacterium tuberculosis* which is the causative agent of Tuberculosis.

A decoy polynucleotide containing the WhiB7 decoy sequence was prepared as described in Example 1.1 and various concentrations were used to transfect *M. smegmatis* grown in 20 ml of broth (in 200 ml shake flasks containing a wire baffle). The media used was 9H11 (Becton Dickinson) supplemented with 10% ODAC (Becton Dickinson) and sub-inhibitory concentrations of the antibiotics isoniazid (IZ) or rifampicin (RF). The flasks were incubated at 37°C with shaking and at various intervals samples were withdrawn and their absorbance measured (to monitor cell growth). Flasks were either treated with 100 nM of the WhiB7 TFD (+) or treated with the negative control TFD (-). It was evident that in the flasks treated with the WhiB7 TFD, but not the control TFD, cells were rendered sensitive to both the antibiotics tested (see Figure 5).

SEQ ID NO: 5 - WhiB7 TFD 5' TGG CCA CGG ATC CGG GTG ACT GCG GGT CCG
TGG CCT 3'

### Example 3

### FabB decoy to down-regulate Escherichia coli essential genes

The FabB decoy contains the binding site for the FadR regulator that controls expression of genes involved in the essential pathway of fatty acid synthesis (including *fab*A and *fab*B)*.* It has been demonstrated that functionally knocking out the fadR gene results in *Escherichia coli* results in a strain that is deficient in aspects of fatty acid synthesis and unable to grow in the presence of the antibiotic cerulenin (which targets fatty acid synthesis (J. Bacteriol. (2005) 183:5292)).

The FabB decoy contained the sequence:

SEQ ID NO: 6 FabB TFD 5' TTT ATT CCG AAC TGA TCG GAC TTG TTC AGC GTA
CAC GTG TTA GCT ATC CTG CGT GCT TCA 3'

The decoy was prepared as described in Example 1.1 and used at various concentrations to transfect a well of a 96-well plate containing 200 µl of Rich media (10 g Tryptone, 5 g NaCl, 1 g Yeast extract per litre and supplemented with 0.2% glucose and 0.4% acetate, final concentrations) supplemented with 1 µg/ml cerulenin (Sigma). The plates were incubated at 37°C with shaking and absorbance readings (at 595 nM) were taken using a plate reader. All data points were performed in triplicate. Bacteria were either untreated with TFDs (Untreated), transfected with FabB TFD (FabB) or a negative control consisting of a TFD whose sequence did not occur within the genome of *E*. *coli* (Van).

As is evident in Figure 6, the untreated sample and negative control both grew at comparable rates in the broth but treatment with the FabB TFD caused the cells to hardly grow at all.

### Example 4

### Decoys to sensitise Staphylococcus aureus to stress response

Decoy polynucleotides were transfected into *S*. *aureus* by electroporation. Bacterial cells were prepared by standard methods. Briefy, cells were grown to early exponential phase in LB media at 37°C as defined as reaching an Absorbance of 0.3 at 420 nM. The cells were chilled to 4°C and harvested by low speed centrifugation and washed three times in a similar volume of sterile, ice-cold 10% (v/v) glycerol. Finally the cells were resuspended in a tenth of their original volume in 10% glycerol. Electroporation was performed by mixing 50 µl of cells with 1 µl of TFD (typically 100 ng to 1 µg of DNA) in an electroporation cuvette and electroporated in a BioRad Genepulser with settings of 2.5 kEV, 100 Ω and 25 µF, giving time constants in the range 3.8 to 4.8 ms. Typically 50 µl of electroporated cells were used to inoculate 10 ml of BHI or LB media with 30 mM KOH (to induce stress response). 200 µl of cells were aliquoted into wells of a 96 well plate that was incubated at 37°C with moderate shaking, growth was monitored by measuring absorbance. Plots of growth curves demonstrated the effects of specific TFDs in slowing the rate of growth compared to a control that had been taken through a mock-electroporation (where no DNA had been transfected), or transfected by an unrelated sequence (Figure 7). The following decoy sequences were capable of slowing the growth of *S*. *aureus* under conditions of stress: 
SEQ ID NO: 7: LytM TFD 5' GCT ATT TTG TAA TGA CAA TGT AAT GAG TTT
AGT AAA AA 3'
 SEQ ID NO: 8 SsaA TFD 5' ATT ACA AAT TTG TAA CAG ACT TAT TTT A 3'.

### Example 5

### Sig TFD inhibited growth of S. aureus

Sig_TFD contained the binding site for the *S*. *aureus* SigB protein. This protein is the alternative sigma factor that controls a large set of stress related genes (Wigneshawerajaj, Molecular Microbiology (2008) 68:538). By transfecting *S*. *aureus* cells with this TFD and growing the cells in 96 well plates in defined medium, it is evident that the TFD inhibits growth of the cells. Fhu DNA contains the binding site for the Fur transcription factor that regulates uptake of iron under conditions where that ion is limiting (Horsburgh et al, J. Bacteriol. (2001) 183:468). SsaA contains the binding site for the two-component regulator WalR, which has previously been shown to regulate 12 genes that affect the rate of growth (Dubrac & Msadek, J. Bacteriology (2004) 186:1175).

### 5.1 Preaparation of TFDs by PCR

The TFDs are prepared by PCR as described in co-pending application PCT/GB2008/003353. The oligonucleotide primers used in the PCR reaction typically modified at the 5' end, for example, one of which has a 5' cholesterol modification. Other modifications used include biotin, amines, cell penetrating peptides, fluorescent dye, such as Cy5 or fluorescin, so that the uptake of the TFD can be easily measured. Other modifications considered for testing include lipids that are characteristic of the membranes of pathogenic bacteria. TFDs generated by PCR can therefore be modified at their 5' ends with a variety of molecules that serve to: increase the *in vivo* stability of the TFD; increase the efficiency of uptake; enable detection by fluorescence. If the TFD has been previously cloned into a vector (pGEMT-Easy) the primers are designed to anneal to the vector sequences immediately flanking the insert, for example: 
SEQ ID NO: 1 – Chol_TEf: 5' Cholesterol-TEG-GGC CGC CAT GGC GGC CGC GGG
AAT TC 3'
 SEQ ID NO: 2 - Cy5_TEr: 5' Cy5- AGG CGG CCG CGA ATT CAC TAG TG 3'.

The PCR product is ethanol precipitated and resuspended in TE buffer at a concentration of 500-1000 ng/µl.

The TFDs are cloned by annealing together pairs of synthetic phosphorylated oligonucleotides that contain the binding site to be tested. Each pair of oligonucleotides contain an adenine at the 3' end, this is for ease of cloning into the pGEM_Teasy vector that has 5' thymine overhangs (to expedite cloning of PCR products).

The primer sequences that were used for the Sig TFD are:

SEQ ID NO: 28 – SasigB FOR: GAA GAT TAG AAA TTA TTT CGA T GGG TAT ATA
ATA A

SEQ ID NO: 29 – SASigB REV: TAT TAT ATA CCC ATC GAA ATA ATT TCT AAT
CTT C A

The primer sequences that were used for the Fhu TFD are:

SEQ ID NO: 30 – SAfhu FOR: ACT ACA AGT ACT ATT AGT AAT AGT TAA CCC
TA

SEQ ID NO: 31 – SAfhu REV: AGG GTT AAC TAT TAC TAA TAG TAC TTG TAG
TA

The primer sequences that were used for the SsaA TFD are:
SEQ ID NO: 32 - SsaA FOR: ATT ACA AAT TTG TAA CAG ACT TAT TTT A
SEQ ID NO: 33 - SsaA REV: AAA ATA AGT CTG TTA CAA ATT TGT AAT A

### 5.2 Performing growth studies in 96-well plates.

Cholesterol/Cy5-labeled decoy polynucleotides were prepared as in section 5.1. Typically 1 µg of TFD was then complexed with 0.3 µl of the liposomal transfection reagent DOTAP (Roche) and incubated at room temperature for 10 min. The assays to determine their effect on growth of bacterial cells were performed using 96 well plates, each well containing 200 µl of broth consisting of BHI media (from Becton Dickinson), which is not iron-limited, supplemented with 60 nM Gramicidin (from Sigma Corporation. Cat# G5002) or iron-depleted media (NRPMI) similarly supplemented with Gramicidin. The method for iron-depletion is described in full elsewhere (Science (2004) 305:1626-1628). 1 µl of various concentrations of a Cholesterol/Cy5-labeled decoy complexed with TFD were added to each well and their effect on bacterial growth of *S. aureus* monitored by measuring absorbance of the broth at intervals during incubation. The plates were incubated at 37°C with shaking and absorbance readings (at 450 nM) were taken using a plate reader. Several decoys were tested: Sig, fhu and SsaA.

As can be seen in Figure 8, it is evident that treatment with as little as 10 nM Sig TFD prevented growth in BHI medium, while a similar amount of the fhu TFD had no effect on bacterial growth in this media, which is non-iron limiting. 10 nM of the SsaA TFD resulted in a measurable delay in bacterial growth.

When an iron-limited media was used 10 nM of the fhu TFD proved able to prevent growth, whereas a similar amount of a control TFD (SsaA) had no effect on bacterial growth under these conditions.

### Example 6

Fur TFDs control expression *of fhu* and preventing growth of S. *aureus* in iron-limited media.

TFDs were designed to bind to the Fur transcription factor, which plays a central role in controlling bacterial iron uptake (Somerville and Proctor (2009) Microbiol. Mol. Biol. Rev. 73: 233-248), negatively regulating the *fhu* operon that controls import of scavenged iron. Iron is essential for growth - it is needed for electron transfer and acts as a cofactor in many enzymatic processes. However, in the host, iron is present in limiting amounts and is usually rendered inaccessible to the bacteria by incorporation into host carrier proteins such as transferrin and lactoferrin. In response, *S. aureus* synthesises high-affinity iron-chelators, siderophores, that bind iron and use ABC transporters, such as that encoded by *fhuCBG,* to import the iron-siderophore complex into the bacterial cytoplasm. Since the import of excess iron would result in free radical formation by the Fenton reaction, with subsequent cell damage (Wandersman and Delepelaire, Ann Rev Microbiol (2004) 58:611-647), iron uptake must be tightly regulated. Fur knock-out strains grow poorly in iron-limited media *in vitro* and lose their pathogenicity in animal models (Horsburgh, et al. J. Bacteriol (2001) 183:468-475), demonstrating the potential of Fur as a therapeutic target.

### 6.1 METHODS

### 6.1.1 Bacterial strains and culture conditions

*S. aureus* ATCC 6538NA cultures were grown and maintained in BHI media (Becton Dickenson). Frozen stocks were prepared by mixing equal volumes of a culture in early exponential growth (OD of 0.3 at A₆₀₀) with 50% glycerol and freezing at -80°C. BHI was the media used for non-iron limiting conditions and NRPMI, which had been prepared as previously described (Skaar *et al.* 2004), for iron-limiting growth conditions.

### 6.1.2 Preparation of TFDs

Plasmids containing the TFD sequences were created by annealing oligonucleotides containing the binding site for Fur (SAFur1, 5' P- ACT ACA AGT ACT ATT AGT AAT AGT TAA CCC TA- 3' (**SEQ ID NO: 30**) and SAFur2, 5' P- AGG GTT AAC TAT TAC TAA TAG TAC TTG TAG TA-3' (**SEQ ID NO: 31**)), and a control sequence (Con1, 5' P-TGG CCA CGG ATC CGG GTG ACT GCG GGT CCG TA- 3' (**SEQ ID NO: 34**) and Con2, 5' P- ACG GAC CCG CAG TCA CCC GGA TCC GTG GCC AA- 3' (**SEQ ID NO: 35**), ligating them into the pGEMTEasy vector (Promega) and transformed into *E. coli* DH5α (Invitrogen). The TFDs were then amplified by PCR using pGEMTeasy targeted labelled primers chol-Tef (5'- Cholesterol- GGC CGC CAT GGC GGC CGC GGG AAT TC- 3' (**SEQ ID NO: 1**)) and cy5-Ter (5'- Cy5- AGG CGG CCG CGA ATT CAC TAG TGA- 3' (**SEQ ID NO: 2**)) and purified by ethanol precipitation.

### 6.1.3 Transfection procedure

A stock solution of gramicidin (Sigma) was freshly prepared by dissolving 21 mg in 1 ml methanol, diluted prior to use in water and further diluting 1/100 in growth medium to give a final concentration of 60nM. Supplemented media was inoculated 1/100 with the frozen *S*. *aureus* stock and incubated at room temperature for 10 min. TFD-DOTAP complexes were formed by mixing 1 ug of TFD with 2µg of DOTAP (Roche) and incubating at room temperature for 10 min on the bench. The TFD complex was then mixed with inoculated medium, aliquoted into 96 well flat-bottomed plates and growth was analysed by culturing at 37°C with shaking in a BioTek plate reader.

### 6.1.4 Quantification of TFD number by real-time qPCR

Cells from individual wells were washed twice in PBS (phosphate buffered saline) and resuspended in 100µl water. 1µl of each sample was used in each qPCR reaction, mixing DNA standards or samples with primers to either amplify the TFD (Tef and Ter; **SEQ ID NOs 1 & 2**) or 16S rRNA (stypF, 5'- ACG GTC TTG CTG TCA CTT ATA- 3' (**SEQ ID NO: 36**); stypR, 5'- TAC ACA TAT GTT CTT CCC TAA TAA- 3' (**SEQ ID NO: 37**)) in 12.5 ul of SYBR Green DNA mix supplemented with 0.5µl of ROX (Invitrogen). The resultant data was used to calculate the number of TFDs present per genome.

### 6.1.5 RNA Analysis

Cultures were treated with RNA Protect reagent (Qiagen) and the bacteria harvested as described and stored at -80°C. The cell pellets were resuspended in 100µl of TE buffer (10 mM Tris-HCl, I mM EDTA) containing 100µg/ml lysostaphin and incubated at 37°C for 30 min to lyse the cells. 700 µl of RLT buffer was added (Qiagen RNA extraction kit) and RNA harvested as per the manufacturer's protocol. Any remaining DNA was digested using an Invitrogen DNase I, mixing 2.5µg of the RNA sample with 1.25 U DNAsel in 25 µl at room temperature for 15 min. The DNAseI enzyme was deactivated by adding 2.5µl of 25mM EDTA and heating at 65°C for 10 min and the treated RNA used for cDNA synthesis using random primer synthesis (NEB Protoscript). Using standard real-time PCR methods the cDNA was used to quantify relative transcription using primers for the fhu gene (qSAfhuF, 5'- CGT CAA TCA TTG GTC CTA ACG GCT GC- 3' (**SEQ ID NO: 38**); qSAfhuR, 5'- GCC ATC TGC TAC TTC AGG TGA TTG AGG- 3' (**SEQ ID NO: 39**)) and normalising using levels of 16s rRNA (using primers stypF and stypR; **SEQ ID NOs: 36 & 37**).

### 6.2 RESULTS

### 6.2. 1 Transfection of Fur TFD in non-iron-limiting medium.

Iron availability affects the regulation of fifty nine S. *aureus* genes of which seventeen have been shown to be Fur regulated (Allard et al, Microbiol. Infect. (2006) 8:1679-1690; Xiong et al, J. Infect. Dis. (2000) 181:1020-1026; Horsburgh et al, J. Bacteriol. (2001a) 183:468-475). Fur also acts as a positive regulator of some Per-regulated genes, including *katA,* which encodes a catalyse required for oxidative stress resistance (Horsburgh et al, Infect. Immun. (2001b) 69:3744-3754; Morrissey et al, Infect. Immun. (2004) 72:972-979) and consequently *fur* mutants are thus compromised in their ability to prevent toxic hydroxyl radical formation. These diverse and complex regulatory properties may account for the reduced virulence of the *S*. *aureus fur* mutant. To evaluate the applicability of the TFD approach for modulating Fur activity, a TFD was designed incorporating the Fur binding site, which had been identified previously by footprinting experiments performed on the *fhu* promoter of *S*. *aureus* (Xiong *et al.* 2000). The control TFD consisted of an unrelated sequence of similar nucleotide composition that does not occur in the *S*. *aureus* genome; this was to establish whether the action of the Fur TFD was sequence-specific and whether there was a killing effect caused by the transfection procedure alone. Each nucleotide sequence was ligated into the *Escherichia coli* vector pGEMTEasy and the resultant plasmids used with primers flanking the insertion site to generate the TFDs by PCR. The primers were routinely modified at the 5' end, partly to increase the stability of the TFDs by protecting them from exonucleases, but also to functionalise the molecule with cholesterol (forward primer) and a cy5 dye (reverse primer). Hence, the total lengths of TFDs were 60 bp for the Fur TFD and 62 bp for the Control TFD.

To achieve transfection, TFDs were mixed with the commercially available lipid preparation DOTAP to form a stable complex and shield the negative charge of the phosphate backbone. The TFD-DOTAP complex was then mixed with *S. aureus* in BHI, a non-iron limiting medium, which had been supplemented with 60 nM gramicidin. Gramicidin is an antimicrobial peptide with activity against the membranes of Gram-positive bacteria (Herrell and Heilman, J. Clin. Invest. (1941) 20:583-591). Previous experiments by the inventors have established that sub-lethal concentrations of gramicidin were sufficient to allow fusion of the TFD-DOTAP complex to the bacterial membrane and uptake of the TFD. The addition of the transfection reagents together with the control TFD resulted in a reduction of the *S. aureus* growth rate. This is probably due to some bactericidal activity caused though weakening of the cell wall. Transfection using 1 nM of either the Fur or the Control TFD resulted in very similar growth curves in an iron-rich medium (Figure 9). Interestingly, the *fur* knock-out showed a growth defect in rich medium (Horsburgh *et al,* 2001) whereas no difference was seen between the control and Fur_TFD treated sample. One explanation for the difference could be the different growth conditions (for example, poor aeration on 96 well plates) and media but more pertinent could be the fundamental differences between a genetic knock-out and the effects of TFD treatment. The Fur_TFD will prevent binding of any transcription factor capable of binding that site, not only the Fur protein but additional proteins that bind to the same site. Increasingly it is being realised that transcriptional regulation in bacteria is more complex than previously thought and, as in eukaryotes, has inputs from many regulatory networks and hence is modular, with more than one transcription factor affecting expression (Barnard et al, Curr. Opin. Microbiol. (2004) 7:102-108).

Quantitative PCR was performed to establish whether transfection of the cell had occurred and if so how many TFDs had entered per genome. In the absence of gramicidin and DOTAP, TFDs were not detected inside of or attached to the bacterial cells, demonstrating that TFDs do not enter the cells spontaneously and that the washing procedure had successfully removed any TFDs non-specifically associated with the cell surface. In the presence of the transfection reagents, Fur and the Control TFD were detected at 4500 and 5340 TFDs per genome, respectively (Figure 10A). Hence, the transfection procedure was delivering a substantial number of both TFDs into the bacterial cells without having any observable differential effect on growth in iron-rich media.

### 6.2.2 TFD-controlled expression of fhu.

If the Fur_TFD was acting to bind Fur and so prevent it from binding to *fhu* promoter it would be expected that repression of the *fhu* genes should be lifted. To test this, the copy number of *fhu* transcripts (standardised to ribosomal DNA) present in the Fur_TFD-treated sample was compared with the Control sample (Figure 10B). Performing quantitative real-time RT-PCR on RNA isolated from the samples shown in Figure 9 established that the Fur_TFD had made the predicted change to *fhu* transcription: it was five-fold higher than in the Control sample. Hence, transfection of specific TFDs can be used to modify patterns of expression of genes in *S. aureus* known to be essential for pathogenicity.

### 6.2.3 Effect on growth of Fur TFD in iron- limiting media

Using the same transfection protocol the Fur and Control TFDs were introduced into *S*. *aureus* cultured in an iron-limited medium (Skaar et al, Science (2004) 305:1626-8). As expected growth was delayed in this medium when compared with growth in BHI. However, the culture that had been transfected with the Fur TFD failed to show any signs of growth over the course of the experiment (Figure 11), demonstrating that perturbation of iron-regulation under iron-limited conditions is deleterious to growth and replicating the results seen with the Fur knock-out mutant (Horsburgh *et al,* 2001a).

The Fur transcription factor is generally thought of as a globally acting repressor which down-regulates the transcription of iron uptake genes under conditions where iron is in excess, preventing iron toxicity, which is caused by Fenton's reaction (Wandersman & Delepelaire, Ann. Rev. Microbiol. (2004) 58:611-647). Mutation of *S. aureus fur* also has a detrimental effect on virulence with an inability to grow under iron-limiting conditions, resulting in attenuation *in vivo* (Horsburgh *et al.* 2001a). The reasons for this remain unclear, but perhaps reflect the central role of Fur in adapting bacteria to changes in the environment, and a compromised ability to respond to oxidative stress. The effect on virulence does not appear to be unique to *S. aureus.* Mutation of *fur* causes the attenuation of *Vibrio cholerae, Campylobacter jejuni, Helicobacter pylori, Actinobacillus pleuropneumoniae* and *Bacillus cereus in vivo* (Mey et al, Infect. Immun. (2005) 73:8167-8178; Palyada et al, J. Bacteriol. (2004) 186:4714-4729; Bury-Mone et al, Mol. Microbiol. (2004) 53:623-638; Jacobsen et al, Infect. Immun. (2005) 73:3740-3744; Harvie et al, Microbiol. (2005) 151:569-577). Bioinformatic analysis of the *S. aureus* Fur consensus sequence (using MEME software (http://meme.sdsc.edu/meme4_l/intro.html) and previously identified sequences (Horsburgh *et al.* 2001 a) demonstrated that it was highly conserved in *Bacillus cereus, Bacillus anthracis, Bacillus subtilis, Lysteria monocytogenes* as well as in *S. aureus* (Figure 12), predicting activity against other Bacillales.

Antisense molecules have been used to treat pathogenic bacteria. Most notably using modified backbones, such as peptide nucleic acids (Good & Nielsen, Nat. Biotech. (1998) 16:355-358) and morpholino oligonucleotides (Shen et al, Proc. Natl. Acad. Sci. USA (2009) 106:8163-8168), and achieving delivery with cationic peptides. These down-regulate translation of targeted mRNAs at micromolar concentrations to inhibit growth of microbes *in vitro* and *in vivo.* The switch to modified backbones has the advantages that it increases the stability of the oligonucleotide in biological fluids and reduces or removes the negative charge of the phosphate backbone. This later effect has the advantage that modified oligonucleotides are easier to transfect through the negatively charged outer membrane of bacteria. However, as TFDs work by interfering with DNA-protein interactions and it is probable that the phosphate backbone is important for protein recognition it cannot be readily replaced.

In this example, it has been demonstrated that TFDs, as sizeable oligonucleotides with natural backbones, can be efficiently delivered into cells, using a combination of peptides and liposomes to kill bacteria at nanomolar concentrations. TFDs are effective at much lower concentrations as they block an enzymatic process (transcription) whereas antisense work as steric blocks on the products of transcription (mRNA). A further advantage of using TFDs is that they block the expression of many essential genes, rather than having a single target, making them less susceptible to resistance mechanisms.

### Example 7

### Sig TFD inhibits growth of MRSA strains

Cholesterol/Cy5-labeled decoy polynucleotides were prepared as in Example 5.1. Typically 1 µg of Sig TFD was then complexed with 0.3 µl of the liposomal transfection reagent DOTAP (1,2-dioleoyl-3-trimethylammonium propane; Roche) and incubated at room temperature for 10 min. The assays to determine their effect on growth of bacterial cells were performed using 96 well plates, each well containing 200 µl of broth consisting of BHI media (Becton Dickinson) supplemented with 60 nM Gramicidin (Sigma Corporation. Cat# G5002). 1 µl of various concentrations of a Cholesterol/Cy5-labeled decoy complexed with TFD were added to each well and the effect on bacterial growth of clinical isolates of *S*. *aureus* that had been determined to be methicillin-resistant strains (MRSA) was monitored. Growth was monitored by measuring absorbance of the broth at time intervals during incubation. The plates were incubated at 37°C with shaking and absorbance readings (at 450 nM) were taken using a plate reader.

As shown in Figure 13, it was evident that treatment with as little as 10 nM Sig prevented growth of the MRSA strains in BHI medium. A similar amount of the control TFD containing an unrelated sequence of similar length not found in the *S. aureus* genome had no effect on bacterial growth in this media.

### Example 8

### Sig TFD potentiates the action of antibiotics against EMRSA15 (a clincially isolated epidemic strain)

Sig dumbbell TFD was prepared as described in Example 9.1 (see below). A growth assay was carried out as described in Example 5.2 with a well-characterised MRSA strain, EMRSA15. This strain shows resistance to methicillin (being able to grow in 2 mg/ml of the antibiotics), 0.5 mg/ml erythromycin and 0.5 mg/ml Ciprofloxacin (Porter and Damani, J. Hospital Infection (2007) 65:88).

As shown in Table 1 below, the EMRSA15 strain was resensitised to methicillin, erythromycin and ciprofloxacin, in the presence of 10 nM of Sig TFD, as measured by determining the lag-time in growth of the bacterial culture. In such experiments a lag-time of 0 h for the media-treated with Control TFD (as described in Example 5.1) and an antibiotic indicates that the culture began growing at the same time-point as the untreated bacteria grown in media supplemented with the antibiotic. For the cultures treated with the Sig TFD, the lag-time is the delay in growth compared to that seen with the Control TFD in the presence of antibiotic. Hence, lag-times of 6.4 h (in the presence of 2 mg/ml methicillin), 8.2 h (in the presence of 0.5 mg/ml erythromycin) and 8.3 h (in the presence of 0.5 mg/ml Ciprofloxacin) indicate that the Sig TFD acted synergenistically with these antibiotics to retard growth.

The mechanism for this effect is likely to be the Sig TFD preventing or down-regulating the antibiotic-mediated induction of the stress-response regulon, rendering the cells more sensitive. As most antibiotics cause stress-response we would expect if TFDs were used in combination to increase the efficacy of all or the majority of such antibiotics.

**Table 1**

| **Antibiotic** | **Control TFD Lagtime (h)** | **Sig TFD Lagtime (h)** |
|---|---|---|
| Methicillin 2mg/L | 0.2 | 6.4 |
| Erythromycin 0.5 mg/L | -0.3 | 8.2 |
| Ciprofloxacin 0.5 mg/L | 0.3 | 8.3 |

Lagtime is defined as the time that growth is delayed (in comparison to a mock-transfected culture) due to treatment of the TFD.

### Example 9

### Sig TFDs in dumbbell configuration repress growth of a clinically-islated MRSA strain

### 9.1 Preparation of TFD dumbbells by ligation (DB-TFD)

Two oligonucleotides were synthesised, each containing one strand of the recognition site for the *S*. *aureus* alternative sigma protein. At either end of the molecule a small hairpin loop acted to protect the molecule from degradation. Each oligonucleotide was re-suspended in dH₂O at a concentration of 250 pmol/µl.

To form the Sig dumbbell TFD (referred to as SA3 TFD) the following phosphorylated oligonucleotides were synthesised:

SEQ ID NO: 40 – SigDB_SA3: CTTGG TTTTT CCAAG GAA GAT TAG AAA TTA
TTT CGA T GGG TAT ATA ATA

SEQ ID NO: 41 – SigDB_SA3: P-CCG TCT TTT TGA CGG TAT TAT ATA CCC ATC
GAA ATA ATT TCT AAT CTT C

When annealed these formed the following molecule:

T CCAAG gaa gat tag aaa tta ttt cgat ggg tat ata ata PCCGTC T

TTT TTT

T GGTTCPCTT CTA ATC TTT AAT AAA GCTA CCC ATA TAT TAT GGCAG T

Typically 30 µl of each oligonucleotide was mixed with 27 µl of dH₂O and annealed using the following PCR programme: ANNEAL: 95°C 3 min, cool at -0.1°C/s to 8°C, end. Following which, 10 µl of 10xNEB Ligase buffer and 3 µl HC T4 DNA ligase (NEB) were added. The mixture was incubated overnight at 16°C. The material was then extensively digested with T7 exonuclease (NEB) to remove any unligated oligonucleotides and then recovered by two rounds of ethanol preicpitation.

A DB_TFD was also prepared containing a scrambled version of the Sig binding site. In this instance the phosphorylated primers used were:

SEQ ID NO: 42 – SigScr_SA1: CTTGG TTTTT CCAAG TAG AAA GAA GAT TTA
GGG CGA T TTT ATA ATA TAT

SEQ ID NO: 43 – SigScr_SA2: CCG TCT TTT TGA CGG ATA TAT TAT AAA ATC
GCC CTA AAT CTT CTT TCT A

### 9.2 Performing growth studies in 96-well plates

A growth assay was performed as described in Example 5.1 using the Sig TFD in its dumbbell configuration and a clinically-isolated MRSA strain.

As shown in Figure 14, Sig TFD concentrations as low as 10 nM could inhibit cell growth, whereas a similar concentration of the control TFD could not.

### Example 10

### WalR DB-TFD prevents growth of EMRSA15

### 10.1 Preparation of DB TFDs containing WalR binding site

The binding sequence for WalR was incorporated into dumbbell oligonucleotides and prepared as described in Example 9.1. The sequences of these phosphorylated oligonucleotides were:

SEQ ID NO: 44 – WalRDB2.1: CTTGG TTTTT CCAAG TAA TGA ATG A GTT TAA
AGC CCA TGT AAA AG GGG TAT CAG TAC

SEQ ID NO: 45 – WalRDB2.2: CCC TCT TTT TGA GGG GTA CTG ATA CCC CTT
TTA CAT GGG CTT TAA ACT CAT TCA TTA

A second pair of oligonucleotides containing a scrambled version of the WalR binding site were used to generate the control dumbbell (Scr. WalR). The sequences of these phosphorylated oligonucleotides were:

SEQ ID NO: 46 – WalRDB3.1: P-CTTGG TTTTT CCAAG GTA ATA TGA C AAG ATT
GTA AAT GAC CTA GTT GAG AG ATGCCA

SEQ ID NO: 47 – WalRDB3.1: P-CCC TCT TTT TGA GGG TGG CAT CTC TCA ACT
AGG TCA TTT ACA ATC TTG TCA TAT TAC

### 10.2 Transfection of EMRSA15 by co-treatment with Lysostaphin

Growth assays were performed as described for Example 7.1 with the sole difference being that the media was supplemented with between 0.5 and 2.5 pg/ml Lysostaphin instead of Gramicidin. Lysostaphin is a lysozyme-like enzyme that is specific for the walls of *S*. *aureus.*

As can be seen in Figure 15, as little as 10 nM of Sig DB-TFD was capable of inhibiting bacterial growth, whereas similar amounts of the control DB-TFD had no effect.

### Example 11

### WalR TFD inhibits growth of MRSA in a mouse sepsis model

The TFD used in this Example blocks the action of the two component system WalKR. These proteins form an essential signal transduction pathway that controls expression of a small regulon of genes involved in cell wall metabolism, particularly peptidoglycan synthesis.

### 11.1 METHODS

### 11.1.1 Bacterial strains and growth

*S. aureus* EMRSA16 cultures were grown and maintained in BHI media (Becton Dickenson). Frozen stocks were prepared by mixing equal volumes of a culture in early exponential growth (OD of 0.3 at A₆₀₀) with 50% glycerol and freezing at -80°C.

### 11.1.2 Preparation of TFDs

TFDs were prepared in a dumbbell configuration as previously described (Ahn *et al,* 2003). TFDs were prepared by ligating pairs of phosphorylated oligonucleotides to form a TFD containing the binding site for the WalR protein as it occurs in the promoter of lytM (WalR_TFD) or a TFD containing a scrambled version of the binding site (Scr_TFD). The oligonucleotides were resuspended at a final concentration of 100 pmol/ul in a T4 DNA ligase buffer (New England Biolabs) and 400 U of T4 DNA ligase and incubated at 16°C overnight. The following morning the reaction buffer was supplemented with 10 U Exonuclease I (NEB) and digested for 30 min at 37°C, before purification by phenolchloroform extraction and ethanol precipitation. The TFDs were resuspended in water at a concentration of 1mM.

The sequences of the pairs of oligonucleotides used to form WalR_TFD were: 
SEQ ID NO: 48 - WalR1: 5'- P-CTT GGT TTT TCC AAG TAA TGA ATG AGT TTA
AAG CCC ATG TAA AAG GGG TAT CAG TAC- 3'
 and

SEQ ID NO: 49 - WalR2: 5'- P-CCC TCT TTT TGA GGG GTA CTG ATA CCC CTT
TTA CAT GGG CTT TAA ACT CAT TCA TTA- 3'.

For Scr_TFD the sequences were: 
SEQ ID NO: 46 – WalRDB3.1: 5'- P-CTT GGT TTT TCC AAG GTA ATA TGA CAA
GAT TGT AAA TGA CCT AGT TGA GAG ATG CCA- 3'
 and

SEQ ID NO: 47 – WalRDB3.1: 5'- P-CCC TCT TTT TGA GGG TGG CAT CTC TCA
ACT AGG TCA TTT ACA ATC TTG TCA TAT TAC- 3'.

### 11.1.3 Transfection of MRSA in vitro

TFD-DOTAP complexes were formed by mixing 1 ug of TFD with 2µg of DOTAP (Roche) and incubating at room temperature for 10 min on the bench. The TFD complex was then mixed with inoculated medium supplemented with 10 ng/ml Lysostaphin (Sigma; L9043), aliquoted into 96 well flat-bottomed plates and growth was analysed by culturing at 37°C with shaking in a BioTek plate reader.

### 11.1.4 Mouse sepsis model

The mouse sepsis study was performed by Euprotec (UK). Mice used in this study, male CD1 mice were supplied by Charles River (Margate UK) and were specific pathogen free (16-18g at delivery). All mice weighed 22-25g at the beginning of the experiment.

### 11.1.5 Tolerability study

For the tolerability study, animals were treated in groups of 2 mice per treatment group, therefore 10 in total for the study. All the mice were weighed on day 1 of the study and placed randomly in to boxes. The mice had the following treatments administered intravenously using 10 ml/kg, the five treatment groups were: 100 µM WalR_TFD; 2.5 mM DOTAP; 6.3 µg/ml Lysostaphin; a mixture of TFD/DOTAP/Lysostaphin; saline. The mice were weighed dialy post-treatment over a 100 h period before they were euthenised and lungs, liver, spleen and kidneys were removed and visually examined and weighed.

### 11.1.6 PK Study

For the PK study, animals were treated in groups of 3 mice per time point, therefore 24 in total for the study. Mice were treated with intravenous delivery of a mixture of 5nM/kg WalR_TFD, 25nM/kg DOTAP, 31.5 m/kg Lysotsaphin. Sets of mice were bled by cardiac puncture following isofluorane anaesthesia at 5 minutes, 15 minutes, 30 minutes, 1 hour, 2 hours, 4 hours, 8 hours and 24 hours post dose. All samples were collected as plasma (anticoagulated with heparin and stored on ice before separation). Following blood collection kidneys were removed and homogenized in 1ml ice cold PBS. TFDs were detected in the biological samples with qPCR.

### 11.1.7 Tissue burden study

For the tissue burden study, animals were treated in groups of 8 mice per treatment group, therefore 48 in total for the study. 2 x 10ml cultures of *Staphylococcus aureus* EMRSA 16 were prepared and placed on orbital shaker (220rpm) overnight at 37°C. The following day, the *Staphylococcus aureus* EMRSA 16 cultures were removed from shaker, pelleted and washed twice before being resuspended in saline to an OD of 0.132 (1.5 x 10⁸cfu/ml). This stock solution of *Staphylococcus aureus* EMRSA 16 was then further diluted 1:1.5 in saline (1 x 108 cfu/ml) i.e. 2.0 x 10⁷ bacteria per mouse. 48 mice were then infected with 0.2ml of the 1.0 x 108/mL suspension. The number of *Staphylococcus aureus EMRSA 16* bacteria per mL in the remainder of the suspensions after inoculation was also counted to confirm infection load. Mice were treated 1, 9 and 17 hours post infection with either compound or vehicle, though vancomycin was only administered after 1h. The treatments were as previously a mixture of TFD/DOTAP/Lysostaphin using a concentration of 1 nM/kg TFD in a 5:1 molar ratio with DOTAP and 63µg/kg Lysostaphin. After 25 hours post infection all animals were weighed and then euthanized. The kidneys were immediately removed and homogenised in ice cold sterile phosphate buffered saline + 0.05% Tween 80. Organ homogenates were quantitatively cultured onto CLED agar and incubated at 37°C for up to 3 days and colonies counted. The data from the culture burdens was analysed by the Kruskal-Wallis test using Stats Direct.

### 11.2 RESULTS

### 11.2.1 WalR_TFD rapidly kills MRSA growth in vitro

The highly conserved two component system WalK/WalR (also referred to as YycG/YycF) has been shown in genetic knockout experiments to be essential for viability in *S. aureus* and other Gram-positive pathogens. The binding site for the transcription factor WalR has been identified in several promoters of genes in the regulon, including lytM/SA0265 (Dubrac, Boneca *et al.* 2007). It was this version that was used in the WalR_TFD and a scrambled version where the sequence was randomly re-arranged, Scr_TFD.

EMRSA-16, an MRSA strain endemic in UK hospitals, was used in the study (Cox, Mallaghan et al. 1995). TFDs were mixed with a commercially available lipid formulation, DOTAP, and mixed with media inoculated with EMRSA-16 and Lysostaphin. Lysostaphin is a lysozyme agent that acts on the walls of *S*. *aureus,* use of it in this experiment was to thin the outer peptidoglycan layer of the bacteria and allow the TFD to transfect the cell following fusion of the DOTAP carrier with the exposed inner membrane of the bacterium. Using this transfection protocol it was observed that treatment with the control TFD, Scr_TFD, had no detectable effect on bacterial growth when compared to an untreated sample. However, the WalR_TFD prevented growth of EMRSA-16 in vitro at a concentration of 1 nM (Figure 16). Total viable counts confirmed the results that treatment with WalR_TFD reduced the number of living cells by five-fold orders of magnitude (Figure 17A). The bacteria that were not killed by WalR_TFD treatment were sub-cultured in order to determine whether there was any incidence of resistance. The process was repeated a total of four times and both the reduction in viable counts (Figure 17B) and *in vitro* growth curves were recorded (Figure 17C, black squares: bacteria in fourth passage). Hence, no evidence for the development of a resistance mechanism to TFD-mediated killing.

In order to determine whether TFDs killed bacteria on contact or needed prolonged exposure to work, EMRSA-16 were transfected with 1 nM WalR_TFD/DOTAP complex and incubated for 30 minutes before being microdiluted into fresh media, without any TFD complexes or Lysostaphin. *In vitro* growth was followed for a further 48 hours (Figure 18) and it was evident that no growth was observed, confirming that TFDs effectively killed the cells on first contact.

### 11.2.2 Tolerability study

All drugs were well tolerated following IV administration; there were no acute events to report. Following treatment, mice fed and drank normally with no signs of distress. The weight increase of the treated mice was the same as the vehicle controls. Autopsy showed no gross abnormalities of kidneys, lungs, liver or GI tract. The weights of kidneys, lungs and liver were within the normal range. All test compounds are tolerated and suitable for further dosing up to the maximum dose used in this tolerability study.

### 11.2.3 Tissue burden study

The infectious dose administered was targeted at 2.0 x 10⁷ bacteria per mouse to ensure a relatively mild infection was established (this is more sensitive to treatment). The mice were treated with systemic injection of either the vehicle, 1 nM of the Scr_TFD complex, 1 nM of WalR_TFD complex or vancomycin, used at a concentration sufficient to achieve a 2-fold reduction in colony forming units (cfu). Following treatment the mice were sacrificed and the burden found within the kidneys measured (Figure 19). Statistical analysis of the results showed that the WalR_TFD-treated mice achieved a similar reduction in burden to that achieved by vancomycin when compared to the Scr_TFD control (Table 2 below).

**Table 2. Statistical analysis of in vivo results. Kruskal-Wallis: all pairwise comparisons (Dwass-Steel-Chritchlow-Fligner) for Scr_TFD**

| | 1 nM Scr_TFD | 1 nM WalR_TFD | Vancomycin | Vehicle |
|---|---|---|---|---|
| 1 nM Scr_TFD | | 0.0043 | 0.0062 | 0.7804 |
| 1 nM WalR_TFD | | | 0.2549 | 0.0062 |
| Vancomycin | | | | 0.0173 |
| Vehicle | | | | |

The WalR_TFD in this experiment was found to have a rapid bacteriocidal activity at nanomolar concentrations against MRSA both *in vitro* and *in vivo.*

### Example 12

### Transfection of E. coli with FabB TFD sensitises the bacteria to antibiotics that inhibit fatty-acid synthesis

### 12.1 Preparation of FabB TFD

FabB TFDs were designed to incorporate the binding site for the transcriptional regulator of fatty acid synthesis enzymes, FadR, which occurs upstream of the FabB gene in *Escherichia coli.* The FabB gene encodes an enzyme involved in fatty acid synthesis (J. Bacteriology (2005) 183:5292).

PCR TFDs were made as described in Example 4.1 with the exception that a 60 base pair portion of the FabB promoter that was cloned into the pGEMT-Easy vector. The oligonucleotides used to amplify the promoter sequence were:
**SEQ ID NO: 50 -** fabBf: TCT TTA AAT GGC TGA TCG GAC TTG
**SEQ ID NO: 51 -** fabBr: AGT AAG TTT CGA ATG CAC AAT AGC GTA

A control TFD having a the sequence that gave rise to a similar sized PCR fragment when used in an amplification reaction with genomic DNA isolated from *Mycobacterium smegmatis* was also generated. The sequences of these oligonucleotides were:
**SEQ ID NO: 52 -** WhiB7.f: CAC CAG CCG AAA AGG CCA CGG
**SEQ ID NO: 53 -** WhiB7.r: CAA AAA TGG CCA CGG ATC CGG GTG

### 12.2 Growth assays

The effect of TFDs on the growth of *E. coli* bacterial cultures was measured as described in Example 4.1 with a few modifications: the media used was LB; the co-transfectant used was 40 nM polymixin, rather than gramicidin; the media was also supplemented with 10 µg/ml Cerulenin (CER). CER is an antibiotic that acts on fatty acid synthesis. At the concentrations used it has no noticeable effect on growth of *E*. *coli.*

Figure 20 shows that treatment of *E*. *coli* with 10 nM of the FabB TFD, but not a control TFD (WhiB7; containing a sequence known not to occur within the *E. coli* genome), rendered the bacteria sensitive to the action of cerulenin. The antibiotic used, cerulenin (CER), does begin to inhibit cell growth in the presence of the control TFD at 20 ug/ml, by which point the cells are being killed by the synergenistic action of the FabB TFD.

As the FabB TFD is designed to downregulate genes of the fatty acid synthesis operon, it is our hypothesis that this TFD could be used in combination with any antibiotic that either perturbs fatty acid synthesis, directly or indirectly, to increase the efficacy of that antibiotic or counteract a resistance mechanism.

### Example 13

### Transfection of a TFD containing the recognition sequence for the σ54 factor of Klebsiella pneumoniae retards bacterial growth

A PCR TFD containing the binding site for the alternative sigma factor, σ54, from the pathogenic organism *Klebsiella pneumoniae,* was made and tested. Compared to the activity of an unrelated control TFD, the Sig TFD inhibited growth of the cells. σ54 is another example of a sigma factor that controls regulation of stress response in bacteria.

### 13.1 Preparation of TFD

The TFD was prepared as described in Example 5.1. The sequences of the annealed oligonucleotides used were:
**SEQ ID NO: 54 -** Ks54f: P-CCG ATA AGG GCG CAC GGT TTG CAT GGT TAT A
**SEQ ID NO: 55 -** Ks54r: P-ATA ACC ATG CAA ACC GTG CGC CCT TAT CGG A

### 13.2 Transfection of K. pneumoniae

Growth assays were performed as described in Section 4.1 with the difference being that the media used was M9 media (Sambrook et al (1989) Molecular Cloning. 2nd Edition vol.3 p.A3). The media was not supplemented with any co-transfectant.

Transfection was achieved by complexing the TFD with a cell penetrating peptide. The peptide consisted of two portions: a linear chain of nine D-arginines and a previously described peptide sequence found to be capable of penetrating the membrane of *K*. *pneumoniae* (Vaara, Antimicrobial Agents and Chemotherapy (1996) 40:1801). The positively charged arginines (R9) serve to bind the phosphate backbone of the TFD through a charge interaction. The use of such R9 tails to complex transfecting moieties to transfect bacteria has been described previously (Kim, Molecular Therapy (2006) 14:343). The entire sequence of the peptide used was:
**SEQ ID NO: 56** - IKFLKFLKFL-(D-arginine)9

TFDs were mixed with increasing amounts of IKFLKFLKKL-R9 peptide in a TE based buffer supplemented with 5% glucose. The mixture was incubated at room temperature for 1 hour and then either used directly in transfections or analysed by agarose gel electrophoresis. Typically the minimum amount of IKFLKFLKKL-R9 was used that caused the complex with DNA not to run in the gel; i.e. the charge of the nucleic acid backbone had been neutralised by binding of poly-arginine. IKFLKFLKKL-R9 -TFD conjugates were mixed at various concentrations into 200 µl of culture in a 96 well plate.

Figure 21 shows that 10 nM of Sig TFD was sufficient to inhibit growth of the *K*. *pneumoniae* culture, whereas no inhibition was seen with the control TFD.

### Example 14

### Bioinformatical analysis to detect the occurrences of the WalR binding sites within S. aureus and deduce its consensus sequence.

A MEME search (http://meme.sdsc.edu/meme4/cgi-bin/meme.cgi) was used to find additional occurrences of published examples of the WalR binding site in *S. aureus* (SA) and other pathogenic organisms where it was found to occur, such as *Enterococcus faecium* (EF), *Streptococcus pneumoniae* (SP), *Lysteria monocytogenes* (LM) and *Streptococcus mutans* (SM).

A subset of the hits derived from the search is shown in Figure 22. From the complete set a consensus site was derived, having the sequence:
TGT WAW NNN NNT GTA AW [**SEQ ID NO: 57**]

The IUPAC single letter code is used, where: W is A or T.

The WalR TFD consensus sequence is therefore expected to affect the growth of the organisms listed above.

### Example 15

### Bioinformatical analysis to detect the occurrences of the SigB binding sites within S. aureus and deduce its consensus sequence.

A MEME search (http://meme.sdsc.edu/meme4/cgi-bin/meme.cgi) was used to find examples of occurrences (with E-values <100) of matches to a consensus sequence of the SigB binding site in *S*. *aureus* in Bacillales (Gram-positive infections) of which representative genera include *Bacillus, Listeria,* and *Staphylococcus.*

Examples of the hits derived from the search is shown in Figure 23. From the complete set, a consensus site was derived, having the sequence:
GKT TWA NNN NNN NNN NNN NNK GGT AW [**SEQ ID NO: 58**]

The IUPAC single letter code is used, where: K is G or T; W is A or T.

### Example 16

### Bioinformatical analysis to detect the occurrences of the Sig binding sites within Kelbsiella pneumoniae and deduce its consensus sequence

A TFD containing the binding site for the alternative sigma factor (sigma 54) was taken from the promoter region of the *K. pneumoniae glnA* gene, as described in Barrios et al. 1999 (Nucl. Acids Res. 22: 4305-4313) and, when incorporated into a PCR_TFD, was shown to kill *K. pneumoniae* cells *in vitro* (see Figure 24A).

The TFD sequence used was:
**SEQ ID NO: 59** - KP_Sig TGG CAC aga ttT CGC T

Cells were transfected as described in Example 13, using a cell penetrating peptide. The TFD containing the KP_Sig sequence prevented cell growth *in vitro* while the two controls showed no effect on cell growth. The two controls used were: untreated cells (*K*. *pneumoniae*) and a control TFD consisting of a scrambled sequence.

Similar binding sites found in other *K. pneumoniae* genes (*nifB, nifE, nifH, nifJ, nifL, nifM* and *nifU*) were used to define a consensus sequence:
**SEQ ID NO: 60** - TGG NNN NNN WTT TGC W

The IUPAC single letter code is used, where W is A or T.

### Organization Applicant

Street : Norwich Bio-Incubator, Norwich Research Park, Colney Lane
City : Norwich
State :
Country : UK
PostalCode : NR4 7UH
PhoneNumber :
FaxNumber : EmailAddress :

<110> OrganizationName : Procarta Biosystems Limited

### Application Project

<120> Title : Transcription Factor Decoys
   <130> AppFileReference : P3950WO
   <140> CurrentAppNumber :
   <141> CurrentFilingDate : - -

### Earlier Applications

<150> PriorAppNumber : US 61/102,414
   <151> PriorFilingDate : 2008-10-03

### Earlier Applications

<150> PriorAppNumber : US 61/167,592
   <151> PriorFilingDate : 2009-08-04

### Earlier Applications

<150> PriorAppNumber : GB 09069130
   <151> PriorFilingDate : 2009-04-08

### Earlier Applications

<150> PriorAppNumber : PCT/GB2008/003353
   <151> PriorFilingDate : 2008-10-03

### Sequence

<213> OrganismName :
   <400> PreSequenceString :
   ggccgccatg gcggccgcgg gaattc 26
   <212> Type : DNA
   <211> Length : 26
   SequenceName : SEQ ID NO: 1
   SequenceDescription :

### Sequence

<213> OrganismName :
   <400> PreSequenceString :
   aggcggccgc gaattcacta gtg 23
   <212> Type : DNA
   <211> Length : 23
   SequenceName : SEQ ID NO: 2
   SequenceDescription :

### Sequence

<213> OrganismName :
   <400> PreSequenceString :
   cttggttttt ccaagagaag agcccgccat ggcggccgcg ggaattc 47
   <212> Type : DNA
   <211> Length : 47 SequenceName : SEQ ID NO: 3 SequenceDescription :

### Sequence

<213> OrganismName :
   <400> PreSequenceString :
   ccgtcttttt gacggcgaag agcaggcggc cgcgaattca ctagtga 47
   <212> Type : DNA
   <211> Length : 47
   SequenceName : SEQ ID NO: 4
   SequenceDescription :

### Sequence

<213> OrganismName : M. smegmatis str MC2 155
   <400> PreSequenceString :
   tggccacgga tccgggtgac tgcgggtccg tggcct 36
   <212> Type : DNA
   <211> Length : 36
   SequenceName : SEQ ID NO: 5
   SequenceDescription :

### Sequence

<213> OrganismName : E. coli str K12
   <400> PreSequenceString :
   tttattccga actgatcgga cttgttcagc gtacacgtgt tagctatcct gcgtgcttca 60
<212> Type : DNA
   <211> Length : 60 SequenceName : SEQ ID NO: 6 SequenceDescription :

### Sequence

<213> OrganismName : S. aureaus
   <400> PreSequenceString :
   gctattttgt aatgacaatg taatgagttt agtaaaaa 38
   <212> Type : DNA
   <211> Length : 38
   SequenceName : SEQ ID NO: 7
   SequenceDescription :

### Sequence

<213> OrganismName : S. aureus
   <400> PreSequenceString :
   attacaaatt tgtaacagac ttatttta 28
   <212> Type : DNA
   <211> Length : 28
   SequenceName : SEQ ID NO: 8
   SequenceDescription :

### Sequence

<213> OrganismName : M. smegmatis str MC2 155
   <400> PreSequenceString : 
caccagccga aaaggccacg gacccgcagt cacccggatc cgtggccatt tttgtcggac 60
cccccgagaa atctggtcgc aggatccatc agctcagaca gatcac 106
 <212> Type : DNA
   <211> Length : 106
   SequenceName : SEQ ID NO: 9
   SequenceDescription :

### Sequence

<213> OrganismName : E. coli K12
   <400> PreSequenceString : 
agtaagtttc gaatgcacaa tagcgtacac ttgtacgccg aacaagtccg atcagccatt 60
taa 63
 <212> Type : DNA
   <211> Length : 63
   SequenceName : SEQ ID NO: 10
   SequenceDescription :

### Sequence

<213> OrganismName : S. aureus
   <400> PreSequenceString :
   gctattttgt aatgacaatg taatgagttt agtaaaaa 38
   <212> Type : DNA
   <211> Length : 38
   SequenceName : SEQ ID NO: 11
   SequenceDescription :

### Sequence

<213> OrganismName : S. aureus
   <400> PreSequenceString :
   attacaaatt tgtaacagac ttatttta 28
   <212> Type : DNA
   <211> Length : 28
   SequenceName : SEQ ID NO: 12
   SequenceDescription :

### Sequence

<213> OrganismName : S. aureus
   <400> PreSequenceString :
   ttattatata cccatcgaaa taatttctaa tcttc 35
   <212> Type : DNA
   <211> Length : 35
   SequenceName : SEQ ID NO: 13
   SequenceDescription :

### Sequence

<213> OrganismName : K. pneumoniae
   <400> PreSequenceString :
   ccgataaggg cgcacggttt gcatggttat 30
   <212> Type : DNA
   <211> Length : 30 SequenceName : SEQ ID NO: 14 SequenceDescription :

### Sequence

<213> OrganismName : S. aureus
   <400> PreSequenceString :
   actacaagta ctattagtaa tagttaaccc tt 32
   <212> Type : DNA
   <211> Length : 32
   SequenceName : SEQ ID NO: 15
   SequenceDescription :

### Sequence

<213> OrganismName : E. coli
<400> PreSequenceString :
   gataatgata atcattatc 19
   <212> Type : DNA
   <211> Length : 19
   SequenceName : SEQ ID NO: 16
   SequenceDescription :

### Sequence

<213> OrganismName : H. pylori
   <400> PreSequenceString :
   gttgtcccat aattatagca taaatgataa tgaaaaagta aa 42
   <212> Type : DNA
   <211> Length : 42 SequenceName : SEQ ID NO: 17 SequenceDescription :

### Sequence

<213> OrganismName : C. difficile
   <400> PreSequenceString :
   aagtttacaa aattatatta gaataacttt tttatt 36
   <212> Type : DNA
   <211> Length : 36 SequenceName : SEQ ID NO: 18 SequenceDescription :

### Sequence

<213> OrganismName : P. aeruginosa
   <400> PreSequenceString :
   aaatgtgatc tagatcacat tt 22
   <212> Type : DNA
   <211> Length : 22
   SequenceName : SEQ ID NO: 19
   SequenceDescription :

### Sequence

<213> OrganismName : P.aeruginosa
   <400> PreSequenceString :
   cactctgcaa tccagttcat aaatcc 26
   <212> Type : DNA
   <211> Length : 26
   SequenceName : SEQ ID NO: 20
   SequenceDescription :

### Sequence

<213> OrganismName : P. aeruginosa
   <400> PreSequenceString :
   gtaacagcgg aaccactgca cag 23
   <212> Type : DNA
   <211> Length : 23
   SequenceName : SEQ ID NO: 21
   SequenceDescription :

### Sequence

<213> OrganismName : K. pneumoniae
   <400> PreSequenceString :
   gctttgcact accgcggccc atccctgccc caaaacgatc gct 43
   <212> Type : DNA
   <211> Length : 43
   SequenceName : SEQ ID NO: 22
   SequenceDescription :

### Sequence

<213> OrganismName : H. pylori
   <400> PreSequenceString :
   ataatcataa tgattaaagt tttcatattc attataaatc cgtttacaca attatt 56
<212> Type : DNA
   <211> Length : 56
   SequenceName : SEQ ID NO: 23
   SequenceDescription :

### Sequence

<213> OrganismName : H. pylori
   <400> PreSequenceString : 
gaaattgttc tatttattat ccatttgctt attaataatt ggttgttaat tttggtttag 60
a 61
 <212> Type : DNA
   <211> Length : 61
   SequenceName : SEQ ID NO: 24
   SequenceDescription :

### Sequence

<213> OrganismName : S. aureus
   <400> PreSequenceString :
   tgaacacctt cttttta 17
   <212> Type : DNA
   <211> Length : 17
   SequenceName : SEQ ID NO: 25
   SequenceDescription :

### Sequence

<213> OrganismName : S. aureus
<400> PreSequenceString :
   agaaagacaa acaggagtaa 20
   <212> Type : DNA
   <211> Length : 20
   SequenceName : SEQ ID NO: 26
   SequenceDescription :

### Sequence

<213> OrganismName : S. aureus
   <400> PreSequenceString :
   gaagaaacaa aaagcagcat 20
   <212> Type : DNA
   <211> Length : 20
   SequenceName : SEQ ID NO: 27
   SequenceDescription :

### Sequence

<213> OrganismName : S. aureus
   <400> PreSequenceString :
   gaagattaga aattatttcg atgggtatat aataa 35
   <212> Type : DNA
   <211> Length : 35
   SequenceName : SEQ ID NO: 28
   SequenceDescription :

### Sequence

<213> OrganismName : S. aureus
   <400> PreSequenceString :
   tattatatac ccatcgaaat aatttctaat cttca 35
   <212> Type : DNA
   <211> Length : 35
   SequenceName : SEQ ID NO: 29
   SequenceDescription :

### Sequence

<213> OrganismName : S. aureus
   <400> PreSequenceString :
   actacaagta ctattagtaa tagttaaccc ta 32
   <212> Type : DNA
   <211> Length : 32
   SequenceName : SEQ ID NO: 30
   SequenceDescription :

### Sequence

<213> OrganismName : S. aureus
   <400> PreSequenceString :
   agggttaact attactaata gtacttgtag ta 32
   <212> Type : DNA
   <211> Length : 32
   SequenceName : SEQ ID NO: 31
   SequenceDescription :

### Sequence

<213> OrganismName : S. aureus
   <400> PreSequenceString :
   attacaaatt tgtaacagac ttatttta 28
   <212> Type : DNA
   <211> Length : 28
   SequenceName : SEQ ID NO: 32
   SequenceDescription :

### Sequence

<213> OrganismName : S. aureus
   <400> PreSequenceString :
   aaaataagtc tgttacaaat ttgtaata 28
   <212> Type : DNA
   <211> Length : 28
   SequenceName : SEQ ID NO: 33
   SequenceDescription :

### Sequence

<213> OrganismName :
   <400> PreSequenceString :
   tggccacgga tccgggtgac tgcgggtccg ta 32
   <212> Type : DNA
   <211> Length : 32
   SequenceName : SEQ ID NO: 34
   SequenceDescription :

### Sequence

<213> OrganismName :
   <400> PreSequenceString :
   acggacccgc agtcacccgg atccgtggcc aa 32
   <212> Type : DNA
   <211> Length : 32
   SequenceName : SEQ ID NO: 35
   SequenceDescription :

### Sequence

<213> OrganismName :
   <400> PreSequenceString :
   acggtcttgc tgtcacttat a 21
   <212> Type : DNA
   <211> Length : 21
   SequenceName : SEQ ID NO: 36
   SequenceDescription :

### Sequence

<213> OrganismName :
   <400> PreSequenceString :
   tacacatatg ttcttcccta ataa 24
   <212> Type : DNA
   <211> Length : 24
   SequenceName : SEQ ID NO: 37
   SequenceDescription :

### Sequence

<213> OrganismName : S. aureus
   <400> PreSequenceString :
   cgtcaatcat tggtcctaac ggctgc 26
   <212> Type : DNA
   <211> Length : 26
   SequenceName : SEQ ID NO: 38
   SequenceDescription :

### Sequence

<213> OrganismName : S. aureus
   <400> PreSequenceString :
   gccatctgct acttcaggtg attgagg 27
   <212> Type : DNA
   <211> Length : 27
   SequenceName : SEQ ID NO: 39
   SequenceDescription :

### Sequence

<213> OrganismName : S. aureus
   <400> PreSequenceString :
   cttggttttt ccaaggaaga ttagaaatta tttcgatggg tatataata 49
   <212> Type : DNA
   <211> Length : 49
   SequenceName : SEQ ID NO: 40
   SequenceDescription :

### Sequence

<213> OrganismName : S. aureus
   <400> PreSequenceString :
   ccgtcttttt gacggtatta tatacccatc gaaataattt ctaatcttc 49
   <212> Type : DNA
   <211> Length : 49
   SequenceName : SEQ ID NO: 41
   SequenceDescription :

### Sequence

<213> OrganismName : S. aureus
   <400> PreSequenceString :
   cttggttttt ccaagtagaa agaagattta gggcgatttt ataatatat 49
   <212> Type : DNA
   <211> Length : 49
   SequenceName : SEQ ID NO: 42
   SequenceDescription :

### Sequence

<213> OrganismName : S. aureus
   <400> PreSequenceString :
   ccgtcttttt gacggatata ttataaaatc gccctaaatc ttctttcta 49
   <212> Type : DNA
   <211> Length : 49
   SequenceName : SEQ ID NO: 43
   SequenceDescription :

### Sequence

<213> OrganismName : S. aureus
   <400> PreSequenceString :
   cttggttttt ccaagtaatg aatgagttta aagcccatgt aaaaggggta tcagtac 57
<212> Type : DNA
   <211> Length : 57
   SequenceName : SEQ ID NO: 44
   SequenceDescription :

### Sequence

<213> OrganismName : S. aureus
   <400> PreSequenceString :
   ccctcttttt gaggggtact gatacccctt ttacatgggc tttaaactca ttcatta 57
<212> Type : DNA
   <211> Length : 57
   SequenceName : SEQ ID NO: 45
   SequenceDescription :

### Sequence

<213> OrganismName : S. aureus
   <400> PreSequenceString :
   cttggttttt ccaaggtaat atgacaagat tgtaaatgac ctagttgaga gatgcca 57
<212> Type : DNA
   <211> Length : 57
   SequenceName : SEQ ID NO: 46
   SequenceDescription :

### Sequence

<213> OrganismName : S. aureus
   <400> PreSequenceString :
   ccctcttttt gagggtggca tctctcaact aggtcattta caatcttgtc atattac 57
<212> Type : DNA
   <211> Length : 57
   SequenceName : SEQ ID NO: 47
   SequenceDescription :

### Sequence

<213> OrganismName : S. aureus
<400> PreSequenceString :
   cttggttttt ccaagtaatg aatgagttta aagcccatgt aaaaggggta tcagtac 57
<212> Type : DNA
   <211> Length : 57
   SequenceName : SEQ ID NO: 48
   SequenceDescription :

### Sequence

<213> OrganismName : S. aureus
   <400> PreSequenceString :
   ccctcttttt gaggggtact gatacccctt ttacatgggc tttaaactca ttcatta 57
<212> Type : DNA
   <211> Length : 57
   SequenceName : SEQ ID NO: 49
   SequenceDescription :

### Sequence

<213> OrganismName :
   <400> PreSequenceString :
   tctttaaatg gctgatcgga cttg 24
   <212> Type : DNA
   <211> Length : 24
   SequenceName : SEQ ID NO: 50
   SequenceDescription :

### Sequence

<213> OrganismName : S. aureus
   <400> PreSequenceString :
   agtaagtttc gaatgcacaa tagcgta 27
   <212> Type : DNA
   <211> Length : 27
   SequenceName : SEQ ID NO: 51
   SequenceDescription :

### Sequence

<213> OrganismName : M. smegmatis
   <400> PreSequenceString :
   caccagccga aaaggccacg g 21
   <212> Type : DNA
   <211> Length : 21
   SequenceName : SEQ ID NO: 52
   SequenceDescription :

### Sequence

<213> OrganismName : M. smegmatis
   <400> PreSequenceString :
   caaaaatggc cacggatccg ggtg 24
   <212> Type : DNA
   <211> Length : 24
   SequenceName : SEQ ID NO: 53
   SequenceDescription :

### Sequence

<213> OrganismName : K. pneumoniae
   <400> PreSequenceString :
   ccgataaggg cgcacggttt gcatggttat a 31
<212> Type : DNA
   <211> Length : 31
   SequenceName : SEQ ID NO: 54
   SequenceDescription :

### Sequence

<213> OrganismName : K. pneumoniae
   <400> PreSequenceString :
   ataaccatgc aaaccgtgcg cccttatcgg a 31
   <212> Type : DNA
   <211> Length : 31
   SequenceName : SEQ ID NO: 55
   SequenceDescription :

### Sequence

<213> OrganismName :
   <400> PreSequenceString :
   IKFLKFLKKL R 11
   <212> Type : PRT
   <211> Length : 11
   SequenceName : SEQ ID NO: 56
   SequenceDescription :

### Sequence

<213> OrganismName : Bacteria
   <400> PreSequenceString :
   tgtwawnnnn ntgtaarv 17
   <212> Type : DNA
   <211> Length : 17
   SequenceName : SEQ ID NO: 57
   SequenceDescription :

### Sequence

<213> OrganismName : Bacteria
   <400> PreSequenceString :
   gkttwannnn nnnnnnnnnn kggtaw 26
   <212> Type : DNA
   <211> Length : 26
   SequenceName : SEQ ID NO: 58
   SequenceDescription :

### Sequence

<213> OrganismName : K. pneumoniae
   <400> PreSequenceString :
   tggcacagat ttcgct 16
   <212> Type : DNA
   <211> Length : 16
   SequenceName : SEQ ID NO: 59
   SequenceDescription :

### Sequence

<213> OrganismName : K. pneumoniae
   <400> PreSequenceString :
   tggnnnnnnw tttgcw 16
   <212> Type : DNA
   <211> Length : 16
   SequenceName : SEQ ID NO: 60
   SequenceDescription :

## Claims

1. A non-therapeutic method of reducing the viability of prokaryotic cells, the method comprising:
(a) providing a decoy polynucleotide comprising a binding site for a target transcription factor (a decoy sequence);
(b) introducing the decoy polynucleotide into a prokaryotic cell which comprises a binding site for the transcription factor, operably linked to a gene or genes;
wherein introduction of the decoy polynucleotide reduces binding of the target transcription factor to the binding site in the cell and causes an alteration in expression of the operably linked gene or genes;
and wherein the target transcription factor comprises a regulator of expression of a gene or genes encoding one or more of:
(i) a cellular adaptive response;
(ii) a cellular intrinsic antibiotic resistance mechanism;
(iii) a cellular virulence factor;
(iv) a cellular stress response; or
(v) a cellular essential gene.

2. A non-therapeutic method of increasing prokaryotic antibiotic susceptibility, the method comprising:
(a) providing a decoy polynucleotide comprising a binding site for a target transcription factor (a decoy sequence);
(b) introducing the decoy polynucleotide into a prokaryotic cell which comprises a binding site for the transcription factor, operably linked to a gene or genes;
wherein introduction of the decoy polynucleotide reduces binding of the target transcription factor to the binding site in the cell and causes an alteration in expression of the operably linked gene or genes, thereby increasing antibiotic susceptibility of the cell;
and wherein the target transcription factor comprises a regulator of expression of a gene or genes encoding one or more of:
(i) a cellular adaptive response;
(ii) a cellular intrinsic antibiotic resistance mechanism;
(iii) a cellular virulence factor;
(iv) a cellular essential gene.

3. A method according to claim 1 or claim 2, wherein reducing viability comprises one or more of:
(i) inhibiting a cellular adaptive response such as a stress response;
(ii) increasing cellular susceptibility to antibiotics;
(iii) inhibiting expression of one or more essential genes;
(iv) inhibiting expression of one or more virulence genes.

4. A method according to any one of claims 1 to 3, wherein the target transcription factor is selected from: WhiB7; FadR; YycG/YycF; Sigma 54 (or SigA); Fur; TcdR; Vfr; NtrC; ArsR; TcaA; AgrA; WalR; sigB; Ksig; fhu; or a functional variant or homolog of any thereof.

5. A method according to any of claims 1 to 4, wherein the prokaryote is a bacterium.

6. A method according to claim 5, wherein the bacterium is pathogenic.

7. A method according to any of the preceding claims wherein the transcription factor binding site in the decoy polynucleotide is not operably linked to a gene.

8. A decoy polynucleotide as defined in any of claims 1 to 7 for use in treating bacterial infection.

9. A decoy polynucleotide according to claim 8 for use in treating bacterial infection, wherein treating the bacterial infection comprises use of one or more antibiotics and/or other antibacterial agent(s).

10. A decoy polynucleotide according to claim 8 or claim 9 for use in treating bacterial infection, wherein treating bacterial infection comprises treating a condition selected from: pneumonia, bacteremia, whooping cough, lyme's disease, brucellosis, acute enteritis, septicaemia, tularemia, influenza, peptic ulcers, Legionnaire's disease, gonorrhoeae, noscomial infections, sepsis, ricketts, typhoid, dysentery, cholera, plague, anthrax, pseudomembranous colitis, diptheria, listerosis, tuberculosis, septicaemia, meningitis.

11. An ex vivo method of killing bacteria, inhibiting bacterial growth, or reducing bacterial virulence, the method comprising applying a decoy polynucleotide as defined in any of claims 1 to 10, optionally in combination with one or more antibiotics and/or antibacterial agents.

12. Use of transcription factor decoys (TFDs) to reduce the viability of prokaryotic cells ex vivo.

13. Use of transcription factor decoys (TFDs) to reduce the virulence of prokaryotic cells ex vivo.

## Patentansprüche

1. Nicht-therapeutisches Verfahren zum Reduzieren der Lebensfähigkeit von prokaryotischen Zellen, welches umfasst:
(a) Bereitstellen eines Decoy-Polynukleotids, welches eine Bindungsstelle für einen Zieltranskriptionsfaktor (eine Decoy-Sequenz) umfasst;
(b) Einführen des Decoy-Polynukleotids in eine prokaryotische Zelle, die eine Bindungsstelle für den Transkriptionsfaktor umfasst, in Wirkbeziehung verknüpft mit einem Gen oder Genen;
wobei die Einführung des Decoy-Polynukleotids die Bindung des Zieltranskriptionsfaktors an die Bindungsstelle in der Zelle reduziert und eine Änderung der Expression des in Wirkbeziehung verknüpften Gens oder der in Wirkbeziehung verknüpften Gene verursacht;
und wobei der Zieltranskriptionsfaktor einen Regulator der Expression eines Gens oder von Genen umfasst, das/die für einen oder mehrere von:
(i) einer zellulären adaptiven Antwort;
(ii) einem zellulären intrinsischen Antibiotikaresistenzmechanismus;
(iii) einem zellulären Virulenzfaktor;
(iv) einer zellulären Stressantwort oder
(v) einem zellulären essentiellen Gen kodiert/kodieren.

2. Nicht-therapeutisches Verfahren zum Erhöhen der prokaryotischen Antibiotikaempfindlichkeit, welches umfasst:
(a) Bereitstellen eines Decoy-Polynukleotids, welches eine Bindungsstelle für einen Zieltranskriptionsfaktor (eine Decoy-Sequenz) umfasst;
(b) Einführen des Decoy-Polynukleotids in eine prokaryotische Zelle, die eine Bindungsstelle für den Transkriptionsfaktor umfasst, in Wirkbeziehung verknüpft mit einem Gen oder Genen;
wobei die Einführung des Decoy-Polynukleotids die Bindung des Zieltranskriptionsfaktors an die Bindungsstelle in der Zelle reduziert und eine Änderung der Expression des in Wirkbeziehung verknüpften Gens oder der in Wirkbeziehung verknüpften Gene verursacht, wodurch die Antibiotikaempfindlichkeit der Zelle erhöht wird;
und wobei der Zieltranskriptionsfaktor einen Regulator der Expression eines Gens oder von Genen umfasst, das/die für einen oder mehrere von:
(i) einer zellulären adaptiven Antwort;
(ii) einem zellulären intrinsischen Antibiotikaresistenzmechanismus;
(iii) einem zellulären Virulenzfaktor;
(iv) einem zellulären essentiellen Gen kodiert/kodieren.

3. Verfahren nach Anspruch 1 oder Anspruch 2, wobei das Reduzieren der Lebensfähigkeit ein oder mehrere der Folgenden umfasst:
(i) Inhibieren einer zellulären adaptiven Antwort, wie einer Stressantwort;
(ii) Erhöhen der zellulären Empfindlichkeit gegenüber Antibiotika;
(iii) Inhibieren der Expression von einem oder mehreren essentiellen Genen;
(iv) Inhibieren der Expression von einem oder mehreren Virulenzgenen.

4. Verfahren nach einem der Ansprüche 1 bis 3, wobei der Zieltranskriptionsfaktor ausgewählt ist aus: WhiB7; FadR; YycG/YycF; Sigma 54 (oder SigA); Fur; TcdR; Vfr; NtrC; ArsR; TcaA; AgrA; WalR; sigB; Ksig; fhu; oder einer funktionalen Variante oder einem Homologen von beliebigen davon.

5. Verfahren nach einem der Ansprüche 1 bis 4, wobei der Prokaryot ein Bakterium ist.

6. Verfahren nach Anspruch 5, wobei das Bakterium pathogen ist.

7. Verfahren nach einem der vorhergehenden Ansprüche, wobei die Transkriptionsfaktorbindungsstelle in dem Decoy-Polynukleotid nicht in Wirkbeziehung mit einem Gen verknüpft ist.

8. Decoy-Polynukleotid nach einem der Ansprüche 1 bis 7 zur Verwendung in der Behandlung von bakterieller Infektion.

9. Decoy-Polynukleotid nach Anspruch 8 zur Verwendung in der Behandlung von bakterieller Infektion, wobei die Behandlung der bakteriellen Infektion die Verwendung von einem Antibiotikum oder mehreren Antibiotika und/oder anderem antibakteriellem Mittel/anderen antibakteriellen Mitteln umfasst.

10. Decoy-Polynukleotid nach Anspruch 8 oder Anspruch 9 zur Verwendung in der Behandlung von bakterieller Infektion, wobei die Behandlung der bakteriellen Infektion die Behandlung eines Zustands ausgewählt aus: Pneumonie, Bakteriämie, Keuchhusten, Borreliose, Brucellose, akuter Enteritis, Septikämie, Tularämie, Influenza, Magengeschwüren, Legionärskrankheit, Gonorrhoe, nosokomialen Infektionen, Sepsis, Rickettsieninfektionen, Typhus, Dysenterie, Cholera, Pest, Milzbrand, pseudomembranöser Kolitis, Diphtherie, Listeriose, Tuberkulose, Septikämie, Meningitis umfasst.

11. Ex-vivo-Verfahren zum Töten von Bakterien, Inhibieren des bakteriellen Wachstums oder Reduzieren der bakteriellen Virulenz, welches Anwenden eines Decoy-Polynucleotids wie in einem der Ansprüche 1 bis 10 definiert, gegebenenfalls in Kombination mit einem oder mehreren Antibiotika und/oder antibakteriellen Mitteln, umfasst.

12. Verwendung von Transkriptionsfaktor-Decoys (TFDs) zum Reduzieren der Lebensfähigkeit von prokaryotischen Zellen ex-vivo.

13. Verwendung von Transkriptionsfaktor-Decoys (TFDs) zum Reduzieren der Virulenz von prokaryotischen Zellen ex-vivo.

## Revendications

1. Procédé non thérapeutique de réduction de la viabilité de cellules procaryotes, le procédé comprenant :
(a) l'obtention d'un polynucléotide leurre comprenant un site de liaison pour un facteur de transcription cible (une séquence leurre) ;
(b) l'introduction du polynucléotide leurre dans une cellule procaryote qui comprend un site de liaison pour le facteur de transcription, lié de façon opérationnelle à un ou des gènes ;
dans lequel l'introduction du polynucléotide leurre réduit la liaison du facteur de transcription cible au site de liaison dans la cellule et provoque une modification de l'expression du gène ou des gènes liés de façon opérationnelle ;
et dans lequel le facteur de transcription cible comprend un régulateur de l'expression d'un ou de plusieurs gènes codant un ou plusieurs des éléments suivants :
(i) une réponse adaptative cellulaire ;
(ii) un mécanisme cellulaire intrinsèque de résistance aux antibiotiques ;
(iii) un facteur de virulence cellulaire ;
(iv) une réponse à un stress cellulaire ; ou
(v) un gène essentiel cellulaire.

2. Procédé non thérapeutique d'augmentation de la sensibilité aux antibiotiques des procaryotes, le procédé comprenant :
(a) l'obtention d'un polynucléotide leurre comprenant un site de liaison pour un facteur de transcription cible (une séquence leurre) ;
(b) l'introduction du polynucléotide leurre dans une cellule procaryote qui comprend un site de liaison pour le facteur de transcription, lié de façon opérationnelle à un ou des gènes ;
dans lequel l'introduction du polynucléotide leurre réduit la liaison du facteur de transcription cible au site de liaison dans la cellule et provoque une modification de l'expression du ou des gènes liés de façon opérationnelle, en augmentant ainsi la sensibilité aux antibiotiques de la cellule ;
et dans lequel le facteur de transcription cible comprend un régulateur de l'expression d'un ou de plusieurs gènes codant un ou plusieurs des éléments suivants :
(i) une réponse adaptative cellulaire ;
(ii) un mécanisme cellulaire intrinsèque de résistance aux antibiotiques ;
(iii) un facteur de virulence cellulaire ;
(iv) un gène essentiel cellulaire.

3. Procédé selon la revendication 1 ou 2, dans lequel la réduction de la viabilité comprend un ou plusieurs des effets suivants :
(i) inhibition d'une réponse adaptative cellulaire comme une réponse à un stress ;
(ii) augmentation de la sensibilisé cellulaire aux antibiotiques ;
(iii) inhibition de l'expression d'un ou de plusieurs gènes essentiels ;
(iv) inhibition de l'expression d'un ou de plusieurs gènes de virulence.

4. Procédé selon l'une quelconque des revendications 1 à 3, dans lequel le facteur de transcription cible est choisi parmi : WhiB7 ; FadR ; YycG/YycF ; Sigma 54 (ou SigA) ; Fur ; TcdR ; Vfr ; NtrC ; ArsR ; TcaA ; AgrA ; WalR ; sigB ; Ksig ; fhu ; ou un variant ou un homologue fonctionnel de l'un quelconque de ceux-ci.

5. Procédé selon l'une quelconque des revendications 1 à 4, dans lequel le procaryote est une bactérie.

6. Procédé selon la revendication 5, dans lequel la bactérie est pathogène.

7. Procédé selon l'une quelconque des revendications précédentes, dans lequel le site de liaison du facteur de transcription dans le polynucléotide leurre n'est pas lié de façon fonctionnelle à un gène.

8. Polynucléotide leurre défini dans l'une quelconque des revendications 1 à 7, destiné à être utilisé dans le traitement d'une infection bactérienne.

9. Polynucléotide leurre selon la revendication 8, destiné à être utilisé dans le traitement d'une infection bactérienne, le traitement de l'infection bactérienne comprenant l'utilisation d'un ou de plusieurs antibiotiques et/ou d'un ou de plusieurs autres agents antibactériens.

10. Polynucléotide leurre selon la revendication 8 ou la revendication 9, destiné à être utilisé dans le traitement d'une infection bactérienne, le traitement de l'infection bactérienne comprenant le traitement d'une affection choisie parmi les suivantes : pneumonie, bactériémie, coqueluche, maladie de Lyme, brucellose, entérite aigüe, septicémie, tularémie, grippe, ulcères gastroduodénaux, légionellose, gonorrhée, infections nosocomiales, sepsie, rickettsiose, typhoïde, dysenterie, choléra, peste, charbon, colite pseudo-membraneuse, diphtérie, listériose, tuberculose, septicémie, méningite.

11. Procédé ex-vivo pour tuer des bactéries, inhiber la croissance bactérienne ou réduire la virulence bactérienne, le procédé comprenant l'application d'un polynucléotide leurre défini dans l'une quelconque des revendications 1 à 10, éventuellement en combinaison avec un ou plusieurs antibiotiques et/ou agents antibactériens.

12. Utilisation de leurres de facteur de transcription (LFT) pour réduire la viabilité de cellules procaryotes ex vivo.

13. Utilisation de leurres de facteur de transcription (LFT) pour réduire la virulence de cellules procaryotes ex vivo.
